# EUROPÄISCHE PATENTSCHRIFT

(11) **EP 2 909 286 B1**
(45) Veröffentlichungstag und Bekanntmachung des Hinweises auf die Patenterteilung: **22.11.2017**
(21) Anmeldenummer: 13773610.4
(22) Anmeldetag: 01.10.2013
(51) Int. Cl.: C09K 19/34, C09K 19/04, C09K 19/44, C09K 19/20, C09K 19/30, C09K 19/02, C09K 19/54, C09K 19/58, C07D 213/61

(54) **FLÜSSIGKRISTALLINES MEDIUM**
LIQUID CRYSTALLINE MEDIUM
MILIEU LIQUIDE CRISTALLIN

(30) Priorität: 22.10.2012 DE 102012020685
(43) Veröffentlichungstag der Anmeldung: 26.08.2015
(73) Patentinhaber: Merck Patent GmbH, 64293 Darmstadt (DE)
(72) Erfinder: WITTEK, Michael, 64390 Erzhausen (DE); SCHULER, Brigitte, 63762 Grossostheim (DE); LIETZAU, Lars, 64380 Rossdorf (DE)
(86) Internationale Anmeldenummer: PCT/EP2013/002945
(87) Internationale Veröffentlichungsnummer: WO 2014/063777

(56) Entgegenhaltungen:
- DE-A1-102008 016 053
- DE-A1-102008 035 718

## Beschreibung

Die vorliegende Erfindung betrifft ein flüssigkristallines Medium (FK-Medium), dessen Verwendung für elektrooptische Zwecke und dieses Medium enthaltende FK-Anzeigen.

Flüssigkristalle werden vor allem als Dielektrika in Anzeigevorrichtungen verwendet, da die optischen Eigenschaften solcher Substanzen durch eine angelegte Spannung beeinflusst werden können. Elektrooptische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super-twisted nematic"), SBE-Zellen ("superbirefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannungen und einen hohen Kontrast ergeben.

Weiterhin sollten sie bei üblichen Betriebstemperaturen, d.h. in einem möglichst breiten Bereich unterhalb und oberhalb Raumtemperatur eine geeignete Mesophase besitzen, beispielsweise für die oben genannten Zellen eine nematische oder cholesterische Mesophase. Da Flüssigkristalle in der Regel als Mischungen mehrerer Komponenten zur Anwendung gelangen, ist es wichtig, dass die Komponenten untereinander gut mischbar sind. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine geringe elektrische Leitfähigkeit aufweisen.

Beispielsweise sind für Matrix-Flüssigkristallanzeigen mit integrierten nicht-linearen Elementen zur Schaltung einzelner Bildpunkte (MFK-Anzeigen) Medien mit großer positiver dielektrischer Anisotropie, breiten nematischen Phasen, relativ niedriger Doppelbrechung, sehr hohem spezifischen Widerstand, guter UV- und Temperaturstabilität und geringem Dampfdruck erwünscht.

Derartige Matrix-Flüssigkristallanzeigen sind bekannt. Als nichtlineare Elemente zur individuellen Schaltung der einzelnen Bildpunkte können beispielsweise aktive Elemente (d.h. Transistoren) verwendet werden. Man spricht dann von einer "aktiven Matrix", wobei man zwei Typen unterscheiden kann:
1. MOS (Metal Oxide Semiconductor) oder andere Dioden auf Silizium-Wafer als Substrat.
2. Dünnfilm-Transistoren (TFT) auf einer Glasplatte als Substrat.

Die Verwendung von einkristallinem Silizium als Substratmaterial beschränkt die Displaygröße, da auch die modulartige Zusammensetzung verschiedener Teildisplays an den Stößen zu Problemen führt.

Bei dem aussichtsreicheren Typ 2, welcher bevorzugt ist, wird als elektrooptischer Effekt üblicherweise der TN-Effekt verwendet. Man unterscheidet zwei Technologien: TFT's aus Verbindungshalbleitern wie z.B. CdSe oder TFT's auf der Basis von polykristallinem oder amorphem Silizium. An letzterer Technologie wird weltweit mit großer Intensität gearbeitet.

Die TFT-Matrix ist auf der Innenseite der einen Glasplatte der Anzeige aufgebracht, während die andere Glasplatte auf der Innenseite die transparente Gegenelektrode trägt. Im Vergleich zu der Größe der Bildpunkt-Elektrode ist der TFT sehr klein und stört das Bild praktisch nicht. Diese Technologie kann auch für voll farbtaugliche Bilddarstellungen erweitert werden, wobei ein Mosaik von roten, grünen und blauen Filtern derart angeordnet ist, dass je ein Filterelement einem schaltbaren Bildelement gegenüber liegt.
Die TFT-Anzeigen arbeiten üblicherweise als TN-Zellen mit gekreuzten Polarisatoren in Transmission und sind von hinten beleuchtet.

Der Begriff MFK-Anzeigen umfasst hier jedes Matrix-Display mit integrierten nichtlinearen Elementen, d.h. neben der aktiven Matrix auch Anzeigen mit passiven Elementen wie Varistoren oder Dioden (MIM = Metall-Isolator-Metall).

Derartige MFK-Anzeigen eignen sich insbesondere für TV-Anwendungen (z.B. Taschenfernseher) oder für hochinformative Displays für Rechneranwendungen (Laptop) und im Automobil- oder Flugzeugbau. Neben Problemen hinsichtlich der Winkelabhängigkeit des Kontrastes und der Schaltzeiten resultieren bei MFK-Anzeigen Schwierigkeiten bedingt durch nicht ausreichend hohen spezifischen Widerstand der Flüssigkristallmischungen [TOGASHI, S., SEKIGUCHI, K., TANABE, H., YAMAMOTO, E., SORIMACHI, K., TAJIMA, E., WATANABE, H., SHIMIZU, H., Proc. Eurodisplay 84, Sept. 1984: A 210-288 Matrix LCD Controlled by Double Stage Diode Rings, p. 141 ff, Paris; STROMER, M., Proc. Eurodisplay 84, Sept. 1984: Design of Thin Film Transistors for Matrix Adressing of Television Liquid Crystal Displays, p. 145 ff, Paris]. Mit abnehmendem Widerstand verschlechtert sich der Kontrast einer MFK-Anzeige und es kann das Problem der "after image elimination" auftreten. Da der spezifische Widerstand der Flüssigkristallmischung durch Wechselwirkung mit den inneren Oberflächen der Anzeige im allgemeinen über die Lebenszeit einer MFK-Anzeige abnimmt, ist ein hoher (Anfangs)-Widerstand sehr wichtig, um akzeptable Standzeiten zu erhalten. Insbesondere bei low-volt-Mischungen war es bisher nicht möglich, sehr hohe spezifische Widerstände zu realisieren. Weiterhin ist es wichtig, dass der spezifische Widerstand eine möglichst geringe Zunahme bei steigender Temperatur sowie nach Temperatur- und/oder UV-Belastung zeigt. Besonders nachteilig sind auch die Tieftemperatureigenschaften der Mischungen aus dem Stand der Technik. Gefordert wird, dass auch bei tiefen Temperaturen keine Kristallisation und/oder smektische Phasen auftreten und die Temperaturabhängigkeit der Viskosität möglichst gering ist. Die MFK-Anzeigen aus dem Stand der Technik genügen somit nicht den heutigen Anforderungen.

Neben Flüssigkristallanzeigen, die eine Hintergrundbeleuchtung verwenden, also transmissiv und gegebenenfalls transflektiv betrieben werden, sind besonders auch reflektive Flüssigkristallanzeigen interessant. Diese reflektiven Flüssigkristallanzeigen benutzen das Umgebungslicht zur Informationsdarstellung. Somit verbrauchen sie wesentlich weniger Energie als hintergrundbeleuchtete Flüssigkristallanzeigen mit entsprechender Größe und Auflösung. Da der TN-Effekt durch einen sehr guten Kontrast gekennzeichnet ist, sind derartige reflektive Anzeigen auch bei hellen Umgebungsverhältnissen noch gut abzulesen. Dies ist bereits von einfachen reflektiven TN-Anzeigen, wie sie in z. B. Armbanduhren und Taschenrechnern verwendet werden, bekannt. Jedoch ist das Prinzip auch auf hochwertige, höher auflösende Aktiv-Matrix angesteuerte Anzeigen wie z. B. TFT-Displays anwendbar. Hier ist wie bereits bei den allgemeinen üblichen transmissiven TFT-TN-Anzeigen die Verwendung von Flüssigkristallen mit niedriger Doppelbrechung (Δn) nötig, um eine geringe optische Verzögerung (d · Δn) zu erreichen. Diese geringe optische Verzögerung führt zu einer meist akzeptablen geringen Blickwinkelabhängigkeit des Kontrastes (vgl. DE 30 22 818). Bei reflektiven Anzeigen ist die Verwendung von Flüssigkristallen mit kleiner Doppelbrechung noch wichtiger als bei transmissiven Anzeigen, da bei reflektiven Anzeigen die effektive Schichtdicke, die das Licht durchquert, ungefähr doppelt so groß ist wie bei transmissiven Anzeigen mit derselben Schichtdicke.

Zur Realisierung von 3D-Effekten mittels Shutterbrillen werden insbesondere schnell schaltende Mischungen mit niedrigen Rotationsviskositäten und einer entsprechend hohen optischen Anisotropie (Δn) eingesetzt. Elektrooptische Linsensyteme, mit denen eine 2-dimensionale Darstellung eines Displays in eine 3-dimensionale autostereoskopische Darstellung geschaltet werden kann, können unter Verwendung von Mischungen mit hoher optischer Anisotropie (Δn) realisiert werden.

Es besteht somit immer noch ein großer Bedarf nach MFK-Anzeigen mit sehr hohem spezifischen Widerstand bei gleichzeitig großem Arbeitstemperaturbereich, kurzen Schaltzeiten auch bei tiefen Temperaturen und niedriger Schwellenspannung, die diese Nachteile nicht oder nur in geringerem Maße zeigen.

Bei TN-(Schadt-Helfrich)-Zellen sind Medien erwünscht, die folgende Vorteile in den Zellen ermöglichen:
- erweiterter nematischer Phasenbereich (insbesondere zu tiefen Temperaturen)
- Schaltbarkeit bei extrem tiefen Temperaturen (out-door-use, Automobil, Avionik)
- erhöhte Beständigkeit gegenüber UV-Strahlung (längere Lebensdauer)
- kleine Schwellenspannung.

Mit den aus dem Stand der Technik zur Verfügung stehenden Medien ist es nicht möglich, diese Vorteile unter gleichzeitigem Erhalt der übrigen Parameter zu realisieren. Moderne LCD-Flachbildschirme erfordern immer schnellere Schaltzeiten um Multimediainhalte, wie z.B. Filme und Videospiele, realistisch wiedergeben zu können. Dies wiederum erfordert nematische Flüssigkristallmischungen, die eine sehr kleine Rotationsviskosität γ₁ mit einer hohen optischen Anisotropie Δn aufweisen. Um die geforderten Rotationsviskositäten zu erhalten, werden Substanzen gesucht, die ein besonders vorteilhaftes γ₁/Klärpunkt-Verhältnis bei gleichzeitig hohem Δn mit hoher Polarität aufweisen.

Bei höher verdrillten Zellen (STN) sind Medien erwünscht, die eine höhere Multiplexierbarkeit und/oder kleinere Schwellenspannungen und/oder breitere nematische Phasenbereiche (insbesondere bei tiefen Temperaturen) ermöglichen. Hierzu ist eine weitere Ausdehnung des zur Verfügung stehenden Parameterraumes (Klärpunkt, Übergang smektisch-nematisch bzw. Schmelzpunkt, Viskosität, dielektrische Größen, elastische Größen) dringend erwünscht.

Insbesondere bei FK-Anzeigen für TV- und Video-Anwendungen (z. B. LCD-TV, Monitore, PDAs, Notebooks, Spielkonsolen) ist eine deutliche Verringerung der Schaltzeiten gewünscht. Dies erfordert FK-Mischungen mit niedrigen Rotationsviskositäten und hohen dielektrischen Anisotropien. Gleichzeitig sollten die FK-Medien hohe Klärpunkte, vorzugsweise ≥ 80 °C, aufweisen.

Der Erfindung liegt die Aufgabe zugrunde Medien, insbesondere für derartige MFK-, FFS-, IPS-, TN-, PS-FFS, PS-IPS, positive VA- oder STN-Anzeigen, bereitzustellen, die die oben angegebenen Nachteile nicht oder nur in geringerem Maße zeigen, und vorzugsweise schnelle Schaltzeiten und niedrige Rotationsviskositäten bei gleichzeitig hohem Klärpunkt, sowie eine hohe dielektrische Anisotropie und eine niedrige Schwellenspannung aufweisen.

Es wurde nun gefunden, dass diese Aufgabe gelöst werden kann, wenn man FK-Medien enthaltend eine oder mehrere Verbindungen der Formel IA verwendet. Die Verbindungen der Formel IA führen zu LC-Mischungen mit den oben angegebenen gewünschten Eigenschaften.

Gegenstand der Erfindung ist ein flüssigkristallines Medium, dadurch gekennzeichnet, dass es eine oder mehrere Verbindungen der Formel IA 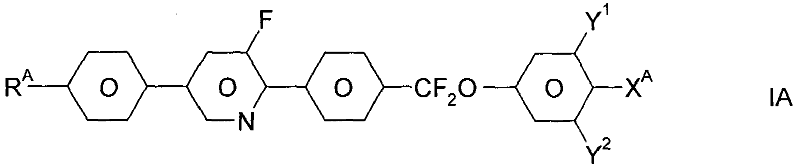 worin
- R^{A}: einen halogenierten oder unsubstituierten Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O- -CH=CH-, 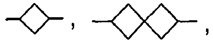 -O-, -CO-O- oder -O-CO- so ersetzt sein können, dass O-Atome nicht direkt miteinander verknüpft sind,
- X^{A}: F, Cl, CN, SF₅, SCN, NCS, halogenierter Alkylrest, halogenierter Alkenylrest, halogenierter Alkoxyrest oder halogenierter Alkenyloxyrest jeweils mit bis zu 6 C-Atomen, und
- Y¹⁻²: jeweils unabhängig voneinander H oder F
bedeuten,
enthält.

Verbindungen dieses Typs sind von der DE 10 2008 016 053 A1, in der allgemein von Stickstoffheterocylen abgeleitete und mit einer Difluormethylenoxy-Gruppe verbrückte Flüssigkristall-Verbindungen beschrieben sind, generisch umfaßt, sind dort aber weder explizit genannt noch nahegelegt.

Überraschenderweise wurde gefunden, dass Mischungen enthaltend eine oder mehrere Verbindungen der Formel IA eine hohe dielektrische Anisotropie Δε und gleichzeitig ein vorteilhaftes Verhältnis Rotationsviskosität γ₁/Klärpunkt aufweisen. Sie sind daher zur Realisierung von Flüssigkristallmischungen mit niedrigem γ₁ und sehr hohem Klärpunkt besonders geeignet. Darüber hinaus zeigen die Verbindungen der Formel IA eine gute Löslichkeit und ein sehr gutes Phasenverhalten in FK-Medien. Erfindungsgemäße FK-Medien enthaltend Verbindungen der Formel IA besitzen eine niedrige Rotationsviskosität, schnelle Schaltzeiten, einen hohen Klärpunkt, eine sehr hohe positive dielektrische Anisotropie, eine relativ hohe Doppelbrechung und einen breiten nematischen Phasenbereich. Sie sind deshalb besonders gut für mobile Telefone, TV-und Video-Anwendungen geeignet.

Die Verbindungen der Formel IA besitzen einen breiten Anwendungsbereich. In Abhängigkeit von der Auswahl der Substituenten können sie als Basismaterialien dienen, aus denen flüssigkristalline Medien zum überwiegenden Teil zusammengesetzt sind; es können aber auch den Verbindungen der Formel IA flüssigkristalline Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel IA besitzen relativ niedrige Schmelzpunkte, zeigen ein gutes Phasenverhalten, sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Falls in den oben- und untenstehenden Formeln R^{A} einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradedoxy. R^{A} und R^{B} bedeuten jeweils vorzugsweise geradkettiges Alkyl mit 2-6 C-Atomen.

Oxaalkyl bedeutet vorzugsweise geradkettiges 2-Oxapropyl (= Methoxymethyl), 2-(= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6-, oder 7-Oxaoctyl, 2-, 3-, 4-, 5-, 6-, 7- oder 8-Oxanonyl, 2-, 3-, 4-, 5-, 6-, 7-, 8- oder 9-Oxadexyl.

Falls R^{A} einen Alkylrest bedeutet, in dem eine CH₂-Gruppe durch -CH=CH-ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3-oder Pent-4-enyl, Hex-1-, 2-, 3-, 4- oder Hex-5-enyl, Hept-1-, 2-, 3-, 4-, 5-oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R^{A} einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

In den oben- und untenstehenden Formeln ist X^{A} vorzugsweise F, Cl oder ein- oder mehrfach fluorierter Alkyl- oder Alkoxyrest mit 1, 2 oder 3 C-Atomen oder ein- oder mehrfach fluorierter Alkenylrest mit 2 oder 3 C-Atomen bedeutet. X^{A} ist besonders bevorzugt F, Cl, CF₃, CHF₂, OCF₃, OCHF₂, OCFHCF₃, OCFHCHF₂, OCFHCHF₂, OCF₂CH₃, OCF₂CHF₂, OCF₂CHF₂, OCF₂CF₂CHF₂, OCF₂CF₂CHF₂, OCFHCF₂CF₃, OCFHCF₂CHF₂, OCF₂CF₂CF₃, OCF₂CF₂CClF₂, OCClFCF₂CF₃, OCH=CF₂ oder CH=CF₂, ganz besonders bevorzugt F oder OCF₃, ferner CF₃, OCF=CF₂, OCHF₂ und OCH=CF₂.

Besonders bevorzugt sind Verbindungen der Formel IA, worin X^{A} F oder OCF₃, vorzugsweise F, bedeutet. Bevorzugte Verbindungen der Formel IA sind solche, worin Y¹ F bedeutet, solche worin Y² F bedeutet, solche worin Y³, Y⁴, Y⁵ und Y⁶ jeweils H bedeuten.

Besonders bevorzugte Verbindungen der Formel IA sind ausgewählt aus folgenden Unterformeln, 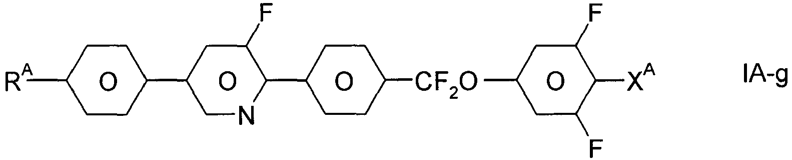 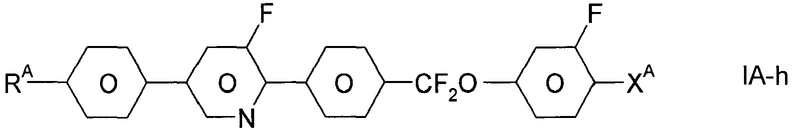 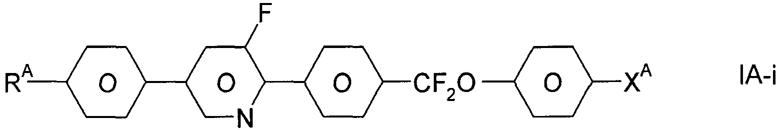 worin
R^{A} und X^{A} die in Anspruch 1 angegebenen Bedeutungen haben. Vorzugsweise bedeutet R^{A} geradkettiges Alkyl mit 1 bis 6 C-Atomen, insbesondere Ethyl und Propyl, ferner Alkenyl mit 2 bis 6 C-Atomen. X^{A} bedeutet vorzugsweise F oder OCF₃.

Ganz besonders bevorzugt ist die Verbindung der Unterformel IA-g, insbesondere wenn X^{A} = F ist.

Die Verbindungen der Formel IA werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Verbindungen der Formel IA können beispielsweise wie folgt hergestellt werden:

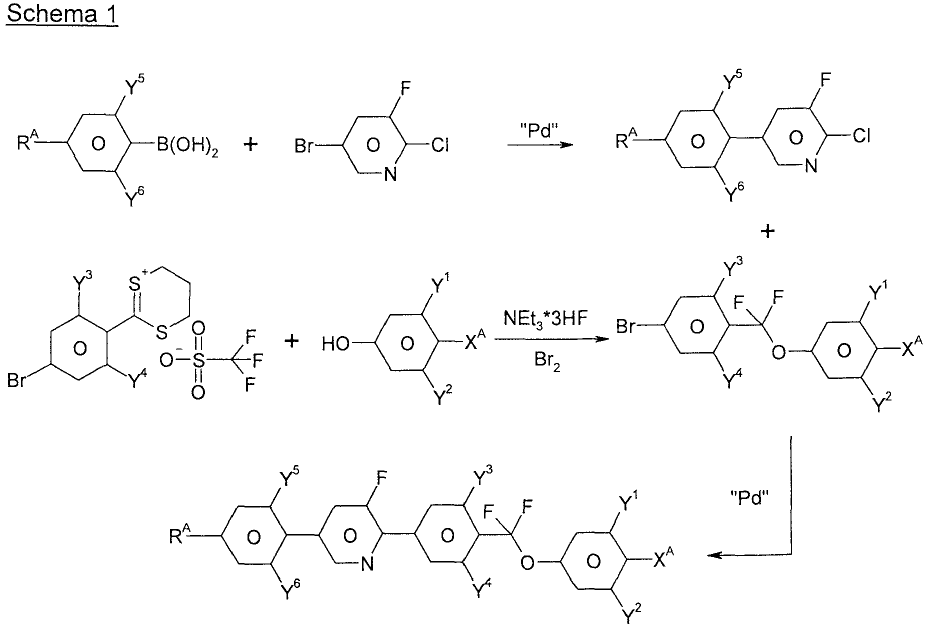

Gegenstand der Erfindung sind auch die Verbindungen der Formel IA.

Bevorzugte Ausführungsformen für die erfindungsgemäßen Mischungen sind im Folgenden angegeben:
- Das Medium enthält zusätzlich eine oder mehrere Verbindungen der Formel II und/oder III 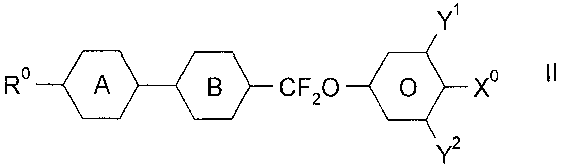 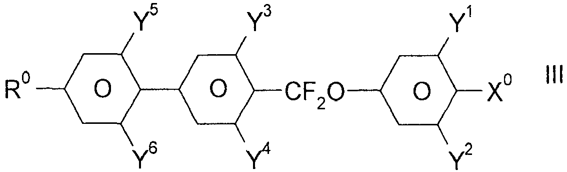 worin
   - R⁰: einen halogenierten oder unsubstituierten Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH-, 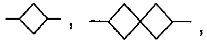 -O-, -CO-O- oder -O-CO- so ersetzt sein können, dass O-Atome nicht direkt miteinander verknüpft sind,
   - X⁰: F, Cl, CN, SF₅, SCN, NCS, halogenierter Alkylrest, halogenierter Alkenylrest, halogenierter Alkoxyrest oder halogenierter Alkenyloxyrest mit bis zu 6 C-Atomen, und
   - Y¹⁻⁶: jeweils unabhängig voneinander H oder F 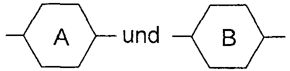 jeweils unabhängig voneinander 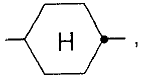 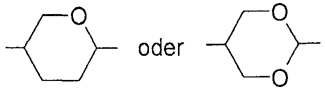
   bedeuten.
- Die Verbindungen der Formel II sind vorzugsweise ausgewählt aus den folgenden Formeln, 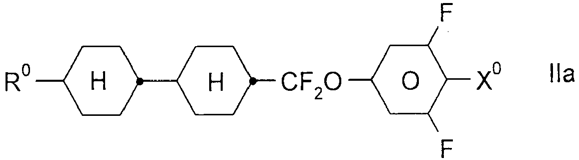 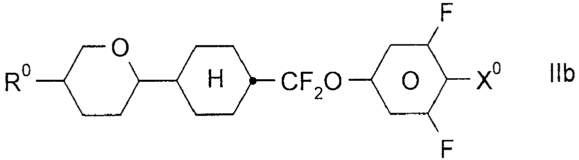 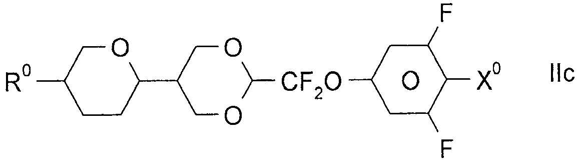 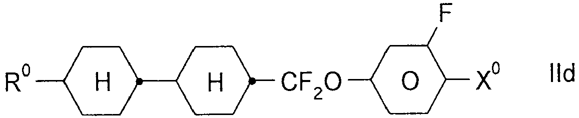 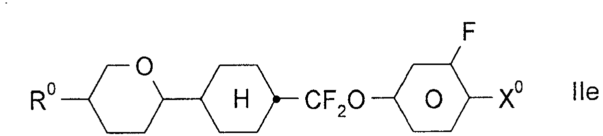 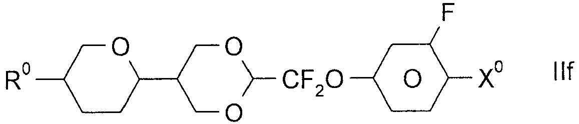 worin R⁰ und X⁰ die oben angegebenen Bedeutungen haben.

Vorzugsweise bedeutet R⁰ Alkyl mit 1 bis 6 C-Atomen. X⁰ bedeutet vorzugsweise F. Besonders bevorzugt sind Verbindungen der Formel IIa und IIb, insbesondere Verbindungen der Formeln IIa und IIb, worin X F bedeutet.
- Die Verbindungen der Formel III sind vorzugsweise ausgewählt aus den folgenden Formeln, 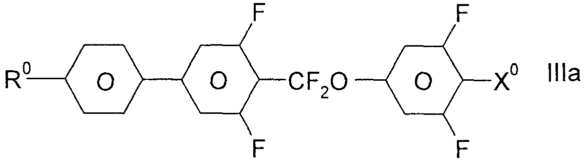 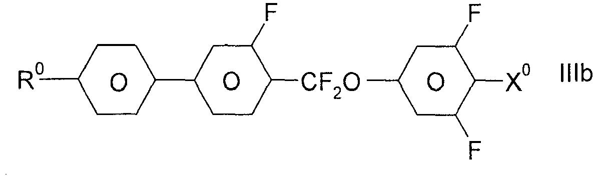 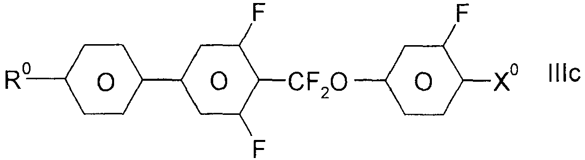 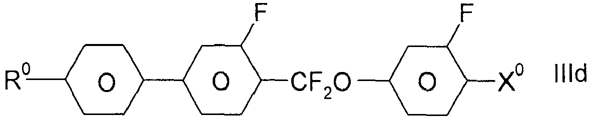 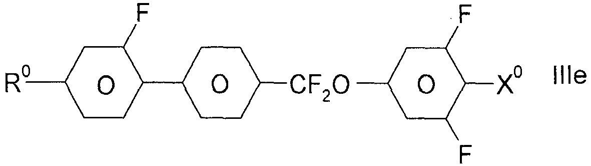 worin R⁰ und X⁰ die oben angegebenen Bedeutungen haben. Vorzugsweise bedeutet R⁰ Alkyl mit 1 bis 6 C-Atomen. X⁰ bedeutet vorzugsweise F. Besonders bevorzugt sind Verbindungen der Formeln IIIa und IIIe, insbesondere Verbindungen der Formel IIIa;
- Das Medium enthält zusätzlich eine oder mehrere Verbindungen ausgewählt aus den folgenden Formeln, 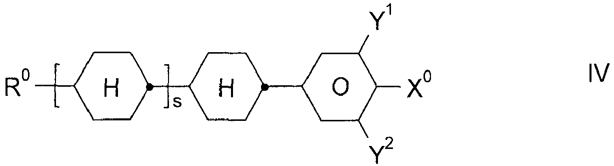 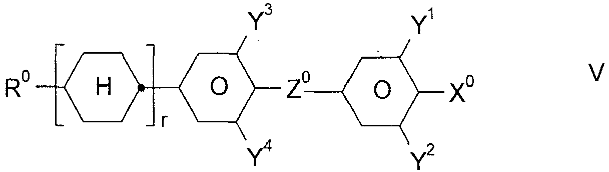 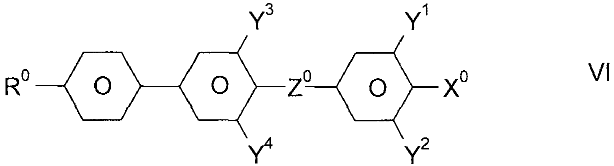 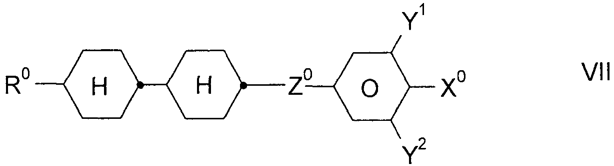 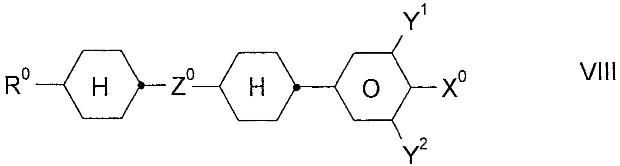 worin
   - R⁰, X⁰ und Y¹⁻⁴: die oben angegebenen Bedeutungen besitzen, und
   - Z⁰: -C₂H₄-, -(CH₂)₄-, -CH=CH-, -CF=CF-, -C₂F₄-, -CH₂CF₂-, -CF₂CH₂-, -CH₂O-, -OCH₂-, -COO- oder -OCF₂-, in Formel V und VI auch eine Einfachbindung, in Formel V und VIII auch -CF₂O-,
   - r: 0 oder 1, und
   - s: 0 oder 1
   bedeuten;
- Die Verbindungen der Formel IV sind vorzugsweise ausgewählt aus folgenden Formeln, 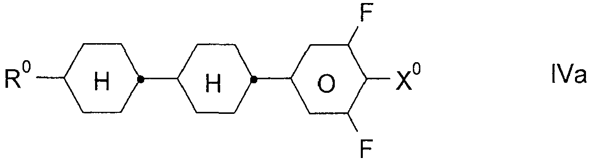 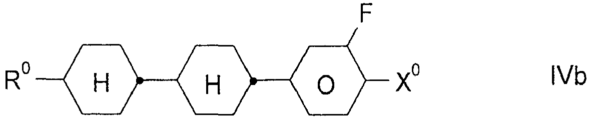 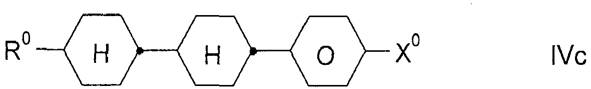 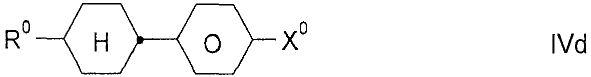
worin R⁰ und X⁰ die oben angegebenen Bedeutungen haben.

Vorzugsweise bedeutet R⁰ Alkyl mit 1 bis 6 C-Atomen. X⁰ bedeutet vorzugsweise F oder OCF₃, ferner OCF=CF₂, CF₃ und Cl;
- Die Verbindungen der Formel V sind vorzugsweise ausgewählt aus den folgenden Formeln, 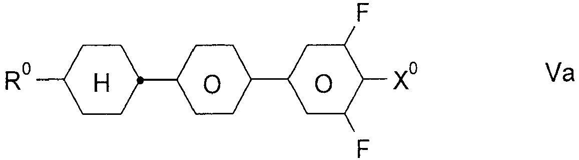 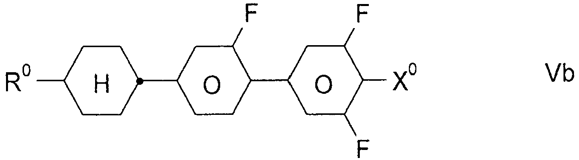 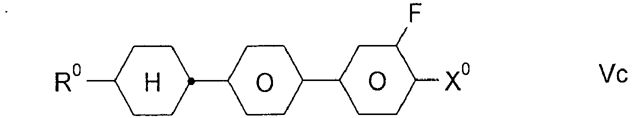 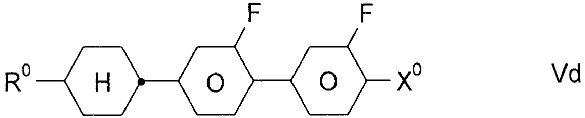 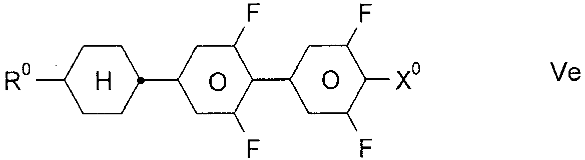 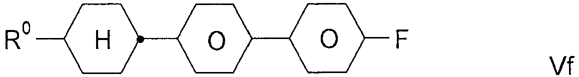 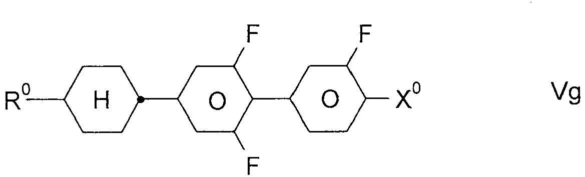 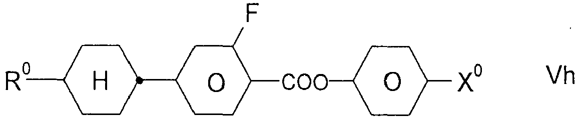 worin R⁰ und X⁰ die oben angegebenen Bedeutungen haben. Vorzugsweise bedeutet R⁰ Alkyl mit 1 bis 6 C-Atomen. X⁰ bedeutet vorzugsweise F und OCF₃, ferner OCHF₂, CF₃, OCF=CF₂ und OCH=CF₂;
- Die Verbindungen der Formel VI sind vorzugsweise ausgewählt aus den folgenden Formeln, 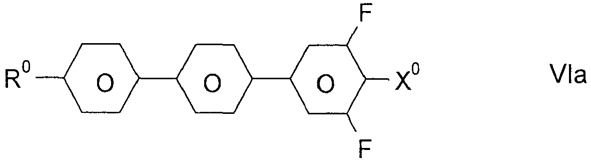 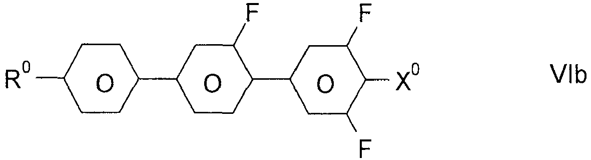 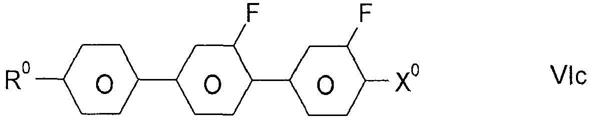 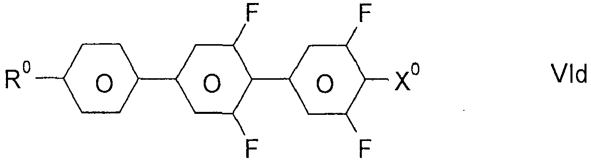 worin R⁰ und X⁰ die oben angegebenen Bedeutungen haben.

Vorzugsweise bedeutet R⁰ Alkyl mit 1 bis 6 C-Atomen. X⁰ bedeutet vorzugsweise F, ferner OCF₃, CF₃, CF=CF₂, OCHF₂ und OCH=CF₂.
- Die Verbindungen der Formel VII sind vorzugsweise ausgewählt aus den folgenden Formeln, 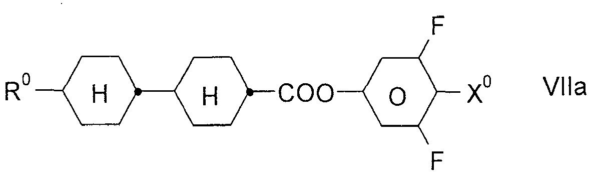 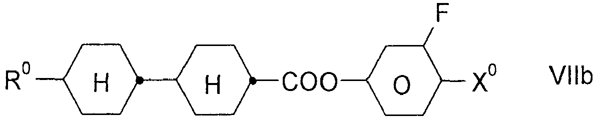 worin R⁰ und X⁰ die oben angegebenen Bedeutungen haben.

Vorzugsweise bedeutet R⁰ Alkyl mit 1 bis 6 C-Atomen. X⁰ bedeutet vorzugsweise F, ferner OCF₃, CF₃, OCHF₂ und OCH=CF₂.
- Das Medium enthält zusätzlich eine oder mehrere Verbindungen ausgewählt aus den folgenden Formeln, 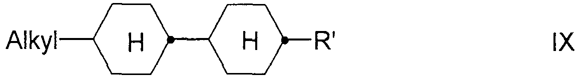 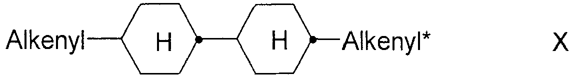 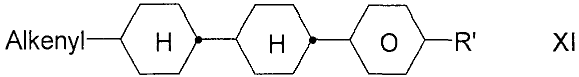 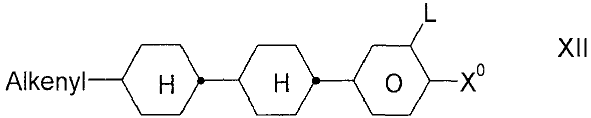 worin X⁰ die oben angegebenen Bedeutungen besitzen, und
   - L: H oder F,
   - "Alkyl": C₁₋₆-Alkyl,
   - R': C₁₋₆-Alkyl, C₁₋₆-Alkoxy oder C₂₋₆-Alkenyl, und
   "Alkenyl" und "Alkenyl*"jeweils unabhängig voneinander C₂₋₆-Alkenyl bedeuten.
- Die Verbindungen der Formeln IX-XII sind vorzugsweise ausgewählt aus den folgenden Formeln, 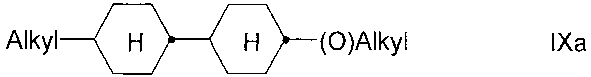 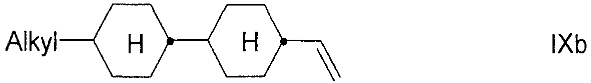 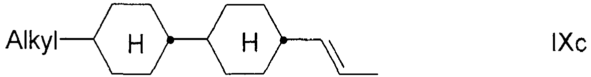 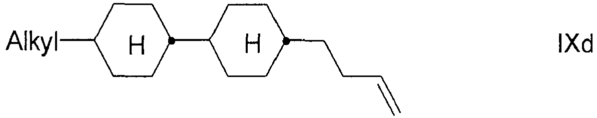 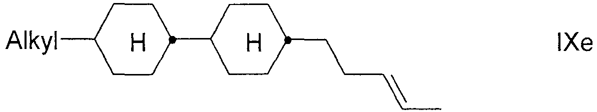 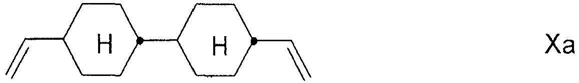 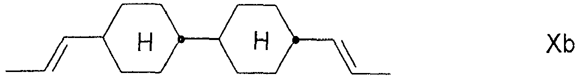 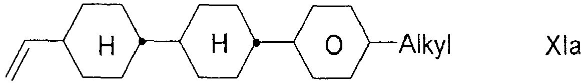 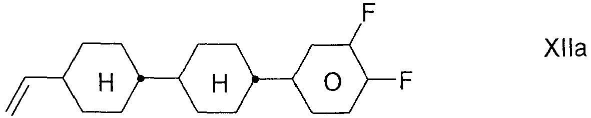
worin "Alkyl" die oben angegebene Bedeutung hat. (O)Alkyl bedeutet "Alkyl" oder "OAlkyl" (= Alkoxy)

Insbesondere bevorzugt sind die Verbindungen der Formeln XIa, IXb, IXc, Xa, Xb, XIa und XIIa. In den Formeln IXb und IXc bedeutet "Alkyl" unabhängig voneinander vorzugsweise n-C₃H₇, n-C₄H₉ oder n-C₅H₁₁, insbesondere n-C₃H₇.
- Das Medium enthält zusätzlich eine oder mehrere Verbindungen ausgewählt aus den folgenden Formeln, 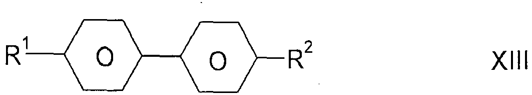 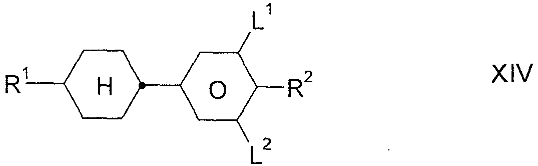 worin L¹ und L² die oben angegebenen Bedeutungen haben, und R¹ und R² jeweils unabhängig voneinander n-Alkyl, Alkoxy, Oxaalkyl, Fluoralkyl oder Alkenyl mit jeweils bis zu 6 C-Atomen bedeuten, und vorzugsweise jeweils unabhängig voneinander Alkyl mit 1 bis 6 C-Atomen bedeuten; In der Verbindung der Formel XIII bedeutet vorzugsweise mindestens einer der Reste R¹ und R² Alkenyl mit 2 bis 6 C-Atomen.
- Das Medium enthält eine oder mehrere Verbindungen der Formel XIII, worin mindestens einer der Reste R¹ und R² Alkenyl mit 2 bis 6 C-Atomen bedeutet, vorzugsweise solche ausgewählt aus folgenden Formeln, 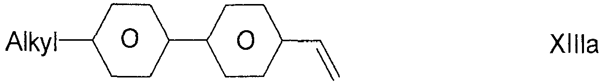 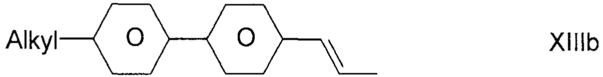 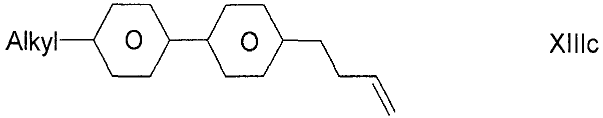 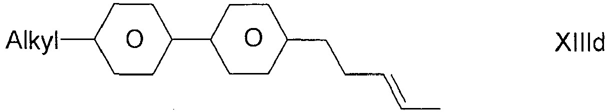 worin "Alkyl" die oben angegebene Bedeutung hat.
- Das Medium enthält eine oder mehrere Verbindungen der folgenden Formeln, 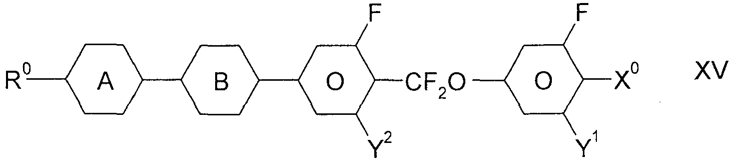 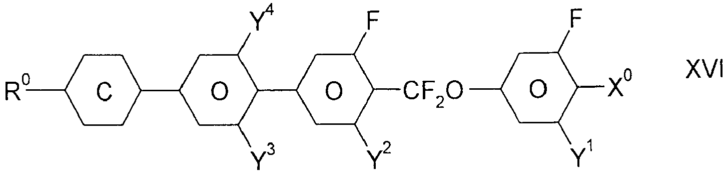 worin R⁰, X⁰ und Y¹⁻⁴ die in Formel I angegebenen Bedeutungen besitzen, und 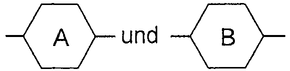 jeweils unabhängig voneinander 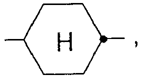 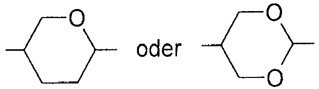 bedeuten, und 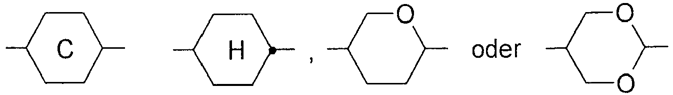 bedeutet;
- Die Verbindungen der Formeln XV und XVI sind vorzugsweise ausgewählt aus den folgenden Formeln, 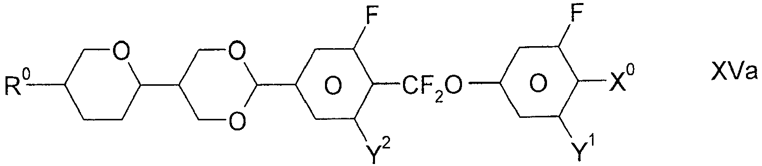 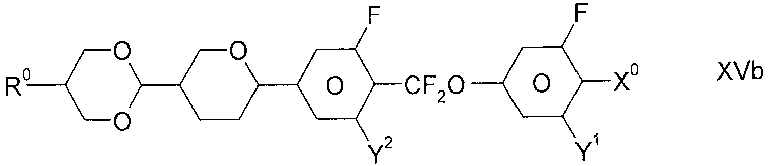 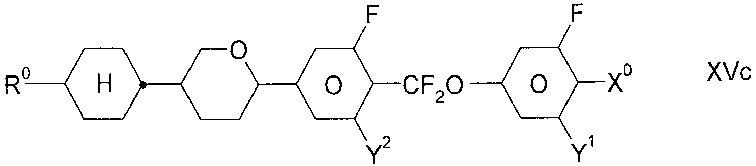 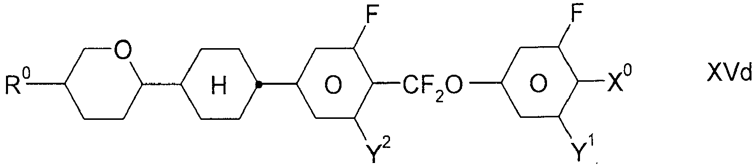 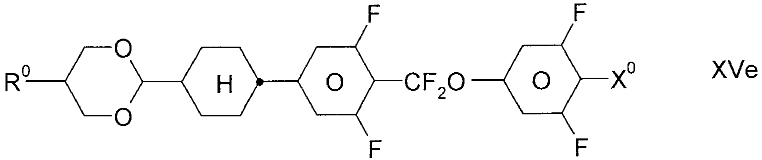 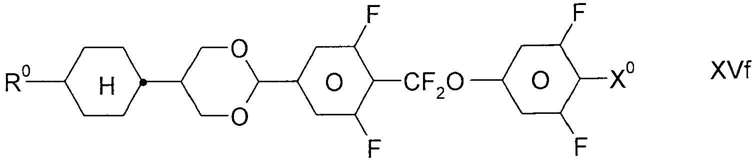 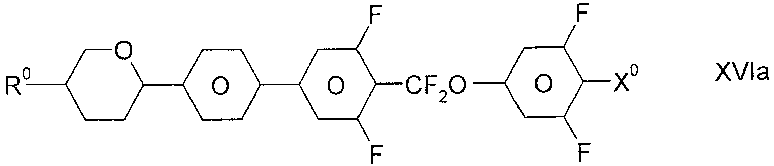 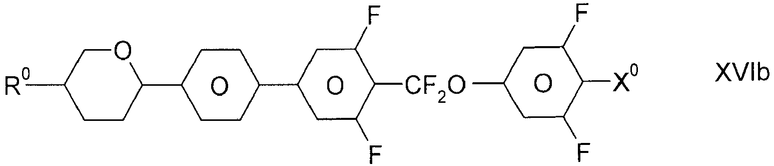 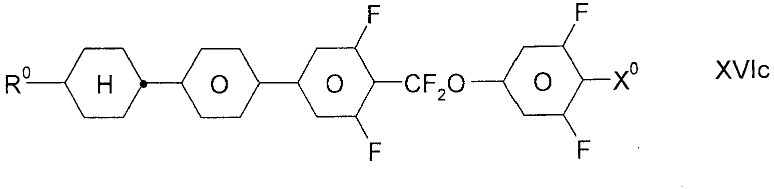 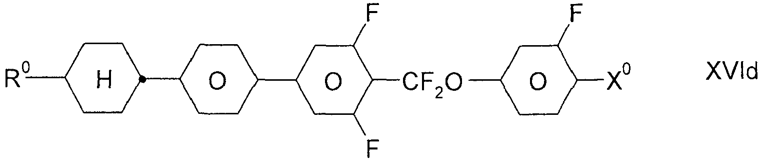 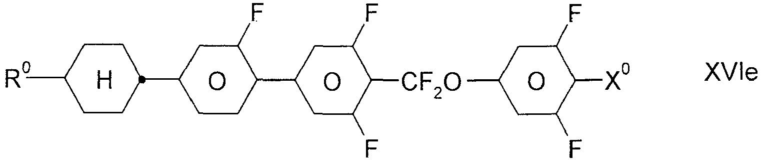 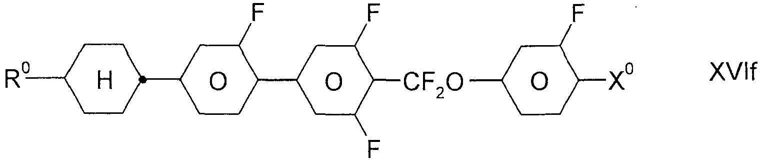
worin R⁰ und X⁰ die oben angegebenen Bedeutungen haben.

Vorzugsweise bedeutet R⁰ Alkyl mit 1 bis 6 C-Atomen. X⁰ bedeutet vorzugsweise F, ferner OCF₃. Besonders bevorzugte Verbindungen der Formeln XV und XVI sowie deren Unterformeln sind solche, worin Y¹ F und Y² H oder F, vorzugsweise F, bedeutet. Besonders bevorzugt enthält die erfindungsgemäße Mischung mindestens eine Verbindung der Formel XVa, XVf und/oder XV1b.
- Das Medium enthält eine oder mehrere Verbindungen der Formel XVII, 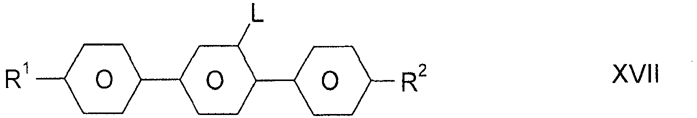 worin R¹ und R² die oben angegebenen Bedeutungen besitzen, und vorzugsweise jeweils unabhängig voneinander Alkyl mit 1 bis 6 C-Atomen bedeuten. L bedeutet H oder F.

Besonders bevorzugte Verbindungen der Formel XVII sind solche der Unterformeln, 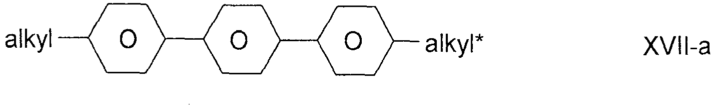 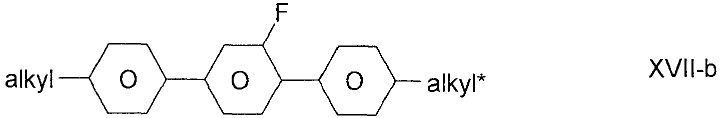 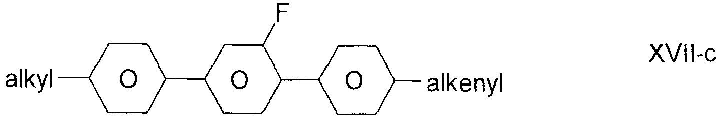 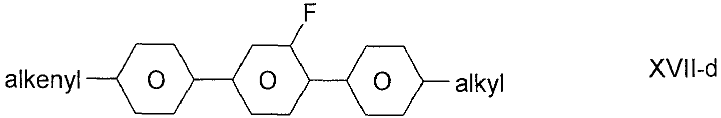 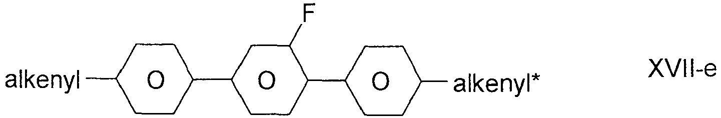 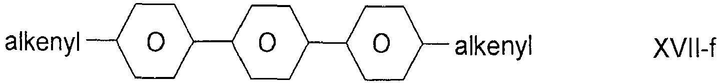 worin
- alkyl und alkyl*: jeweils unabhängig voneinander geradkettiger Alkylrest mit 1-6 C-Atomen, insbesondere Ethyl, Propyl und Pentyl,
- alkenyl und alkenyl*: jeweils unabhängig voneinander geradkettiger Alkenylrest mit 2-6 C-Atomen, insbesondere CH₂=CHC₂H₄, CH₃CH=CHC₂H₄, CH₂=CH und CH₃CH=CH,
bedeuten.

Besonders bevorzugt sind die Verbindungen der Formeln XVII-b und XVII-c. Ganz besonders bevorzugt sind die Verbindungen der Formeln 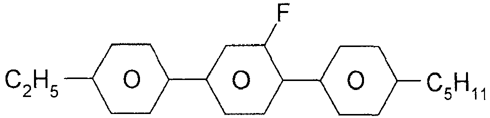 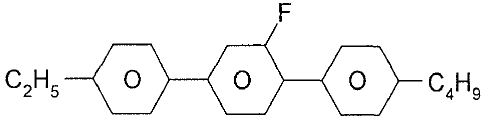 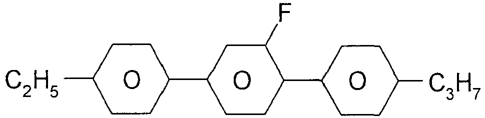 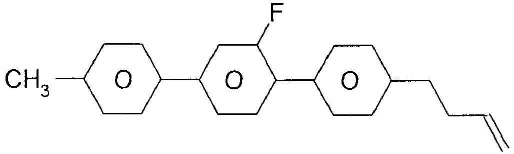 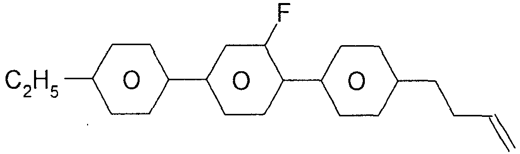 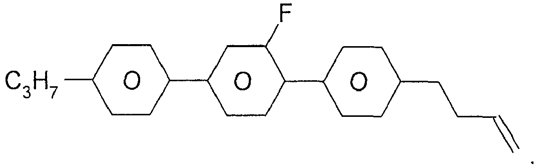
- Das Medium enthält eine oder mehrere Verbindungen der folgenden Formeln, 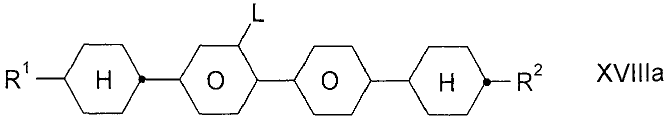 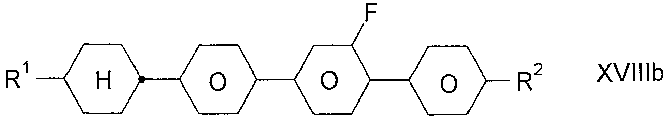 worin R¹ und R² die oben angegebenen Bedeutungen besitzen, und vorzugsweise jeweils unabhängig voneinander Alkyl mit 1 bis 6 C-Atomen bedeuten. L bedeutet H oder F;
- Das Medium enthält zusätzlich eine oder mehrere Verbindungen ausgewählt aus den folgenden Formeln, 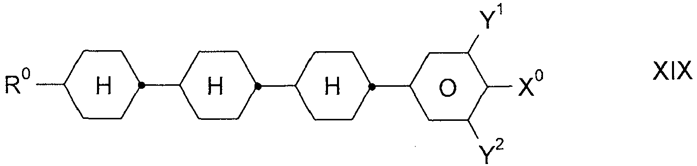 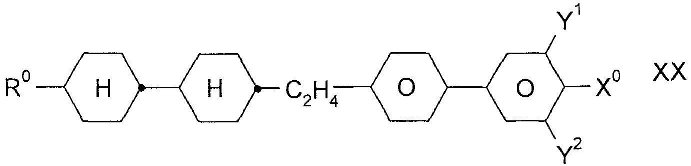 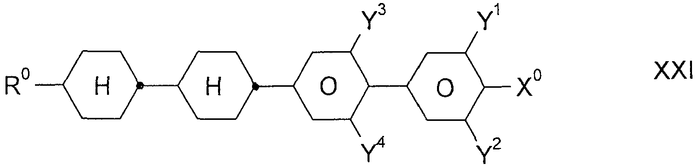 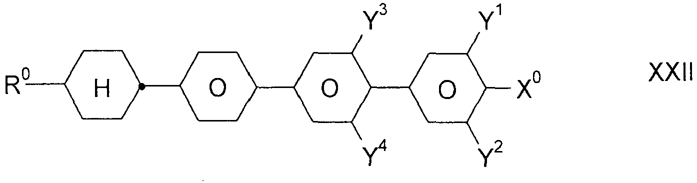 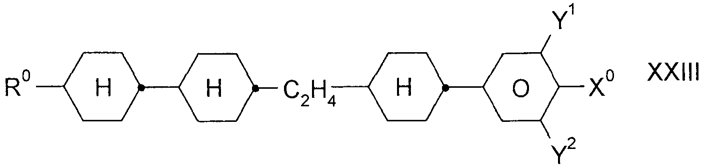 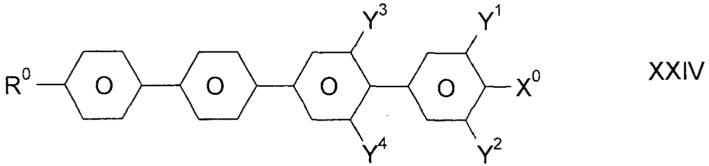 worin R⁰ und X⁰ jeweils unabhängig voneinander eine der oben angegebenen Bedeutungen haben und Y¹⁻⁴ jeweils unabhängig voneinander H oder F bedeuten. X⁰ ist vorzugsweise F, Cl, CF₃, OCF₃ oder OCHF₂. R⁰ bedeutet vorzugsweise Alkyl, Alkoxy, Oxaalkyl, Fluoralkyl oder Alkenyl mit jeweils bis zu 6 C-Atomen.

Besonders bevorzugt erhält die erfindungsgemäße Mischung eine oder mehrere Verbindungen der Formel XXIV-a, 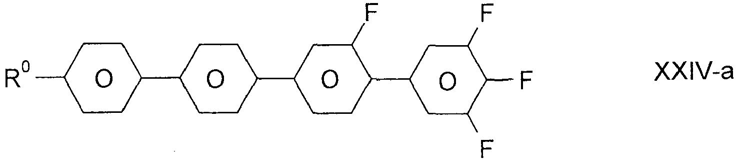 worin R⁰ die oben angegebene Bedeutungen hat. Vorzugsweise bedeutet R⁰ geradkettiges Alkyl, insbesondere Ethyl, n-Propyl, n-Butyl und n-Pentyl, und ganz besonders bevorzugt n-Propyl. Die Verbindung(en) der Formel XXIV, insbesondere der Formel XXIV-a werden vorzugsweise in Mengen von 0,5-20 Gew.%, besonders bevorzugt 1-15 Gew.% in den erfindungsgemäßen Mischungen eingesetzt.
- Das Medium enthält zusätzlich eine oder mehrere Verbindungen aus der Formel XXIV, 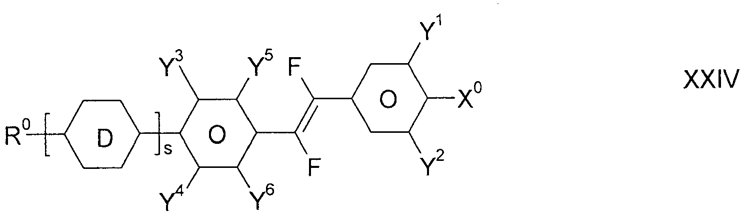 worin R⁰, X⁰ und Y¹⁻⁶ die in Anspruch 3 angegebenen Bedeutungen besitzen, s 0 oder 1, und 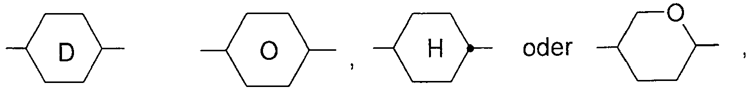 bedeuten.

In der Formel XXIV kann X⁰ auch ein Alkylrest mit 1-6 C-Atomen oder ein Alkoxyrest mit 1-6 C-Atomen bedeuten. Vorzugsweise ist der Alkyl-oder Alkoxyrest geradkettig.

Vorzugsweise bedeutet R⁰ Alkyl mit 1 bis 6 C-Atomen. X⁰ bedeutet vorzugsweise F;
- Die Verbindungen der Formel XXIV sind vorzugsweise ausgewählt aus den folgenden Formeln, 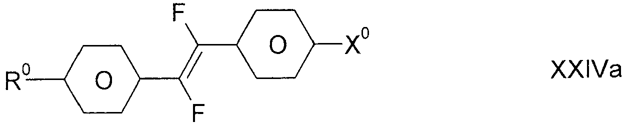 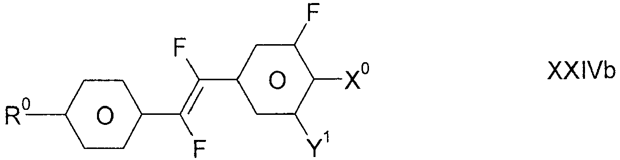 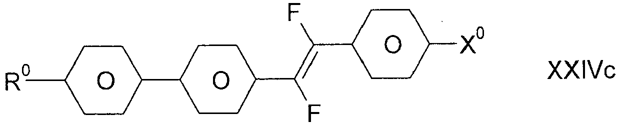 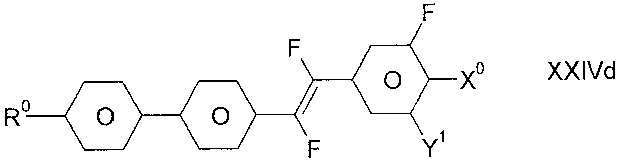 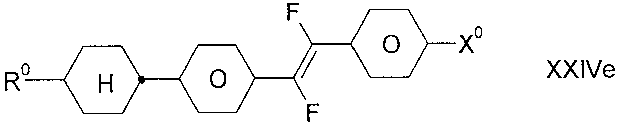 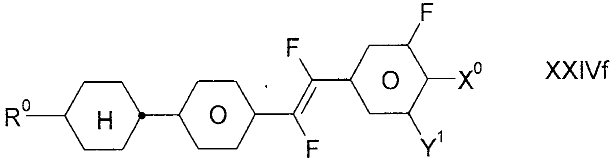 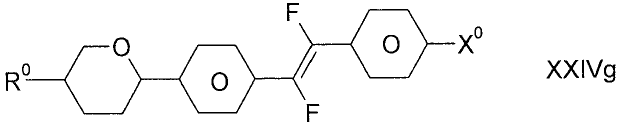 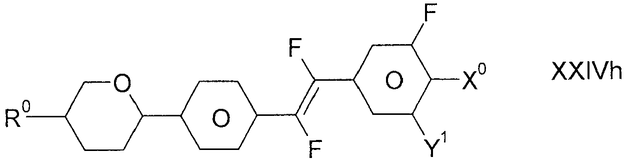 worin R⁰, X⁰ und Y¹ die oben angegebenen Bedeutungen haben. Vorzugsweise bedeutet R⁰ Alkyl mit 1 bis 6 C-Atomen. X⁰ bedeutet vorzugsweise F und Y¹ ist vorzugsweise F; 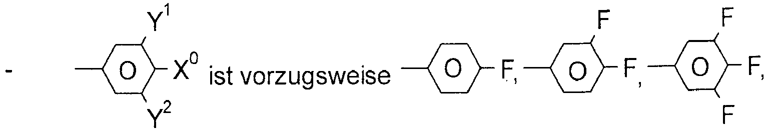 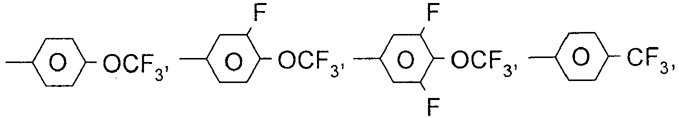 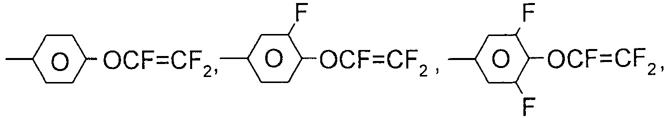 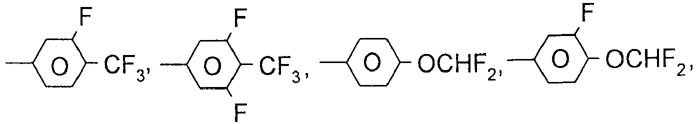 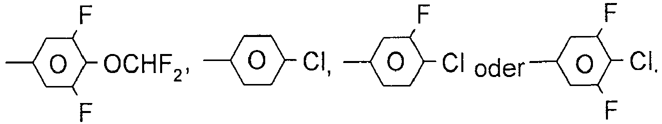
- R⁰ ist geradkettiges Alkyl oder Alkenyl mit 2 bis 6 C-Atomen;
- Das Medium enthält eine oder mehrere Verbindungen der folgenden Formeln 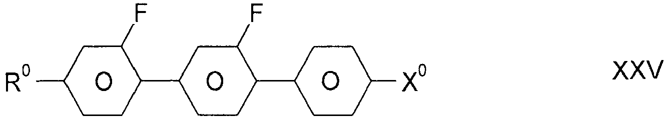 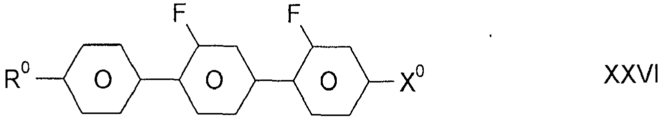 worin R⁰ und X⁰ die oben angegebenen Bedeutungen besitzen. R⁰ bedeutet vorzugsweise Alkyl mit 1 bis 6 C-Atomen. X⁰ bedeutet vorzugsweise F oder Cl. In der Formel XXV bedeutet X⁰ ganz besonders bevorzugt Cl.
- Das Medium enthält eine oder mehrere Verbindungen der folgenden Formeln, 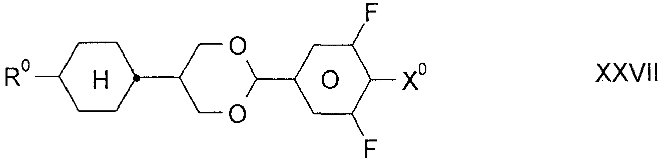 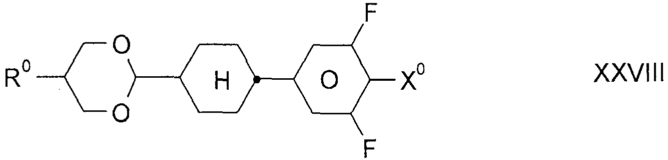 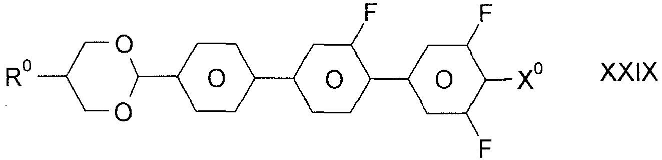 worin R⁰ und X⁰ die oben angegebene Bedeutung besitzen. R⁰ bedeutet vorzugsweise Alkyl mit 1 bis 6 C-Atomen. X⁰ bedeutet vorzugsweise F; Insbesondere bevorzugt enthält das erfindungsgemäße Medium eine oder mehrere Verbindungen der Formel XXIX, worin X⁰ vorzugsweise F bedeutet. Die Verbindung(en) der Formeln XXVII - XXIX werden vorzugsweise in Mengen von 1-20 Gew.%, besonders bevorzugt 1-15 Gew.% in den erfindungsgemäßen Mischungen eingesetzt. Besonders bevorzugte Mischungen enthalten mindestens eine Verbindung der Formel XXIX.
- Das Medium enthält eine oder mehrere Verbindungen der folgenden Pyrimidin- oder Pyridin-Verbindungen der Formeln, 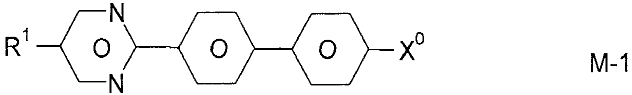 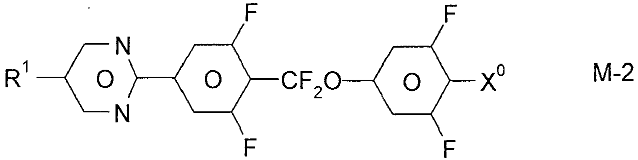 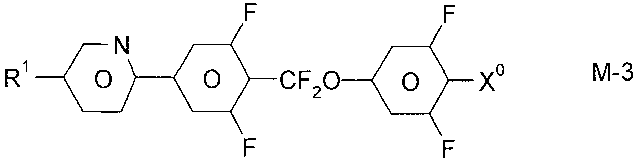 worin R¹ und X⁰ die oben angegebene Bedeutung besitzen. R¹ bedeutet vorzugsweise Alkyl mit 1 bis 6 C-Atomen. X⁰ bedeutet vorzugsweise F; Insbesondere bevorzugt enthält das erfindungsgemäße Medium eine oder mehrere Verbindungen der Formel M-1, worin X⁰ vorzugsweise F bedeutet. Die Verbindung(en) der FormelnM-1 - M-3 werden vorzugsweise in Mengen von 1-20 Gew.%, besonders bevorzugt 1-15 Gew.% in den erfindungsgemäßen Mischungen eingesetzt.

Nachfolgend werden weitere bevorzugte Ausführungsformen angegeben:
- Das Medium enthält zwei oder mehr Verbindungen der Formel IA, insbesondere der Formel IA-g;
- Das Medium enthält 1-30 Gew.%, bevorzugt 1-20 Gew.%, besonders bevorzugt 1-15 Gew.%, an Verbindungen der Formel IA;
- Der Anteil an Verbindungen der Formeln II, III, IX-XIII, XVII und XVIII im Gesamtgemisch beträgt 40 bis 95 Gew.%;
- Das Medium enthält 10-50 Gew.%, besonders bevorzugt 12-40 Gew.% an Verbindungen der Formel II und/oder III;
- Das Medium enthält 20-70 Gew.%, besonders bevorzugt 25-65 Gew.% an Verbindungen der Formeln IX-XIII;
- Das Medium enthält 4-30 Gew.%, besonders bevorzugt 5-20 Gew.% an Verbindungen der Formel XVII;
- Das Medium enthält 1-20 Gew.%, besonders bevorzugt 2-15 Gew.% an Verbindungen der Formel XVIII.
- Das Medium enthält mindestens eine Verbindung der Formeln 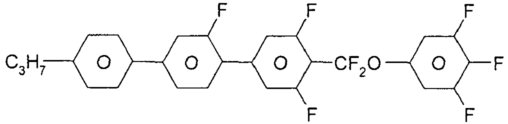 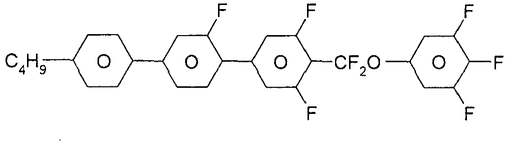 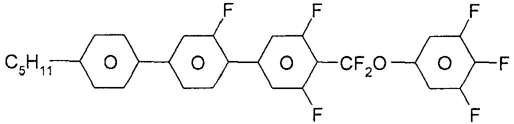
- Das Medium enthält mindestens eine Verbindung der Formeln 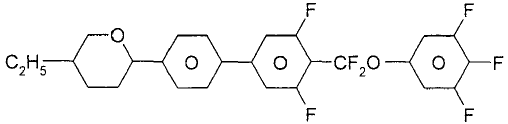 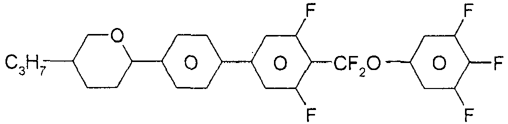 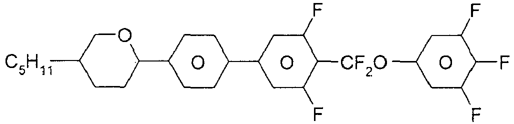
- Das Medium enthält mindestens eine Verbindung der Formel IA und mindestens eine Verbindung der Formel IIIa.
- Das Medium enthält ≥ 20 Gew.%, vorzugsweise ≥ 24 Gew.%, vorzugsweise 25-60 Gew.%, an Verbindungen der Formel IXb, insbesondere die Verbindung der Formel IXb-1, 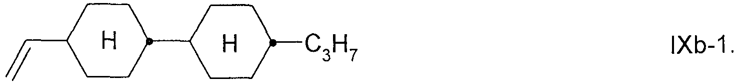
- Das Medium enthält mindestens eine Verbindung der Formel IXb-1 und mindestens eine Verbindung der Formel IXc-1. 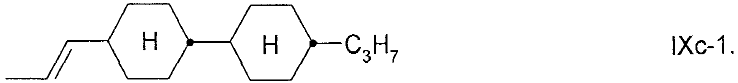
- Das Medium enthält mindestens eine Verbindung der Formel DPGU-n-F.
- Das Medium enthält mindestens eine Verbindung der Formel PPGU-n-F.
- Das Medium enthält mindestens eine Verbindung der Formel PGP-n-m, vorzugsweise zwei oder drei Verbindungen.
- Das Medium enthält mindestens eine Verbindung der Formel PGP-n-2V mit der Struktur

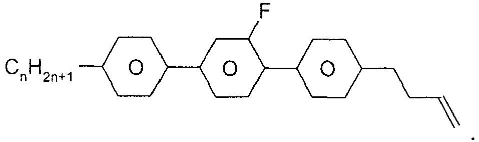

Es wurde gefunden, dass vorzugsweise 1 - 20 Gew.%, vorzugsweise 1 - 15 Gew.% an Verbindungen der Formel IA im Gemisch mit üblichen Flüssigkristallmaterialien, insbesondere jedoch mit einer oder mehreren Verbindungen der Formeln II bis XXVIII zu einer beträchtlichen Erhöhung der Lichtstabilität und zu relativ hohen Werten für die Doppelbrechung führt, wobei gleichzeitig breite nematische Phasen mit tiefen Übergangstemperaturen smektisch-nematisch beobachtet werden, wodurch die Lagerstabilität verbessert wird. Gleichzeitig zeigen die Mischungen sehr niedrige Schwellenspannungen und sehr gute Werte für die VHR bei UV-Belastung und sehr hohe Klärpunkte.

Der Ausdruck "Alkyl" bzw. "Alkyl*" umfasst in dieser Anmeldung geradkettige und verzweigte Alkylgruppen mit 1-6 Kohlenstoffatomen, insbesondere die geradkettigen Gruppen Methyl, Ethyl, Propyl, Butyl, Pentyl und Hexyl. Gruppen mit 2-5 Kohlenstoffatomen sind im allgemeinen bevorzugt.

Der Ausdruck "Alkenyl" bzw. "Alkenyl*" umfasst geradkettige und verzweigte Alkenylgruppen mit 2-6 Kohlenstoffatomen, insbesondere die geradkettigen Gruppen. Bevorzugte Alkenylgruppen sind C₂-C₇-1E-Alkenyl, C₄-C₆-3E-Alkenyl, insbesondere C₂-C₆-1E-Alkenyl. Beispiele besonders bevorzugter Alkenylgruppen sind Vinyl, 1E-Propenyl, 1E-Butenyl, 1E-Pentenyl, 1E-Hexenyl, 3-Butenyl, 3E-Pentenyl, 3E-Hexenyl, 4-Pentenyl, 4Z-Hexenyl, 4E-Hexenyl und 5-Hexenyl. Gruppen mit bis zu 5 Kohlenstoffatomen sind im allgemeinen bevorzugt, insbesondere CH₂=CH, CH₃CH=CH.

Der Ausdruck "Fluoralkyl" umfasst vorzugsweise geradkettige Gruppen mit endständigem Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluors sind jedoch nicht ausgeschlossen.

Der Ausdruck "Oxaalkyl" bzw. "Alkoxy" umfasst vorzugsweise geradkettige Reste der Formel CₙH₂ₙ₊₁-O-(CH₂)ₘ, worin n und m jeweils unabhängig voneinander 1 bis 6 bedeuten. m kann auch 0 bedeuten. Vorzugsweise ist n = 1 und m 1-6 oder m = 0 und n = 1-3.

Durch geeignete Wahl der Bedeutungen von R⁰ und X⁰ können die Ansprechzeiten, die Schwellenspannung, die Steilheit der Transmissionskennlinien etc. in gewünschter Weise modifiziert werden. Beispielsweise führen 1E-Alkenylreste, 3E-Alkenylreste, 2E-Alkenyloxyreste und dergleichen in der Regel zu kürzeren Ansprechzeiten, verbesserten nematischen Tendenzen und einem höheren Verhältnis der elastischen Konstanten k₃₃ (bend) und k₁₁ (splay) im Vergleich zu Alkyl- bzw. Alkoxyresten. 4-Alkenylreste, 3-Alkenylreste und dergleichen ergeben im allgemeinen tiefere Schwellenspannungen und kleinere Werte von k₃₃/k₁₁ im Vergleich zu Alkyl- und Alkoxyresten. Die erfindungsgemäßen Mischungen zeichnen sich insbesondere durch hohe Δε-Werte aus und besitzen somit deutlich schnellere Schaltzeilen als die Mischungen aus dem Stand der Technik.

Das optimale Mengenverhältnis der Verbindungen der oben genannten Formeln hängt weitgehend von den gewünschten Eigenschaften, von der Wahl der Komponenten der oben genannten Formeln und der Wahl weiterer gegebenenfalls vorhandener Komponenten ab.

Geeignete Mengenverhältnisse innerhalb des oben angegebenen Bereichs können von Fall zu Fall leicht ermittelt werden.

Die Gesamtmenge an Verbindungen der oben genannten Formeln in den erfindungsgemäßen Gemischen ist nicht kritisch. Die Gemische können daher eine oder mehrere weitere Komponenten enthalten zwecks Optimierung verschiedener Eigenschaften. Der beobachtete Effekt auf die gewünschte Verbesserung der Eigenschaften der Mischung ist jedoch in der Regel umso größer je höher die Gesamtkonzentration an Verbindungen der oben genannten Formeln ist.

In einer besonders bevorzugten Ausführungsform enthalten die erfindungsgemäßen Medien Verbindungen der Formel II bis VIII (vorzugsweise II, III, IV und V, insbesondere IIa und IIIa), worin X⁰ F, OCF₃, OCHF₂, OCH=CF₂, OCF=CF₂ oder OCF₂-CF₂H bedeutet.

Die einzelnen Verbindungen der oben genannten Formeln und deren Unterformeln, die in den erfindungsgemäßen Medien verwendet werden können, sind entweder bekannt, oder sie können analog zu den bekannten Verbindungen hergestellt werden.

Gegenstand der Erfindung sind auch elektrooptische Anzeigen, wie z. B. STN- oder MFK-Anzeigen mit zwei planparallelen Trägerplatten, die mit einer Umrandung eine Zelle bilden, integrierten nicht-linearen Elementen zur Schaltung einzelner Bildpunkte auf den Trägerplatten und einer in der Zelle befindlichen nematischen Flüssigkristallmischung mit positiver dielektrischer Anisotropie und hohem spezifischem Widerstand), die derartige Medien enthalten sowie die Verwendung dieser Medien für elektrooptische Zwecke.

Die erfindungsgemäßen Flüssigkristallmischungen ermöglichen eine bedeutende Erweiterung des zur Verfügung stehenden Parameterraumes. Die erzielbaren Kombinationen aus Klärpunkt, Viskosität bei tiefer Temperatur, thermischer und UV-Stabilität und hoher optischer Anisotropie übertreffen bei weitem bisherige Materialien aus dem Stand der Technik.

Die erfindungsgemäßen Mischungen sind insbesondere für mobile Anwendungen und TFT-Anwendungen, wie z. B. Mobiltelefone und PDAs geeignet. Weiterhin können die erfindungsgemäßen Mischungen in FFS-, VA-IPS, OCB- und IPS-Anzeigen Anwendung finden.

Die erfindungsgemäßen Flüssigkristallmischungen ermöglichen es, bei Beibehaltung der nematischen Phase bis -20 °C und bevorzugt bis -30 °C, besonders bevorzugt bis -40 °C, und des Klärpunkts ≥ 75 °C, vorzugsweise ≥ 80 °C, gleichzeitig Rotationsviskositäten γ₁ von ≤ 110 mPa·s, besonders bevorzugt ≤ 100 mPa·s zu erreichen, wodurch hervorragende MFK-Anzeigen mit schnellen Schaltzeiten erzielt werden können. Die Rotationsviskositäten sind bei 20 °C bestimmt.

Die dielektrische Anisotropie der erfindungsgemäßen Flüssigkristallmischungen Δε ist bei 20 °C vorzugsweise ≥ +7, besonders bevorzugt ≥ +8, insbesondere bevorzugt ≥ 10. Die Mischungen sind außerdem durch kleine Operationsspannungen gekennzeichnet. Die Schwellenspannung der erfindungsgemäßen Flüssigkristallmischungen ist vorzugsweise ≤ 2.0 V. Die Doppelbrechung Δn der erfindungsgemäßen Flüssigkristallmischungen ist bei 20 °C vorzugsweise ≥ 0,09, besonders bevorzugt ≥ 0,10.

Der nematische Phasenbereich der erfindungsgemäßen Flüssigkristallmischungen ist vorzugsweise mindestens 90°, insbesondere mindestens 100° breit. Vorzugsweise erstreckt sich dieser Bereich mindestens von -25° bis +70°C.

Es versteht sich, dass durch geeignete Wahl der Komponenten der erfindungsgemäßen Mischungen auch höhere Klärpunkte (z.B. oberhalb 100 °C) bei höheren Schwellenspannungen oder niedrigere Klärpunkte bei niedrigeren Schwellenspannungen unter Erhalt der anderen vorteilhaften Eigenschaften realisiert werden können. Ebenso können bei entsprechend wenig erhöhten Viskositäten Mischungen mit größerem Δε und somit geringen Schwellen erhalten werden. Die erfindungsgemäßen MFK-Anzeigen arbeiten vorzugsweise im ersten Transmissionsminimum nach Gooch und Tarry [C.H. Gooch und H.A. Tarry, Electron. Lett. 10, 2-4, 1974; C.H. Gooch und H.A. Tarry, Appl. Phys., Vol. 8, 1575-1584, 1975], wobei hier neben besonders günstigen elektrooptischen Eigenschaften, wie z.B. hohe Steilheit der Kennlinie und geringe Winkelabhängigkeit des Kontrastes (DE-PS 30 22 818) bei gleicher Schwellenspannung wie in einer analogen Anzeige im zweiten Minimum, eine kleinere dielektrische Anisotropie ausreichend ist. Hierdurch lassen sich unter Verwendung der erfindungsgemäßen Mischungen im ersten Minimum deutlich höhere spezifische Widerstände verwirklichen als bei Mischungen mit Cyanverbindungen. Der Fachmann kann durch geeignete Wahl der einzelnen Komponenten und deren Gewichtsanteilen mit einfachen Routinemethoden die für eine vorgegebene Schichtdicke der MFK-Anzeige erforderliche Doppelbrechung einstellen.

Messungen des "Voltage Holding-ratio" (HR) [S. Matsumoto et al., Liquid Crystals 5, 1320 (1989); K. Niwa et al., Proc. SID Conference, San Francisco, June 1984, p. 304 (1984); G. Weber et al., Liquid Crystals 5, 1381 (1989)] haben ergeben, dass erfindungsgemäße Mischungen enthaltend eine oder mehrere Verbindungen der Formel IA eine deutlich geringere Abnahme des HR unter UV-Belastung aufweisen als analoge Mischungen enthaltend anstelle einer oder mehrerer Verbindungen der Formel IA Cyanophenylcyclohexane der Formel 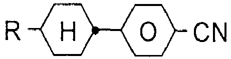 oder Ester der Formel

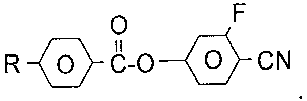

Die Lichtstabilität und UV-Stabilität der erfindungsgemäßen Mischungen ist erheblich besser, d.h. sie zeigen eine deutlich kleinere Abnahme des HR unter Licht- bzw. UV-Belastung.

Der Aufbau der erfindungsgemäßen MFK-Anzeige aus Polarisatoren, Elektrodengrundplatten und Elektroden mit Oberflächenbehandlung entspricht der für derartige Anzeigen üblichen Bauweise. Dabei ist der Begriff der üblichen Bauweise hier weit gefasst und umfasst auch alle Abwandlungen und Modifikationen der MFK-Anzeige, insbesondere auch Matrix-Anzeigeelemente auf Basis poly-Si TFT oder MIM.

Ein wesentlicher Unterschied der erfindungsgemäßen Anzeigen zu den bisher üblichen auf der Basis der verdrillten nematischen Zelle besteht jedoch in der Wahl der Flüssigkristallparameter der Flüssigkristallschicht.

Die Herstellung der erfindungsgemäß verwendbaren Flüssigkristallmischungen erfolgt in an sich üblicher Weise, beispielsweise indem man eine oder mehrere Verbindungen der Formel IA mit einer oder mehreren Verbindungen der Formeln II-XXVIII oder mit weiteren flüssigkristallinen Verbindungen und/oder Additiven mischt. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation.

Die Dielektrika können auch weitere, dem Fachmann bekannte und in der Literatur beschriebene Zusätze, wie z. B. UV-Stabilisatoren wie Tinuvin®, z.B. Tinuvin® 770, der Fa. Ciba Chemicals, Antioxidantien, z.B. TEMPOL, Mikropartikel, Radikalfänger, Nanopartikel, etc. enthalten. Beispielsweise können 0-15 % pleochroitische Farbstoffe oder chirale Dotierstoffe zugesetzt werden. Geeignete Stabilisatoren und Dotierstoffe werden nachfolgend in den Tabellen C und D genannt.

Den erfindungsgemäßen Mischungen können weiterhin polymerisierbare Verbindungen, sogenannte reaktive Mesogene (RMs), beispielsweise wie in U.S. 6,861,107 offenbart, in Konzentrationen von bevorzugt 0,12 - 5 Gew.%, besonders bevorzugt 0,2 - 2 % bezogen auf die Mischung, zugesetzt werden. Optional können diese Mischungen auch einen Initiator enthalten, wie beispielsweise in der U.S 6,781,665 beschrieben. Der Initiator, z.B. Irganox-1076 der Fa. Ciba, wird vorzugsweise der Mischung enthaltend polymerisierbare Verbindungen in Mengen von 0-1 % zugesetzt. Derartige Mischungen können für sogenannte Polymer Stabilized (PS) Modes, bei denen eine Polymerisierung der reaktiven Mesogene in der flüssigkristallinen Mischung erfolgen soll, verwendet werden, z.B. für PS-IPS-, PS-FFS, PS-TN, PS-VA-IPS. Voraussetzung hierfür ist, dass die Flüssigkristallmischung selbst keine polymerisierbaren Komponenten enthält.

In einer bevorzugten Ausführungsform der Erfindung sind die polymerisierbaren Verbindungen ausgewählt aus den Verbindungen der Formel M

R^{Ma}-A^{M1}-(Z^{M1}-A^{M2})ₘ₁-R^{Mb} M

worin die einzelnen Reste folgende Bedeutung haben:
- R^{Ma} und R^{Mb}: jeweils unabhängig voneinander P, P-Sp-, H, Halogen, SF₅, NO₂, eine Alkyl-, Alkenyl- oder Alkinylgruppe, wobei bevorzugt mindestens einer der Reste R^{Ma} und R^{Mb} eine Gruppe P oder P-Sp- bedeutet oder enthält,
- P: eine polymerisierbare Gruppe,
- Sp: eine Abstandsgruppe oder eine Einfachbindung,
- A^{M1} und A^{M2}: jeweils unabhängig voneinander eine aromatische, heteroaromatische, alicyclische oder heterocyclische Gruppe, vorzugsweise mit 4 bis 25 Ringatomen, bevorzugt C-Atomen, welche auch anellierte Ringe umfasst oder enthalten kann, und die optional ein- oder mehrfach durch L substitutiert sein kann,
- L: P, P-Sp-, OH, CH₂OH, F, Cl, Br, I, -CN, -NO₂, -NCO, -NCS, -OCN, -SCN, -C(=O)N(R^{x})₂, -C(=O)Y¹, -C(=O)R^{x}, -N(R^{x})₂, optional substituiertes Silyl, optional substituiertes Aryl mit 6 bis 20 C Atomen, oder geradkettiges oder verzweigtes Alkyl, Alkoxy, Alkylcarbonyl, Alkoxycarbonyl, Alkylcarbonlyoxy oder Alkoxycarbonyloxy mit 1 bis 25 C-Atomen, worin auch ein oder mehrere H-Atome durch F, Cl, P oder P-Sp- ersetzt sein können, bevorzugt P, P-Sp-, H, OH, CH₂OH, Halogen, SF₅, NO₂, eine Alkyl-, Alkenyl- oder Alkinylgruppe,
- Y¹: Halogen,
- Z^{M1}: -O-, -S-, -CO-, -CO-O- -OCO-. -O-CO-O-, -OCH₂-, -CH₂O-, -SCH₂-, -CH₂S-, -CF₂O-, -OCF₂-, -CF₂S-, -SCF₂-, -(CH₂)ₙ₁-, -CF₂CH₂-, -CH₂CF₂-, -(CF₂)ₙ₁-, -CH=CH-, -CF=CF-, -C≡C-, -CH=CH-, -COO-, -OCO-CH=CH-, CR⁰R⁰⁰ oder eine Einfachbindung,
- R⁰ und R⁰⁰: jeweils unabhängig voneinander H oder Alkyl mit 1 bis 12 C-Atomen,
- R^{x}: P, P-Sp-, H, Halogen, geradkettiges, verzweigtes oder cyclisches Alkyl mit 1 bis 25 C-Atomen, worin auch eine oder mehrere nicht benachbarte CH₂-Gruppen durch -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, P oder P-Sp- ersetzt sein können, eine optional substituierte Aryl- oder Aryloxygruppe mit 6 bis 40 C-Atomen, oder eine optional substituierte Heteroaryl- oder Heteroaryloxygruppe mit 2 bis 40 C-Atomen,
- m1: 0, 1, 2, 3 oder 4, und
- n1: 1, 2, 3 oder 4,
wobei mindestens einer, bevorzugt einer, zwei oder drei, besonders bevorzugt einer oder zwei, aus der Gruppe R^{Ma}, R^{Mb} und der vorhandenen Substituenten L eine Gruppe P oder P-Sp- bedeutet oder mindestens eine Gruppe P oder P-Sp- enthält.

Besonders bevorzugte Verbindungen der Formel M sind solche, worin
- R^{Ma} und R^{Mb}: jeweils unabhängig voneinander P, P-Sp-, H, F, Cl, Br, I, -CN, - NO₂, -NCO, -NCS, -OCN, -SCN, SF₅ oder geradkettiges oder verzweigtes Alkyl mit 1 bis 25 C-Atomen, worin auch eine oder mehrere nicht benachbarte CH₂-Gruppen jeweils unabhängig voneinander durch -C(R⁰)=C(R⁰⁰)-, -C≡C-, -N(R⁰⁰)-, -O-, -S-, - CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, Br, I, CN, P oder P-Sp- ersetzt sein können, wobei bevorzugt mindestens einer der Reste R^{Ma} und R^{Mb} eine Gruppe P oder P-Sp- bedeutet oder enthält,
- A^{M1} und A^{M2}: jeweils unabhängig voneinander 1,4-Phenylen, Naphthalin-1,4-diyl, Naphthalin-2,6-diyl, Phenanthren-2,7-diyl, Anthracen-2,7-diyl, Fluoren-2,7-diyl, Cumarin, Flavon, wobei in diesen Gruppen auch eine oder mehrere CH-Gruppen durch N ersetzt sein können, Cyclohexan-1,4-diyl, worin auch eine oder mehrere nicht-benachbarte CH₂-Gruppen durch O und/oder S ersetzt sein können, 1,4-Cyclohexenylen, Bicyclo[1.1.1]pentan-1,3-diyl, Bicyclo[2.2.2]octan-1,4-diyl, Spiro[3.3]heptan-2,6-diyl, Piperidin-1,4-diyl, Decahydronaphthalin-2,6-diyl, 1,2,3,4-Tetrahydronaphthalin-2,6-diyl, Indan-2,5-diyl oder Octahydro-4,7-methano-indan-2,5-diyl, wobei alle diese Gruppen unsubstituiert oder durch L ein- oder mehrfach substituiert sein können,
- L: P, P-Sp-, OH, CH₂OH, F, Cl, Br, I, -CN, -NO₂, -NCO, -NCS, - OCN, -SCN, -C(=O)N(R^{x})₂, -C(=O)Y¹, -C(=O)R^{x}, -N(R^{x})₂, optional substituiertes Silyl, optional substituiertes Aryl mit 6 bis 20 C Atomen, oder geradkettiges oder verzweigtes Alkyl, Alkoxy, Alkylcarbonyl, Alkoxycarbonyl, Alkylcarbonlyoxy oder Alkoxycarbonyloxy mit 1 bis 25 C-Atomen, worin auch ein oder mehrere H-Atome durch F, Cl, P oder P-Sp- ersetzt sein können,
- P: eine polymerisierbare Gruppe,
- Y¹: Halogen,
- R^{x}: P, P-Sp-, H, Halogen, geradkettiges, verzweigtes oder cyclisches Alkyl mit 1 bis 25 C-Atomen, worin auch eine oder mehrere nicht benachbarte CH₂-Gruppen durch -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, P oder P-Sp- ersetzt sein können, eine optional substituierte Aryl-oder Aryloxygruppe mit 6 bis 40 C-Atomen, oder eine optional substituierte Heteroaryl- oder Heteroaryloxygruppe mit 2 bis 40 C-Atomen,
bedeuten.

Ganz besonders bevorzugt sind Verbindungen der Formel M, worin einer von R^{Ma} und R^{Mb} oder beide P oder P-Sp- bedeuten.

Geeignete und bevorzugte RMs für die Verwendung in erfindungs-gemäßen flüssigkristallinen Medien und PS-Mode-Anzeigen, sind beispielsweise ausgewählt aus den folgenden Formeln: 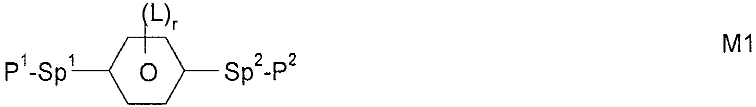 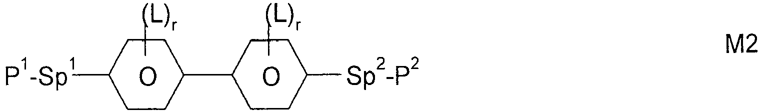 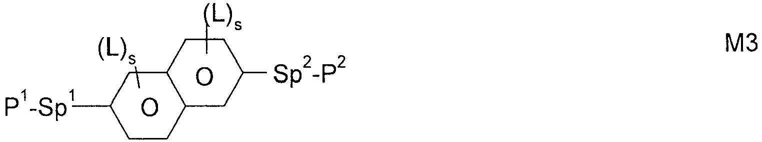 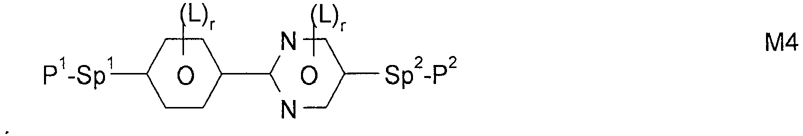 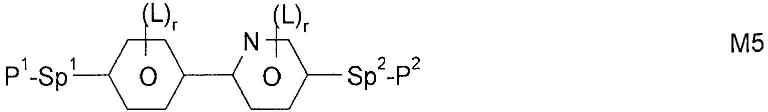 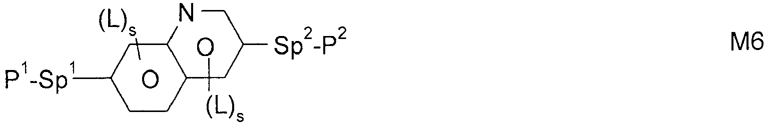 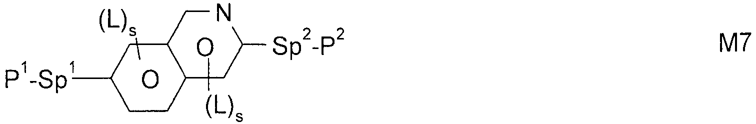 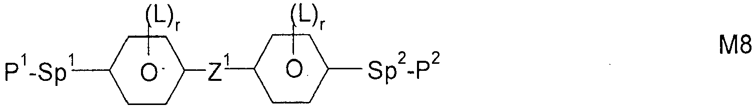 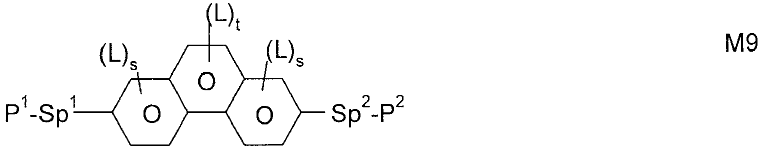 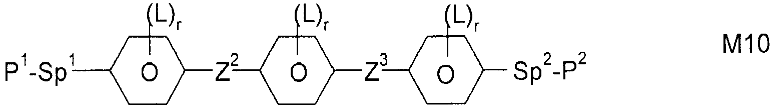 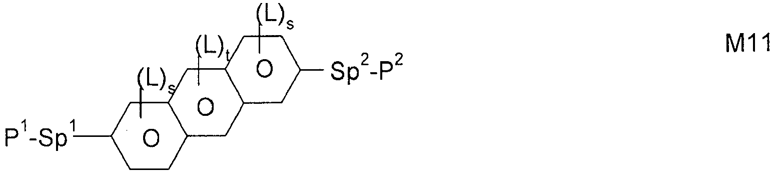 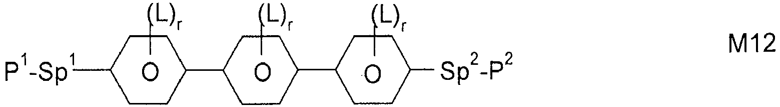 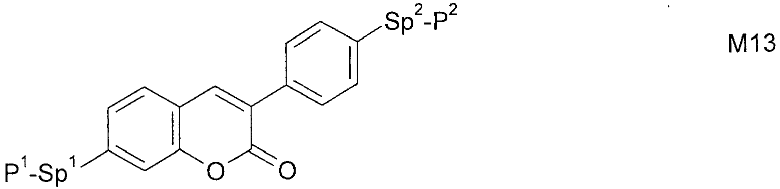 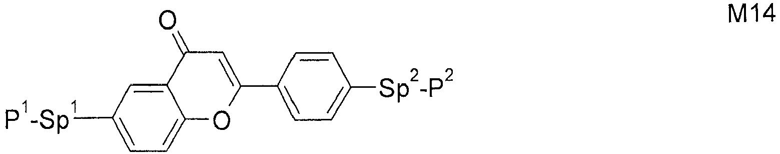 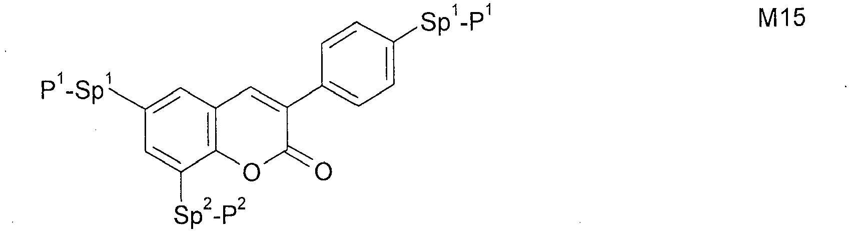 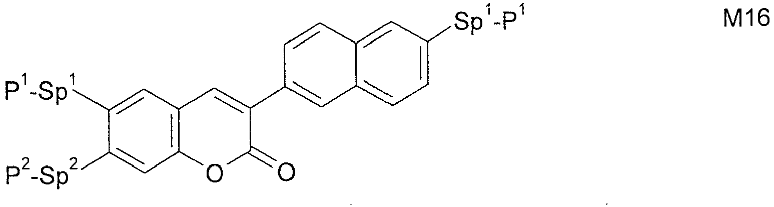 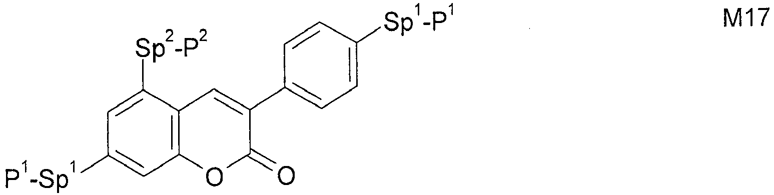 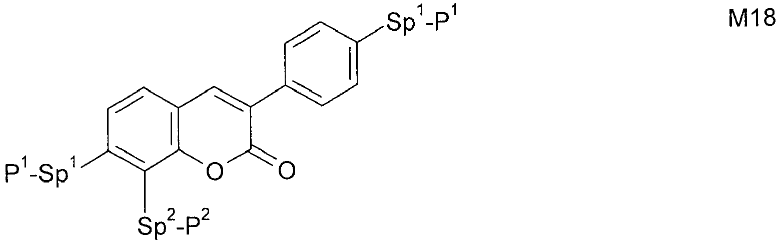 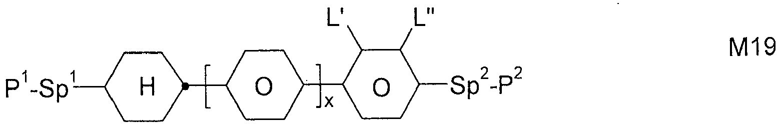 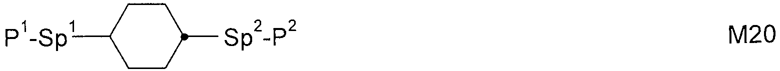 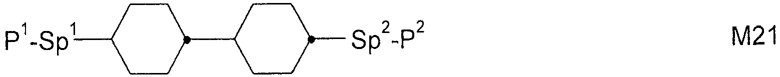 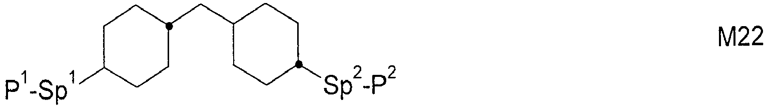 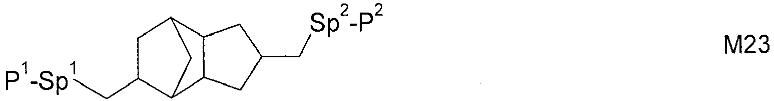 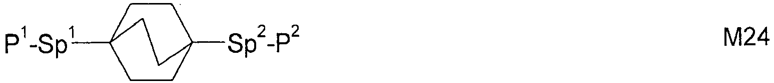 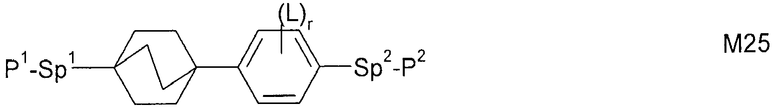 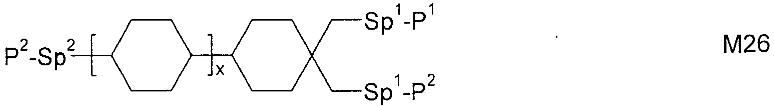 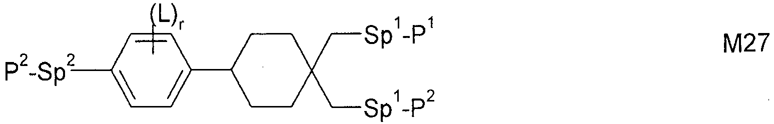 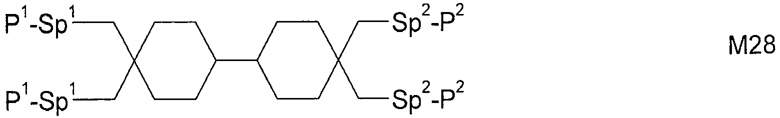 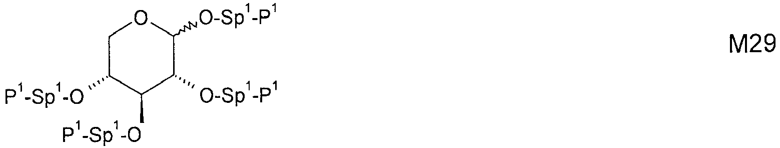 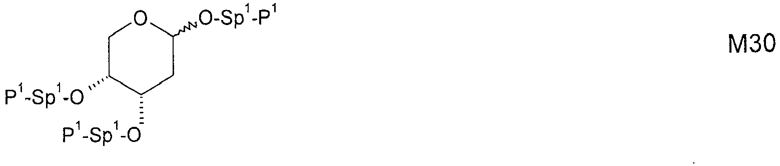 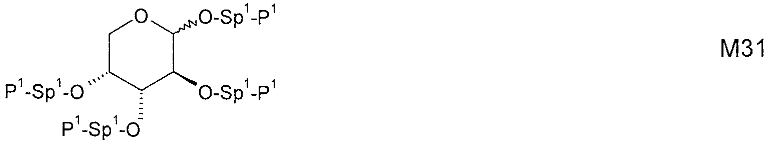 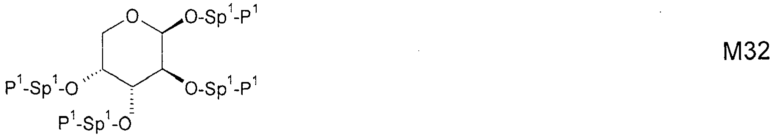 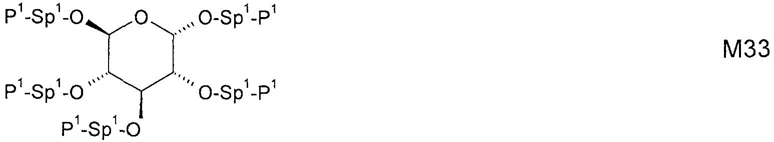 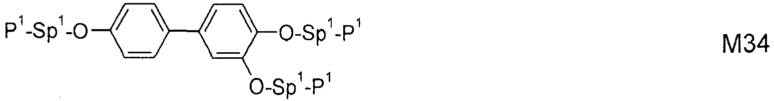 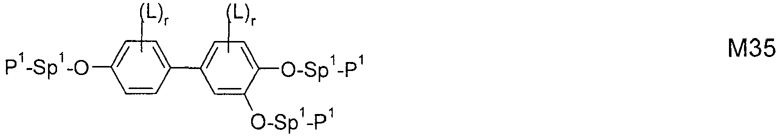 worin die einzelnen Reste folgende Bedeutung besitzen:
- P¹ und P²: jeweils unabhängig voneinander eine polymerisierbare Gruppe, vorzugsweise mit einer der vor- und nachstehend für P angegebenen Bedeutungen, besonders bevorzugt eine Acrylat-, Methacrylat-, Fluoracrylat-, Oxetan-, Vinyloxy- oder Epoxygruppe,
- Sp¹ und Sp²: jeweils unabhängig voneinander eine Einfachbindung oder eine Abstandsgruppe, vorzugsweise mit einer der vor- und nachstehend für Sp angegebenen Bedeutungen, und besonders bevorzugt -(CH₂)ₚ₁-, -(CH₂)ₚ₁-O-, -(CH₂)ₚ₁-CO-O- oder-(CH₂)ₚ₁-O-CO-O- bedeuten, worin p1 eine ganze Zahl von 1 bis 12 ist, und wobei in den letztgenannten Gruppen die Verknüpfung zur benachbarten Ring über das O-Atom erfolgt, wobei einer der Reste P¹-Sp¹- und P²-Sp²- auch R^{aa} bedeuten kann,
- R^{aa}: H, F, Cl, CN oder geradkettiges oder verzweigtes Alkyl mit 1 bis 25 C-Atomen, worin auch eine oder mehrere nicht benachbarte CH₂-Gruppen jeweils unabhängig voneinander durch C(R⁰)=C(R⁰⁰)-, -C≡C-, -N(R⁰)-, -O-, -S-, -CO-, -CO-O-, -O-CO-, -O-CO-O- so ersetzt sein können, dass O- und/oder S-Atome nicht direkt miteinander verknüpft sind, und worin auch ein oder mehrere H-Atome durch F, Cl, CN oder P¹-Sp¹-ersetzt sein können, besonders bevorzugt geradkettiges oder verzweigtes, optional ein- oder mehrfach fluoriertes, Alkyl, Alkoxy, Alkenyl, Alkinyl, Alkylcarbonyl, Alkoxycarbonyl, oder Alkylcarbonyloxy mit 1 bis 12 C-Atomen (wobei die Alkenyl-und Alkinylreste mindestens zwei und die verzweigten Reste mindestens drei C-Atome aufweisen),
- R⁰, R⁰⁰: jeweils unabhängig voneinander und bei jedem Auftreten gleich oder verschieden H oder Alkyl mit 1 bis 12 C-Atomen,
- R^{y} und R^{z}: jeweils unabhängig voneinander H, F, CH₃ oder CF₃,
- Z^{M1}: -O-, -CO-, -C(R^{y}R^{z})-,oder -CF₂CF₂-,
- Z^{M2} und Z^{M3}: jeweils unabhängig voneinander-CO-O-, -O-CO-, -CH₂O-, -OCH₂-, -CF₂O-, -OCF₂-, oder -(CH₂)ₙ-, wobei n 2, 3 oder 4 ist,
- L: bei jedem Auftreten gleich oder verschieden F, Cl, CN, oder geradkettiges oder verzweigtes, optional ein- oder mehrfach fluoriertes, Alkyl, Alkoxy, Alkenyl, Alkinyl, Alkylcarbonyl, Alkoxycarbonyl, oder Alkylcarbonyloxy mit 1 bis 12 C-Atomen vorzugsweise F,
- L' und L": jeweils unabhängig voneinander H, F oder Cl,
- r: 0, 1, 2, 3 oder 4,
- s: 0, 1, 2 oder 3,
- t: 0, 1 oder 2, und
- x: 0 oder 1.

Geeignete polymerisierbare Verbindungen sind beispielsweise in Tabelle E gelistet.

Solche polymerisierbaren Verbindungen sind auch in der
DE 10 2008 035718 A1 zur Verwendung in Flüssikristallanzeigen basierend auf flüssigkristallinen Medien mit negativer dielektrischer Anisotropie beschrieben.

Bevorzugt enthalten die flüssigkristallinen Medien gemäß der vorliegenden Anmeldung insgesamt 0,01 bis 10 %, bevorzugt 0,2 bis 4,0 %, besonders bevorzugt 0,2 bis 2,0 %, an polymerisierbaren Verbindungen.

Insbesondere bevorzugt sind die polymerisierbaren Verbindungen der Formel M.

Gegenstand der vorliegenden Erfindung ist somit auch die Verwendung der erfindungsgemäßen Mischungen in elektrooptischen Anzeigen sowie der Einsatz der erfindungsgemäßen Mischungen in Shutter-Brillen, insbesondere für 3D-Anwendungen, und in TN-, PS-TN-, STN-, TN-TFT-, OCB-, IPS-, PS-IPS-, FFS-, PS-FFS- und PS-VA-IPS-Anzeigen.

In der vorliegenden Anmeldung und in den folgenden Beispielen sind die Strukturen der Flüssigkristallverbindungen durch Acronyme angegeben, wobei die Transformation in chemische Formeln gemäß Tabelle A erfolgt. Alle Reste CₙH₂ₙ₊₁ und CₘH₂ₘ₊₁ sind geradkettige Alkylreste mit n bzw. m C-Atomen; n, m und k sind ganze Zahlen und bedeuten vorzugsweise 0, 1, 2, 3, 4, 5, 6, 7, 8, 9, 10, 11 oder 12. Die Codierung gemäß Tabelle B versteht sich von selbst. In Tabelle A ist nur das Acronym für den Grundkörper angegeben. Im Einzelfall folgt getrennt von Acronym für den Grundkörper mit einem Strich ein Code für die Substituenten R^{1*}, R^{2*}, L^{1*} und L^{2*}:

| Code für R^{1*}, R^{2*}, L^{1*}, L^{2*}, L^{3*} | R^{1*} | R^{2*} | L^{1*} | L^{2*} |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | F | H |
| nN.F.F | CₙH₂ₙ₊₁ | CN | F | F |
| nF | CₙH₂ₙ₊₁ | F | H | H |
| nCl | CₙH₂ₙ₊₁ | Cl | H | H |
| nOF | OCₙH₂ₙ₊₁ | F | H | H |
| nF.F | CₙH₂ₙ₊₁ | F | F | H |
| nF.F.F | CₙH₂ₙ₊₁ | F | F | F |
| nOCF₃ | CₙH₂ₙ₊₁ | OCF₃ | H | H |
| nOCF₃.F | CₙH₂ₙ₊₁ | OCF₃ | F | H |
| n-Vm | CₙH₂ₙ₊₁ | -CH=CH-CₘH₂ₘ₊₁ | H | H |
| nV-Vm | CₙH₂ₙ₊₁-CH=CH- | -CH=CH-CₘH₂ₘ₊₁ | H | H |

Bevorzugte Mischungskomponenten finden sich in den Tabellen A und B.

**Tabelle A**

| | |
|---|---|
| 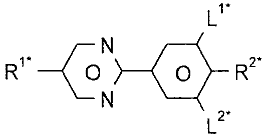 | 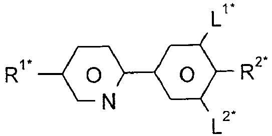 |
| **PYP** | **PYRP** |
| 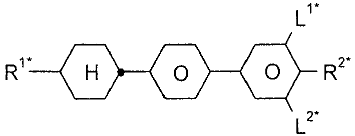 | 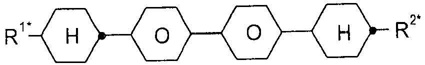 |
| **BCH** | **CBC** |
| 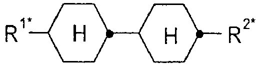 | 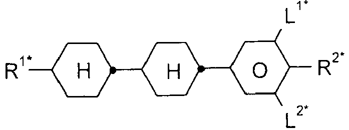 |
| **CCH** | **CCP** |
| 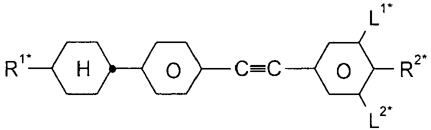 | 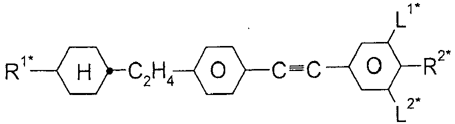 |
| **CPTP** | **CEPTP** |
| 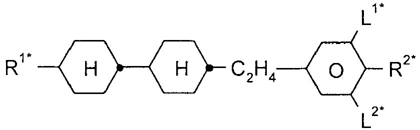 | 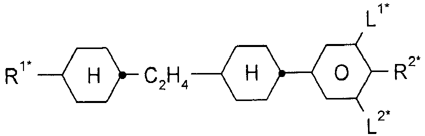 |
| **ECCP** | **CECP** |
| 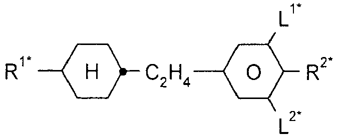 | 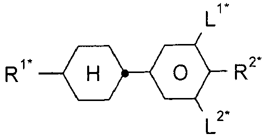 |
| **EPCH** | **PCH** |
| 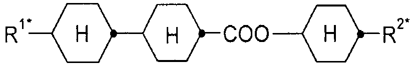 | |
| **CH** | |
| 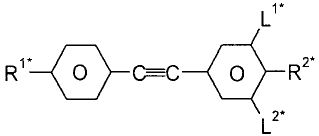 | 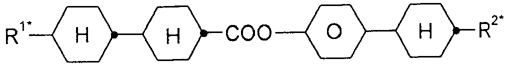 |
| **PTP** | **CCPC** |
| 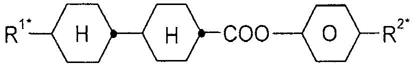 | 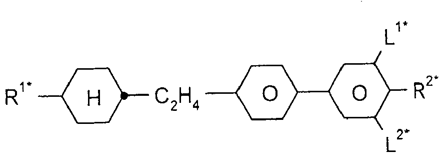 |
| **CP** | **BECH** |
| 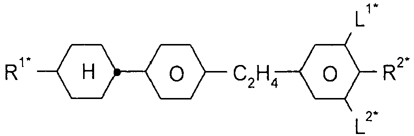 | 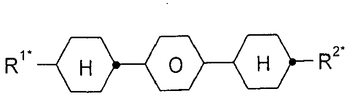 |
| **EBCH** | **CPC** |
| 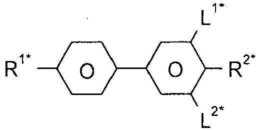 | 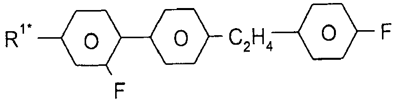 |
| **B** | **FET-nF** |
| 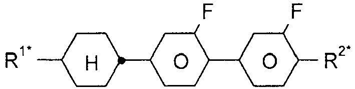 | 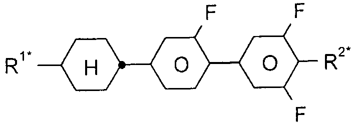 |
| **CGG** | **CGU** |
| 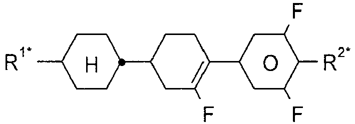 | |
| **CFU** | |

**Tabelle B**

| | |
|---|---|
| In den nachfolgenden Formeln bedeuten n und m jeweils unabhängig voneinander 0, 1, 2, 3, 4, 5, 6, 7, 8, 9, 10, 11 oder 12, insbesondere 2, 3, 5, ferner 0, 4, 6. | |
| 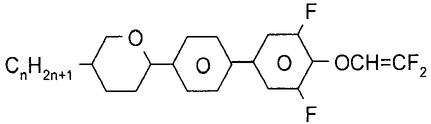 | |
| **APU-n-OXF** | |
| 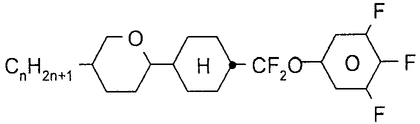 | |
| **ACQU-n-F** | |
| 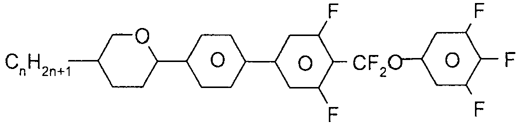 | |
| **APUQU-n-F** | |
| 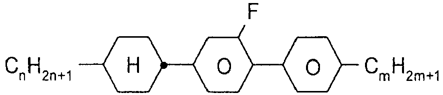 | 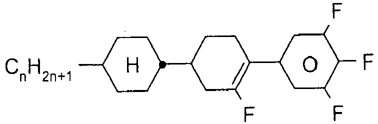 |
| **BCH-n.Fm** | **CFU-n-F** |
| 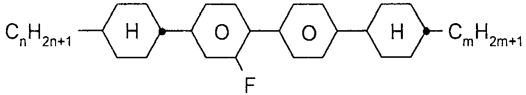 | |
| **CBC-nmF** | |
| 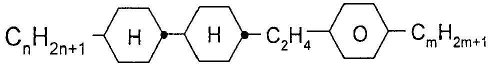 | 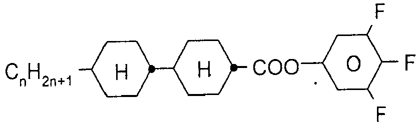 |
| **ECCP-nm** | **CCZU-n-F** |
| 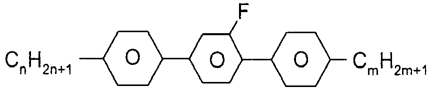 | 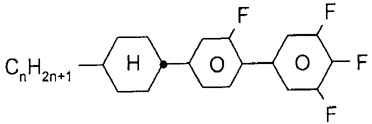 |
| **PGP-n-m** | **CGU-n-F** |
| 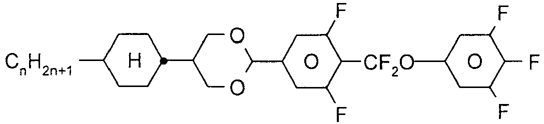 | |
| **CDUQU-n-F** | |
| 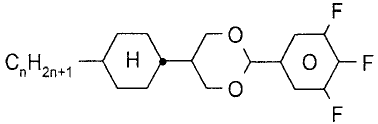 **CDU-n-F** | 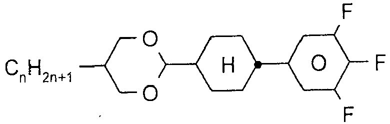 **DCU-n-F** |
| 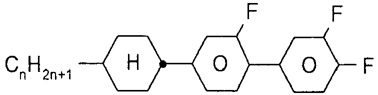 | 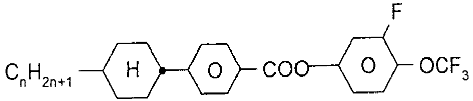 |
| **CGG-n-F** | **CPZG-n-OT** |
| 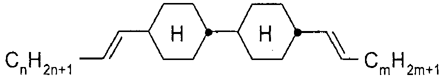 | |
| **CC-nV-Vm** | |
| 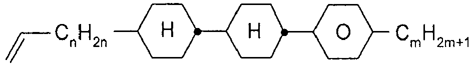 | 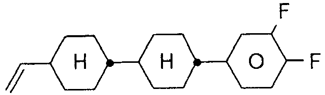 |
| **CCP-Vn-m** | **CCG-V-F** |
| 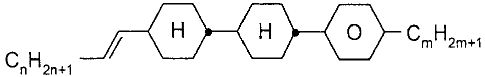 | 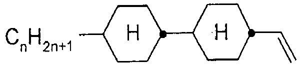 |
| **CCP-nV-m** | **CC-n-V** |
| 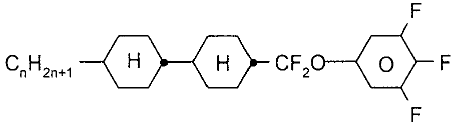 | 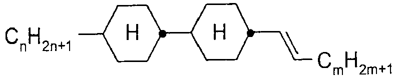 |
| **CCQU-n-F** | **CC-n-Vm** |
| 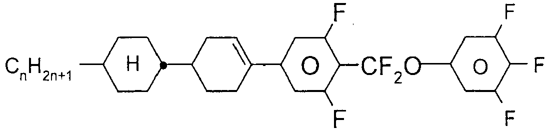 | |
| **CLUQU-n-F** | |
| 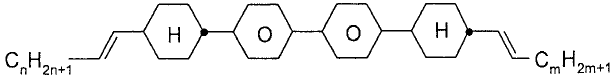 | |
| **CPPC-nV-Vm** | |
| 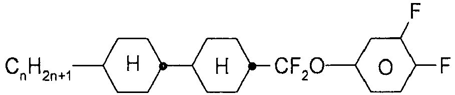 | 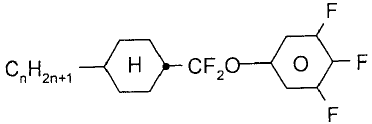 |
| **CCQG-n-F** | **CQU-n-F** |
| 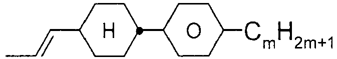 | |
| **CP-1V-m** | |
| 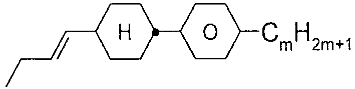 | 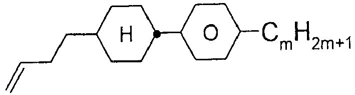 |
| **CP-2V-m** | **CP-V2-m** |
| 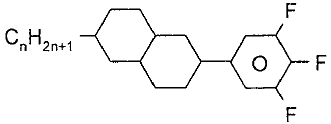 | 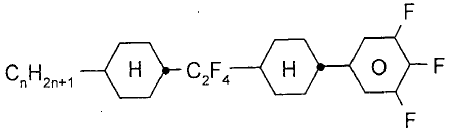 |
| **Dec-U-n-F** | **CWCU-n-F** |
| 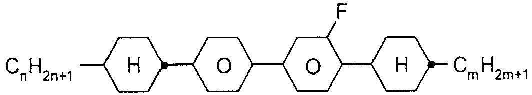 | |
| **CPGP-n-m** | |
| 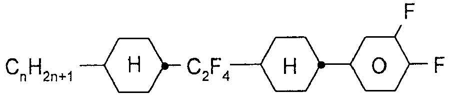 | |
| **CWCG-n-F** | |
| 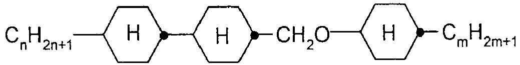 | |
| **CCOC-n-m** | |
| 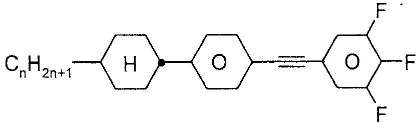 | 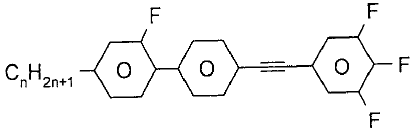 |
| **CPTU-n-F** | **GPTU-n-F** |
| 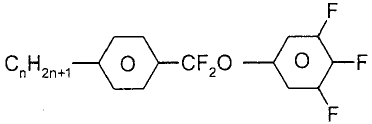 | 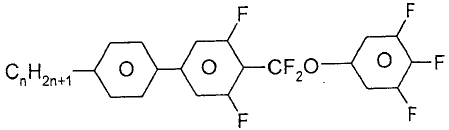 |
| **PQU-n-F** | **PUQU-n-F** |
| 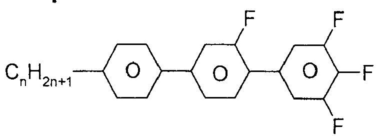 | 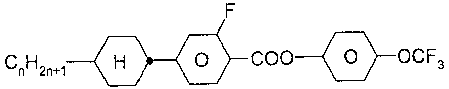 |
| **PGU-n-F** | **CGZP-n-OT** |
| 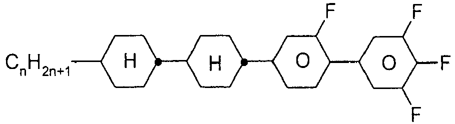 | 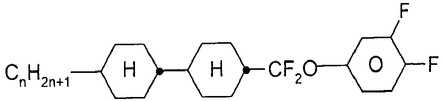 |
| **CCGU-n-F** | **CCQG-n-F** |
| 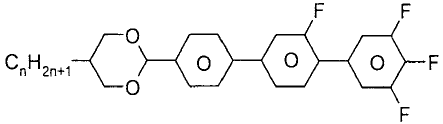 | 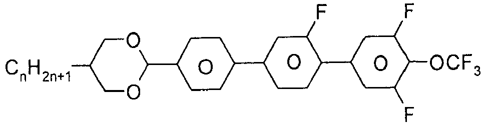 |
| **DPGU-n-F** | **DPGU-n-OT** |
| 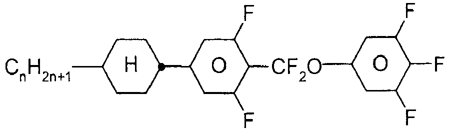 | |
| **CUQU-n-F** | |
| 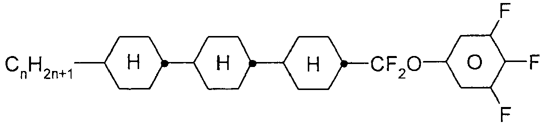 | |
| **CCCQU-n-F** | |
| 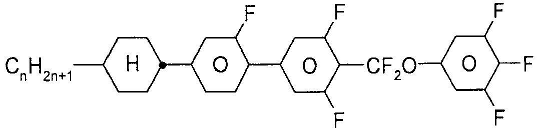 | |
| **CGUQU-n-F** | |
| 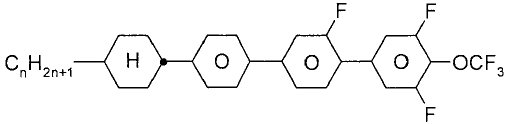 | |
| **CPGU-n-OT** | |
| 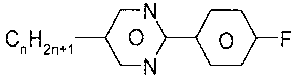 | |
| **PYP-nF** | |
| 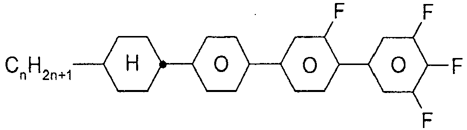 | |
| **CPGU-n-F** | |
| 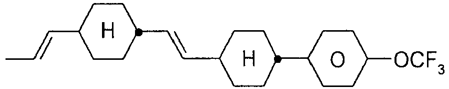 | 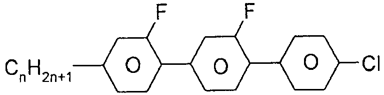 |
| **CVCP-1V-OT** | **GGP-n-Cl** |
| 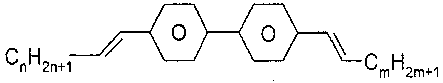 | 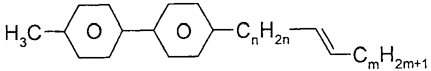 |
| **PP-nV-Vm** | **PP-1-nVm** |
| 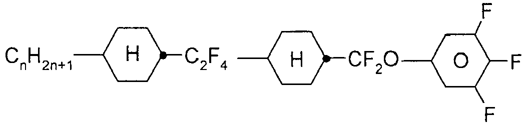 | |
| **CWCQU-n-F** | |
| 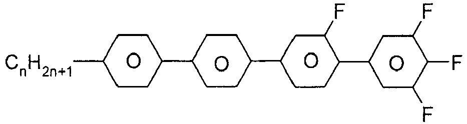 | |
| **PPGU-n-F** | |
| 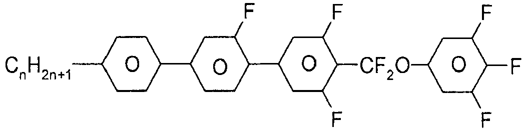 | |
| **PGUQU-n-F** | |
| 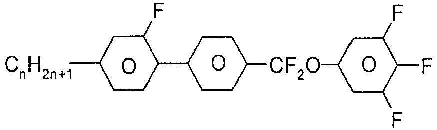 | 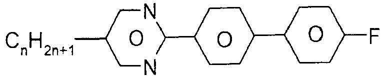 |
| **GPQU-n-F** | **MPP-n-F** |
| 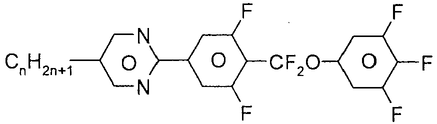 | 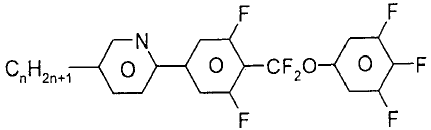 |
| **MUQU-n-F** | **NUQU-n-F** |
| 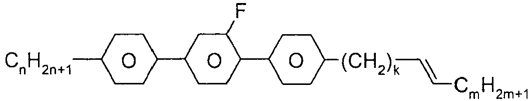 | |
| **PGP-n-kVm** | |
| 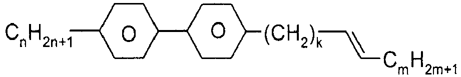 | |
| **PP-n-kVm** | |
| 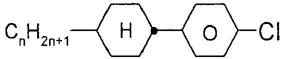 | 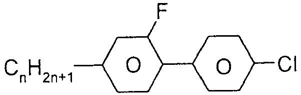 |
| **PCH-nCl** | **GP-n-Cl** |
| 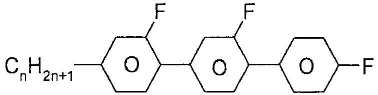 | 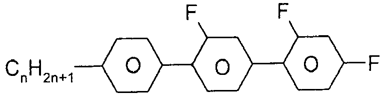 |
| **GGP-n-F** | **PGIGI-n-F** |
| 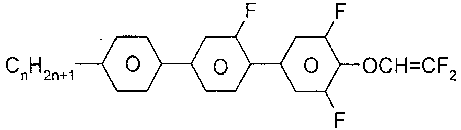 | 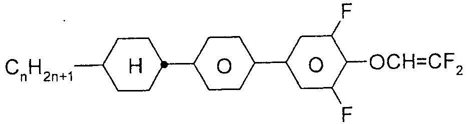 |
| **PGU-n-OXF** | **CPU-n-OXF** |

Besonders bevorzugt sind flüssigkristalline Mischungen, die neben den Verbindungen der Formeln IA und IB mindestens ein, zwei, drei, vier oder mehr Verbindungen aus der Tabelle B enthalten.

**Tabelle C**

| In der Tabelle C werden mögliche Dotierstoffe angegeben, die in der Regel den erfindungsgemäßen Mischungen zugesetzt werden. Vorzugsweise enthalten die Mischungen 0-10 Gew.%, insbesondere 0,01-5 Gew.% und besonders bevorzugt 0,01-3 Gew.% an Dotierstoffen. | |
|---|---|
| 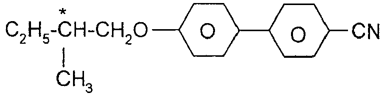 | 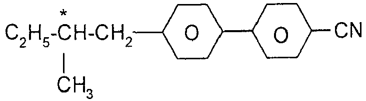 |
| **C 15** | **CB 15** |
| 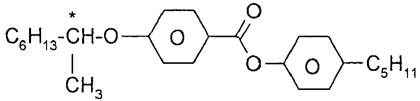 | 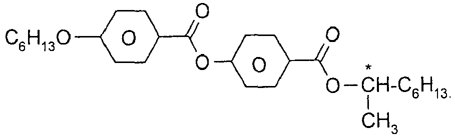 |
| **CM 21** | **R/S-811** |
| 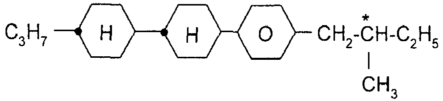 | 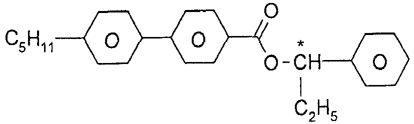 |
| **CM 44** | **CM 45** |
| 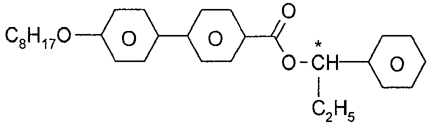 | 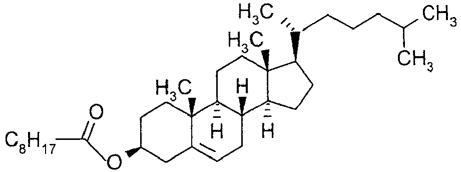 |
| **CM 47** | **CN** |
| 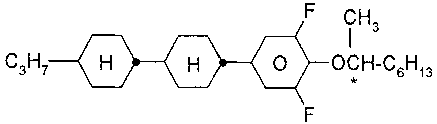 | 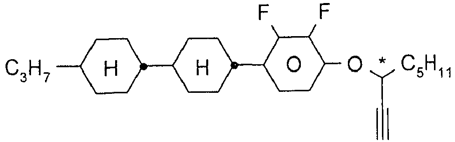 |
| **R/S-2011** | **R/S-3011** |
| 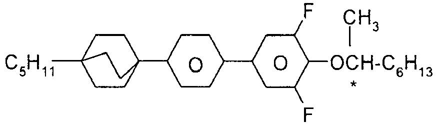 | 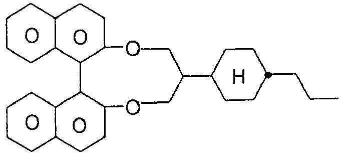 |
| **R/S-4011** | **R/S-5011** |
| 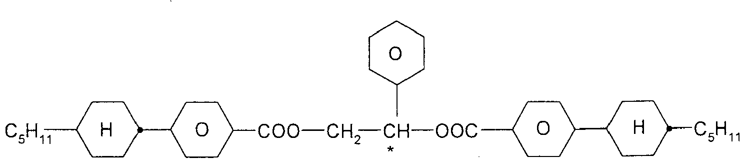 | |
| **R/S-1011** | |

**Tabelle D**

| Stabilisatoren, die beispielsweise den erfindungsgemäßen Mischungen in Mengen von 0-10 Gew.% zugesetzt werden können, werden nachfolgend genannt. | |
|---|---|
| 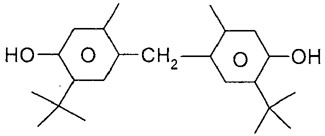 | 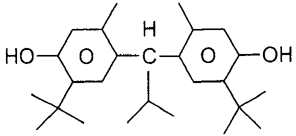 |
| 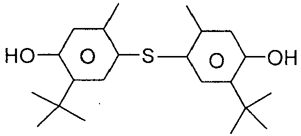 | 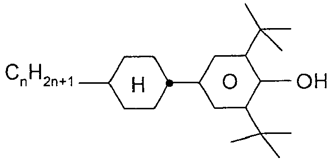 |
| | n = 1, 2, 3, 4, 5, 6 oder 7 |
| 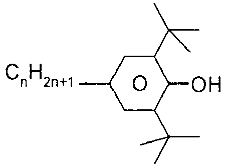 | 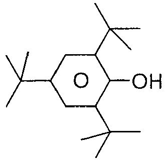 |
| n = 1, 2, 3, 4, 5, 6 oder 7 | |
| 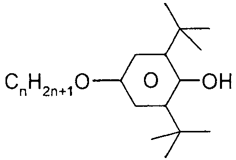 | 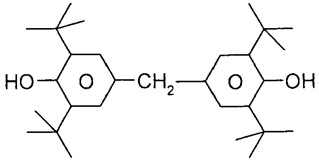 |
| 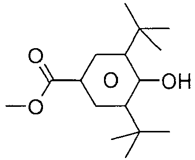 | 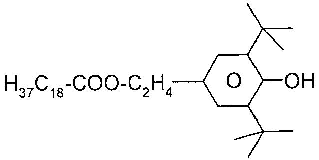 |
| 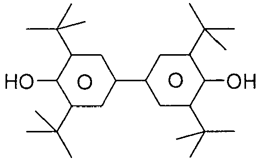 | 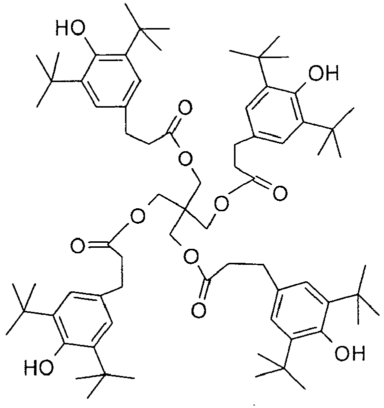 |
| 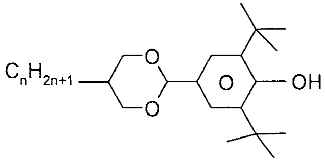 | |
| n = 1, 2, 3, 4, 5, 6 oder 7 | |
| 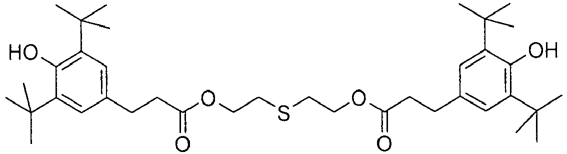 | |
| 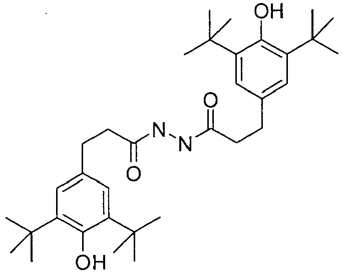 | 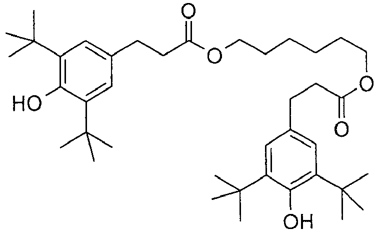 |
| 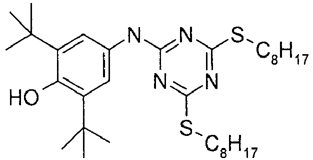 | 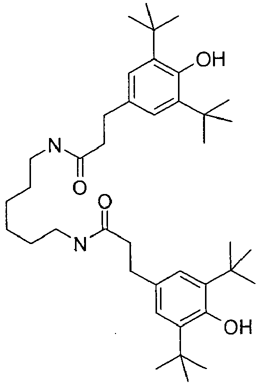 |
| 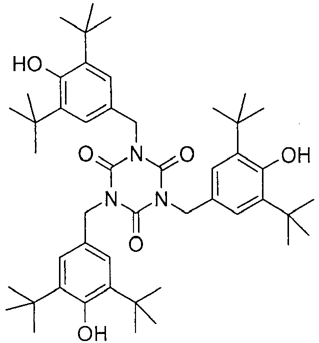 | 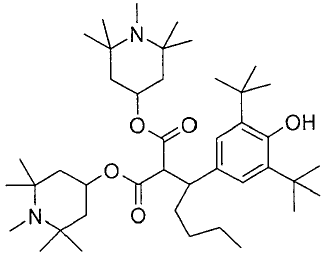 |
| 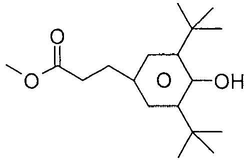 | 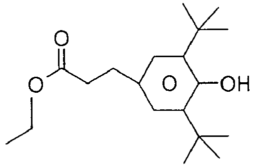 |
| 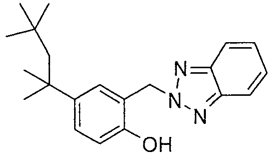 | 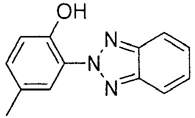 |
| 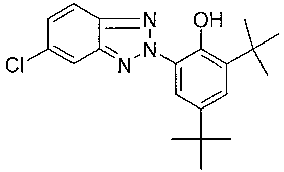 | 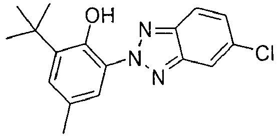 |
| 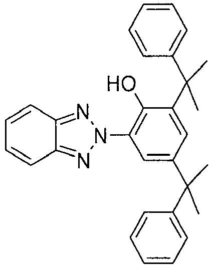 | 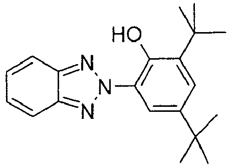 |
| 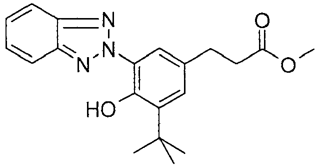 | 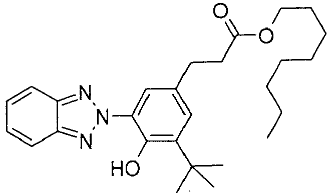 |
| 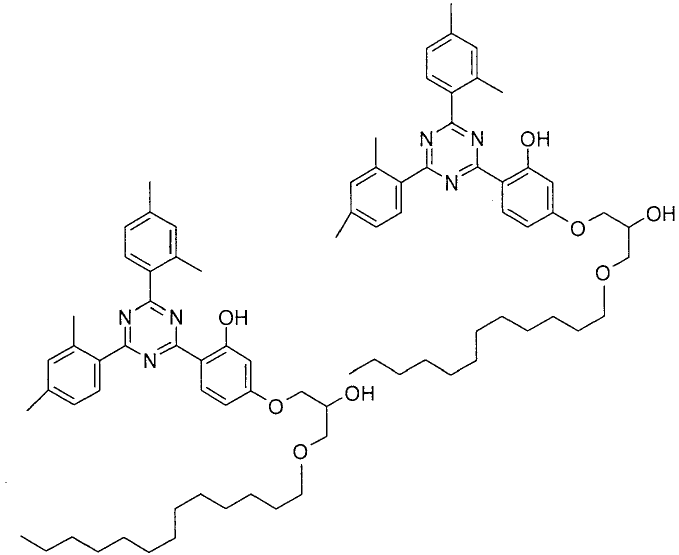 | |
| 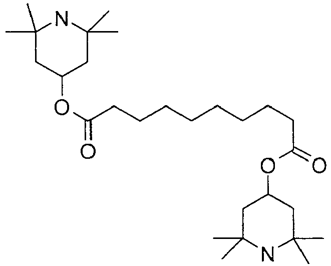 | 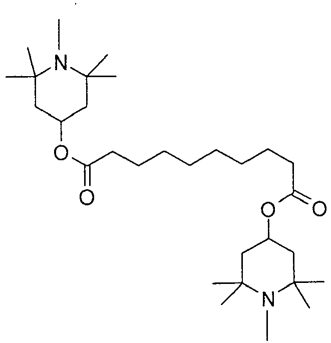 |
| 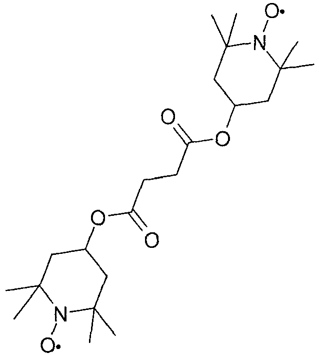 | 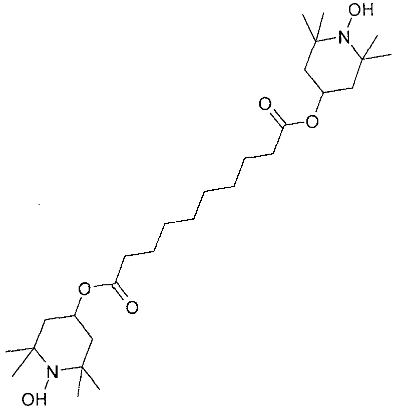 |

**Tabelle E**

| In der Tabelle E sind Beispielverbindungen zusammengestellt, die in den FK-Medien gemäß der vorliegenden Erfindung vorzugsweise als reaktive mesogene Verbindungen verwendet werden können. Sofern die erfindungsgemäßen Mischungen ein oder mehrere reaktive Verbindungen enthalten, werden sie vorzugsweise in Mengen von 0,01-5 Gew.% eingesetzt. Gegebenenfalls muss für die Polymerisation noch ein Initiator oder ein Gemisch aus zwei oder mehr Initiatoren zugesetzt werden. Der Initiator oder das Initiatorgemisch wird vorzugsweise in Mengen von 0,001-2 Gew.% bezogen auf die Mischung zugesetzt. Ein geeigneter Initiator ist z.B. Irgacure (Fa. BASF) oder Irganox (Fa. BASF). | |
|---|---|
| 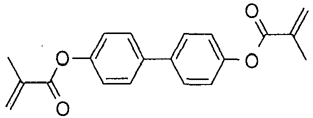 | RM-1 |
| 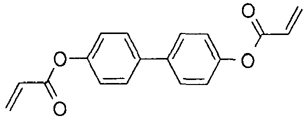 | RM-2 |
| 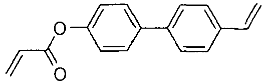 | RM-3 |
| 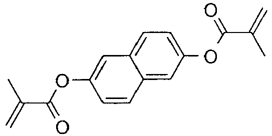 | RM-4 |
| 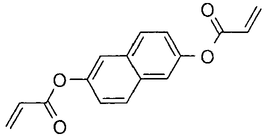 | RM-5 |
| 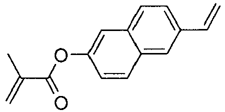 | RM-6 |
| 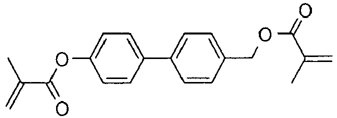 | RM-7 |
| 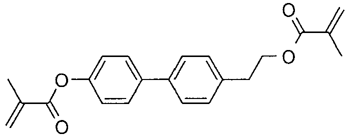 | RM-8 |
| 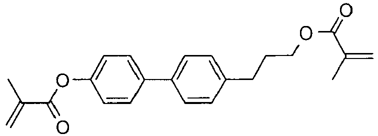 | RM-9 |
| 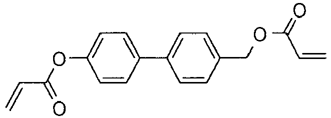 | RM-10 |
| 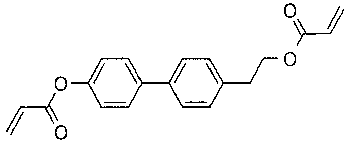 | RM-11 |
| 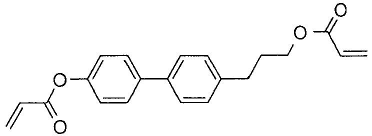 | RM-12 |
| 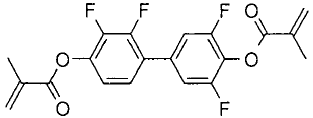 | RM-13 |
| 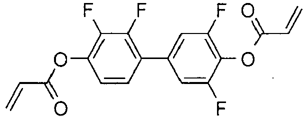 | RM-14 |
| 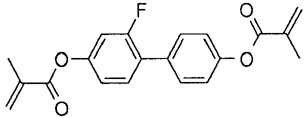 | RM-15 |
| 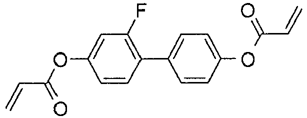 | RM-16 |
| 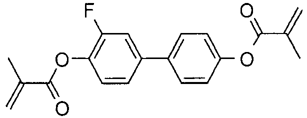 | RM-17 |
| 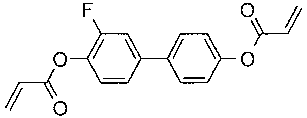 | RM-18 |
| 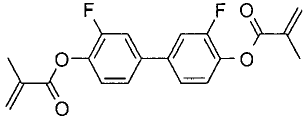 | RM-19 |
| 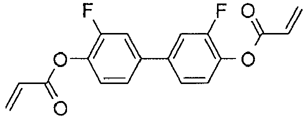 | RM-20 |
| 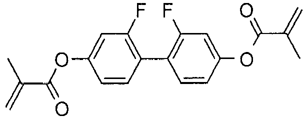 | RM-21 |
| 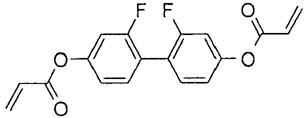 | RM-22 |
| 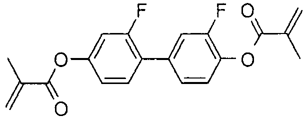 | RM-23 |
| 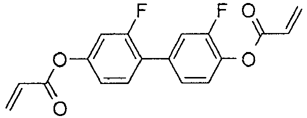 | RM-24 |
| 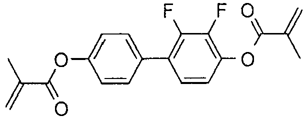 | RM-25 |
| 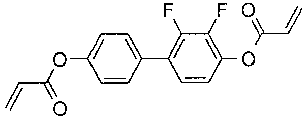 | RM-26 |
| 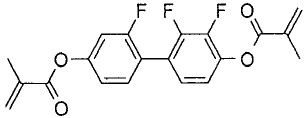 | RM-27 |
| 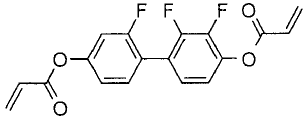 | RM-28 |
| 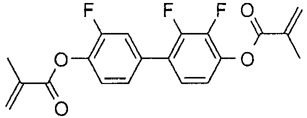 | RM-29 |
| 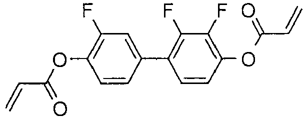 | RM-30 |
| 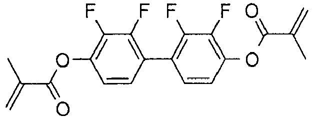 | RM-31 |
| 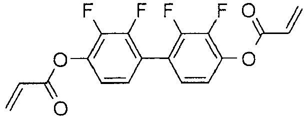 | RM-32 |
| 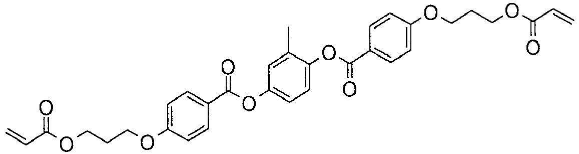 | RM-33 |
| 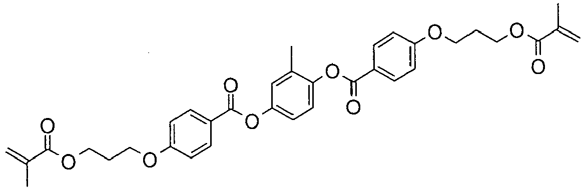 | RM-34 |
| 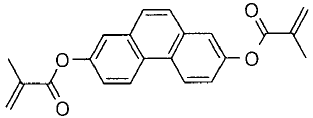 | RM-35 |
| 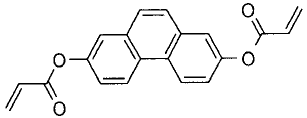 | RM-36 |
| 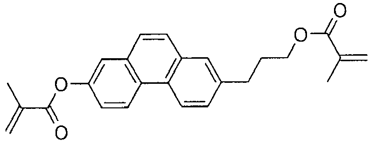 | RM-37 |
| 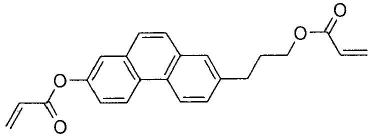 | RM-38 |
| 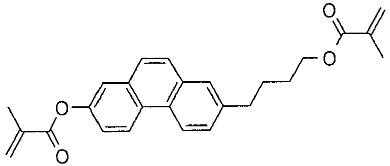 | RM-39 |
| 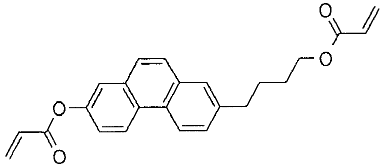 | RM-40 |
| 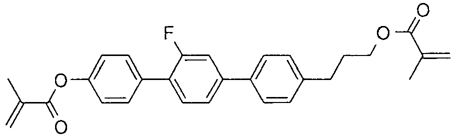 | RM-41 |
| 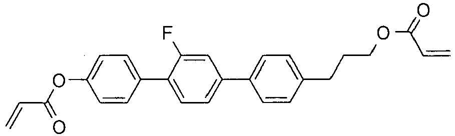 | RM-42 |
| 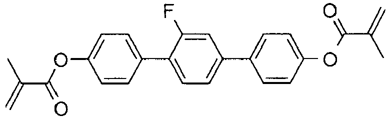 | RM-43 |
| 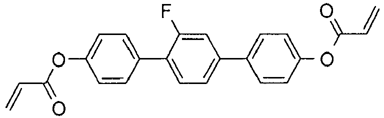 | RM-44 |
| 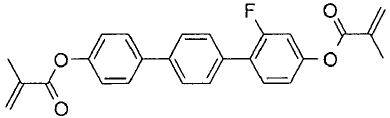 | RM-45 |
| 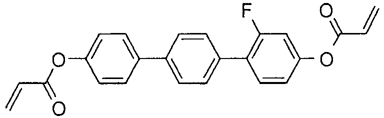 | RM-46 |
| 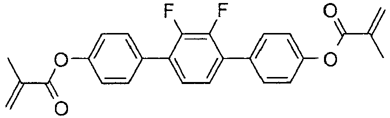 | RM-47 |
| 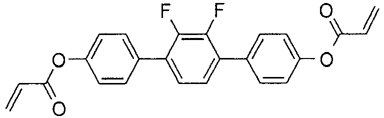 | RM-48 |
| 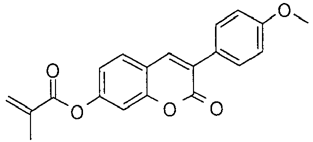 | RM-49 |
| 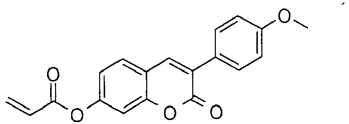 | RM-50 |
| 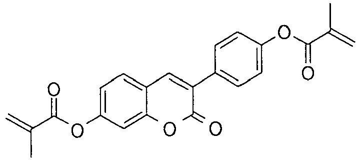 | RM-51 |
| 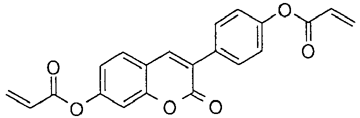 | RM-52 |
| 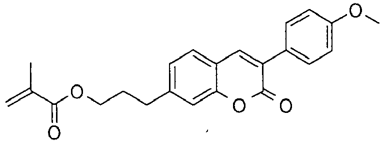 | RM-53 |
| 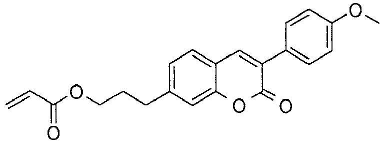 | RM-54 |
| 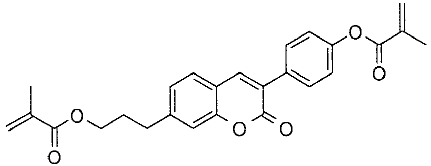 | RM-55 |
| 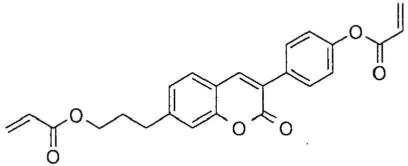 | RM-56 |
| 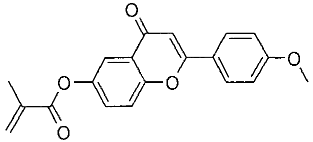 | RM-57 |
| 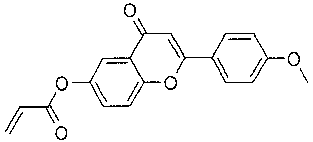 | RM-58 |
| 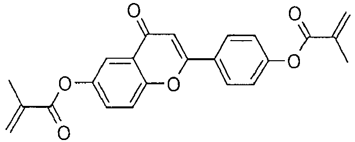 | RM-59 |
| 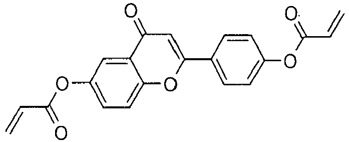 | RM-60 |
| 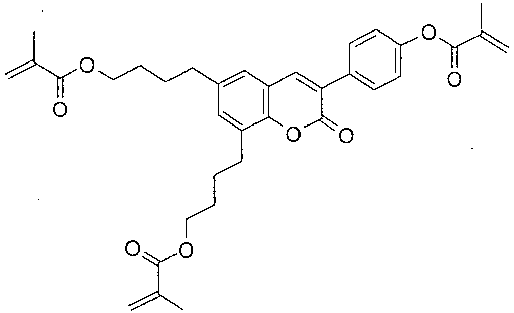 | RM-61 |
| 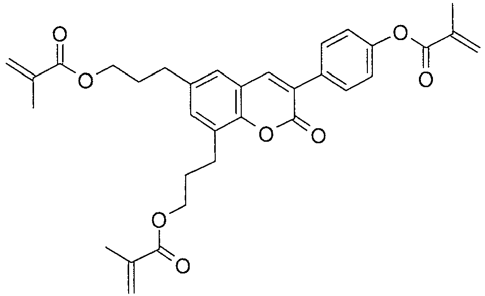 | RM-62 |
| 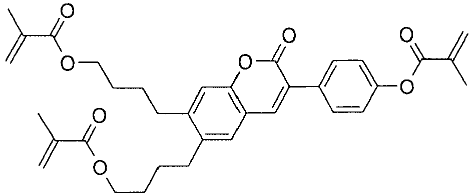 | RM-63 |
| 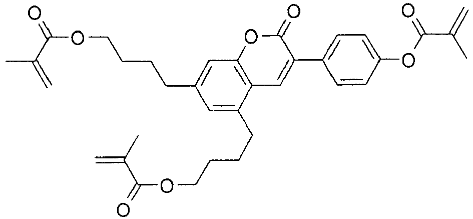 | RM-64 |
| 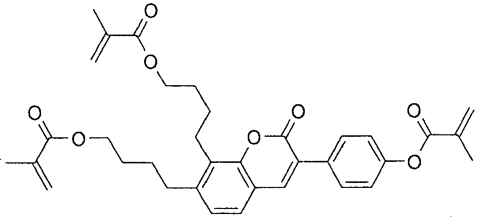 | RM-65 |
| 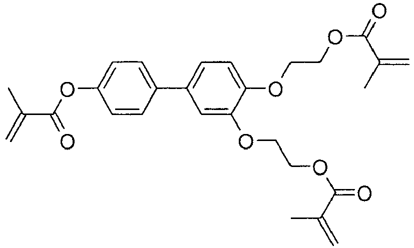 | RM-66 |
| 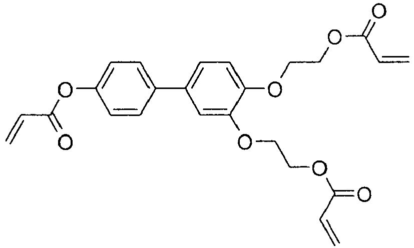 | RM-67 |
| 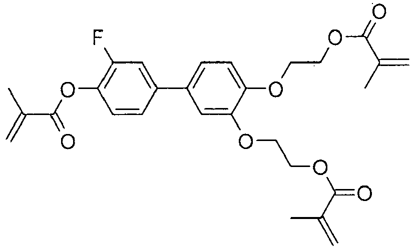 | RM-68 |
| 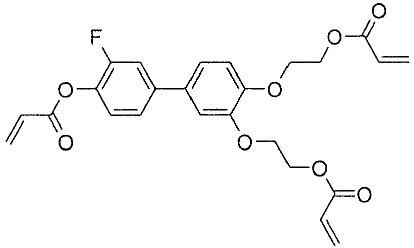 | RM-69 |
| 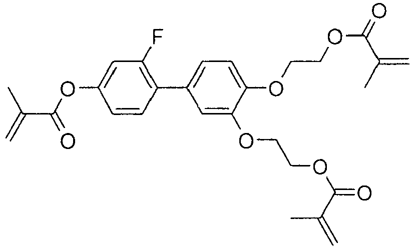 | RM-70 |
| 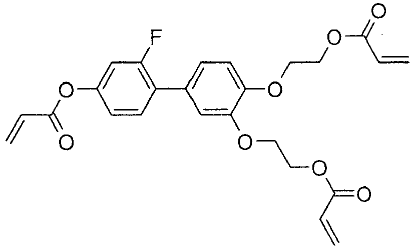 | RM-71 |
| 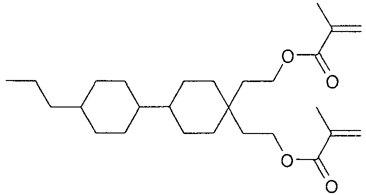 | RM-72 |
| 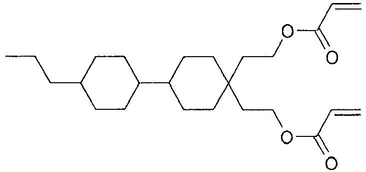 | RM-73 |
| 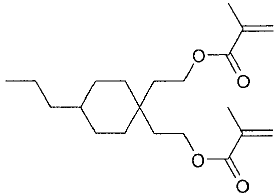 | RM-74 |
| 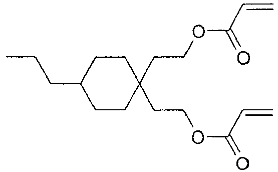 | RM-75 |
| 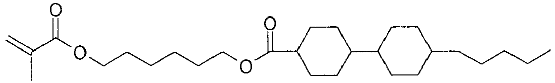 | RM-76 |
| 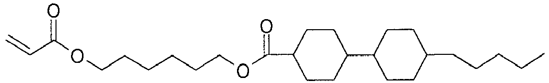 | RM-77 |
| 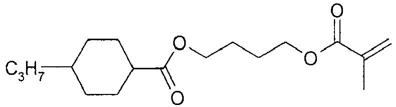 | RM-78 |
| 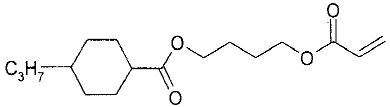 | RM-79 |
| 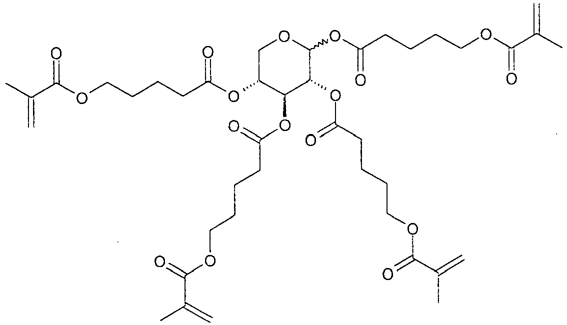 | RM-80 |
| 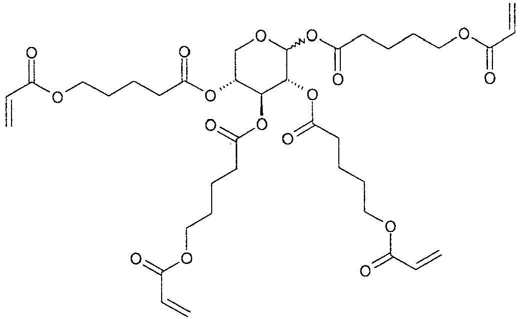 | RM-81 |
| 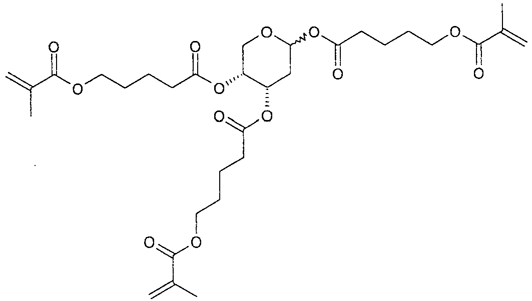 | RM-82 |
| 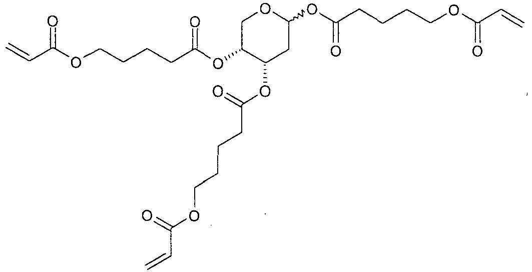 | RM-83 |
| 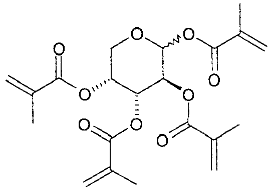 | RM-84 |
| 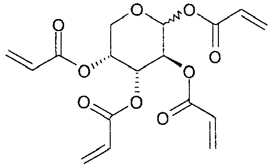 | RM-85 |

In einer bevorzugten Ausführungsform der vorliegenden Erfindung enthalten die mesogenen Medien eine oder mehrere Verbindungen ausgewählt aus der Gruppe der Verbindungen der Tabelle E.

Die folgenden Mischungsbeispiele sollen die Erfindung erläutern, ohne sie zu begrenzen.

Vor- und nachstehend bedeuten Prozentangaben Gewichtsprozent. Alle Temperaturen sind in Grad Celsius angegeben. Fp. bedeutet Schmelzpunkt, Kp. = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. Weiterhin bedeutet
- Δn die optische Anisotropie bei 589 nm und 20 °C,
- γ₁ die Rotationsviskosität (mPa·s) bei 20 °C,
- Δε die dielektrische Anisotropie bei 20 °C und 1 kHz (Δε = ε_{∥} - ε_{⊥}, wobei ε_{∥} die Dielektrizitätskonstante parallel zu den Moleküllängsachsen und ε_{⊥} die Dielektrizitätskonstante senkrecht dazu bedeutet),
- V₁₀ die Spannung (V) für 10 % Transmission (Blickrichtung senkrecht zur Plattenoberfläche), (Schwellenspannung), bestimmt in einer TN Zelle (90 Grad Verdrillung) im 1. Minimum (d.h. bei einem dΔn-Wert von 0,5 µm) bei 20 °C,
- V₀ die kapazitiv bestimmte Freedericksschwellenspannung in einer antiparallel geriebenen Zelle bei 20 °C.

Alle physikalischen Eigenschaften werden nach "Merck Liquid Crystals, Physical Properties of Liquid Crystals" Status Nov. 1997, Merck KGaA, Deutschland bestimmt und gelten für eine Temperatur von 20 °C, sofern nicht explizit anders angegeben.

### Beispiele

**Beispiel M1**

| | | | |
|---|---|---|---|
| CC-3-V | 40,0 % | Klärpunkt [°C]: | 79,5 |
| CC-3-V1 | 9,0 % | Δn [589 nm, 20 °C]: | 0,1242 |
| PUQU-3-F | 7,5 % | Δε [1 kHz, 20 °C]: | 10,2 |
| PGU-3-F | 8,0 % | γ₁ [mPa·s, 20 °C]: | 65 |
| PGP-2-2V | 10,0 % | V₁₀ [V]: | 1,41 |
| CCP-V-1 | 3,0 % | LTS bulk [-20 °C]: | > 1000 h |
| APUQU-3-F | 8,5 % | | |
| DPGU-4-F | 7,0 % | | |
| 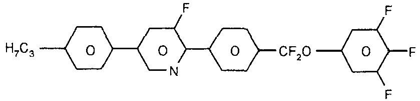 | 7,0 % | | |

**Beispiel M2**

| | | | |
|---|---|---|---|
| CC-3-V | 37,0 % | Klärpunkt [°C]: | 80,0 |
| CC-3-V1 | 9,0 % | Δn [589 nm, 20 °C]: | 0,1252 |
| PUQU-3-F | 6,0 % | Δε [1 kHz, 20 °C]: | 10,7 |
| PGU-3-F | 8,0 % | γ₁ [mPa·s, 20 °C]: | 69 |
| PGP-2-2V | 8,5 % | V₁₀ [V]: | 1,38 |
| CCP-V-1 | 7,0 % | LTS bulk [-25 °C]: | > 1000 h |
| APUQU-3-F | 11,5 % | | |
| PGUQU-3-F | 6,0 % | | |
| 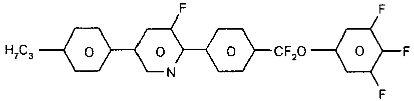 | 7,0 % | | |

**Beispiel M3**

| | | | |
|---|---|---|---|
| BCH-3F.F | 10,8 % | Klärpunkt [°C]: | 100,1 |
| BCH-5F.F | 9,0 % | Δn [589 nm, 20 °C]: | 0,1131 |
| ECCP-3OCF₃ | 4,5 % | Δε [1 kHz, 20 °C]: | 7,87 |
| ECCP-5OCF₃ | 4,5 % | | |
| CBC-33F | 1,8 % | | |
| CBC-53F | 1,8 % | | |
| CBC-55F | 1,8 % | | |
| PCH-6F | 7,2 % | | |
| PCH-7F | 5,4 % | | |
| CCP-2OCF₃ | 7,2 % | | |
| CCP-3OCF₃ | 10,8 % | | |
| CCP-4OCF₃ | 6,3 % | | |
| CCP-5OCF₃ | 9,9 % | | |
| PCH-5F | 9,0 % | | |
| 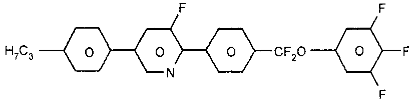 | 10,0 % | | |

## Patentansprüche

1. Flüssigkristallines Medium, **dadurch gekennzeichnet, dass** es eine oder mehrere Verbindungen der Formel IA, 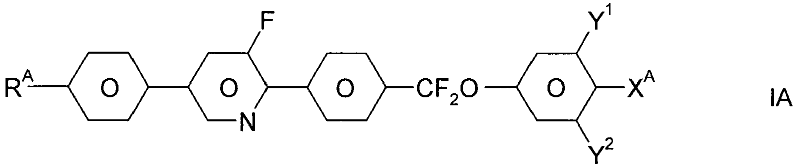 worin
R^{A} einen halogenierten oder unsubstituierten Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH-, 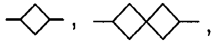 -O-, -CO-O- oder -O-CO- so ersetzt sein können, dass O-Atome nicht direkt miteinander verknüpft sind,
X^{A} F, Cl, CN, SF₅, SCN, NCS, halogenierter Alkylrest, halogenierter Alkenylrest, halogenierter Alkoxyrest oder halogenierter Alkenyloxyrest jeweils mit bis zu 6 C-Atomen, und
Y¹⁻² jeweils unabhängig voneinander H oder F
bedeuten,
enthält.

2. Flüssigkristallines Medium nach Anspruch 1, **dadurch gekennzeichnet, dass** es eine oder mehrere Verbindungen ausgewählt aus den Verbindungen der Formeln IA-g bis IA-i, 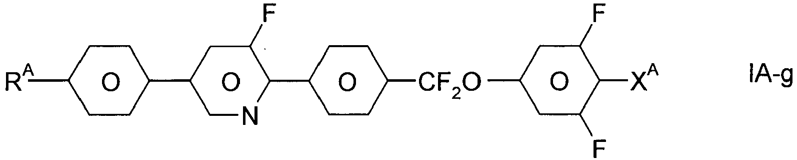 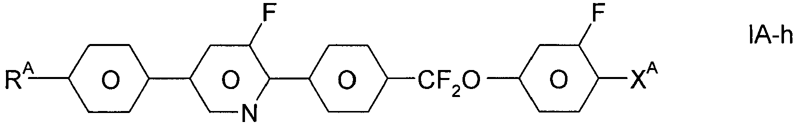 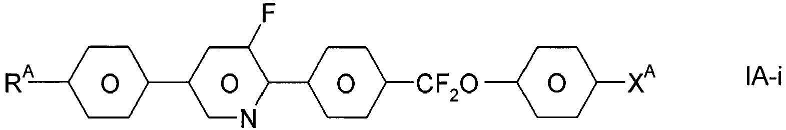 worin R^{A} und X^{A} die in Anspruch 1 angegebenen Bedeutungen haben, enthält.

3. Flüssigkristallines Medium nach Anspruch 1 oder 2, **dadurch gekennzeichnet, dass** es zusätzlich eine oder mehrere Verbindungen ausgewählt aus den Formeln II und/oder III, 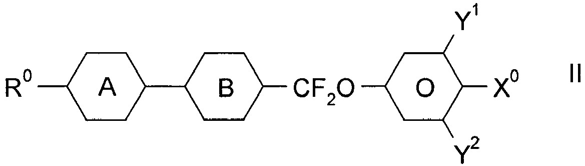 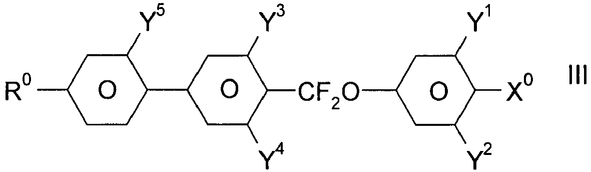 worin
R⁰ einen halogenierten oder unsubstituierten Alkyl- oder Alkoxyrest mit jeweils 1 bis 15 C-Atomen, wobei in diesen Resten auch eine oder mehrere CH₂-Gruppen jeweils unabhängig voneinander durch -C≡C-, -CF₂O-, -CH=CH-, 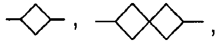 -O-, -CO-O- oder -O-CO- so ersetzt sein können, dass O-Atome nicht direkt miteinander verknüpft sind,
X⁰ F, Cl, CN, SF₅, SCN, NCS, halogenierter Alkylrest, halogenierter Alkenylrest, halogenierter Alkoxyrest oder halogenierter Alkenyloxyrest mit jeweils bis zu 6 C-Atomen, und
Y¹⁻⁵ jeweils unabhängig voneinander H oder F 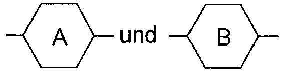 jeweils unabhängig voneinander 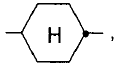 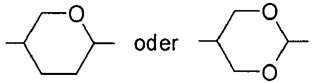
bedeuten,
enthält.

4. Flüssigkristallines Medium nach einem oder mehreren der Ansprüche 1 bis 3, **dadurch gekennzeichnet, dass** es zusätzlich eine oder mehrere Verbindungen ausgewählt aus den Formeln IV bis VIII, 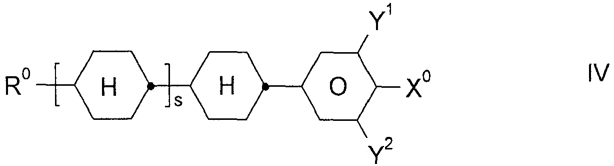 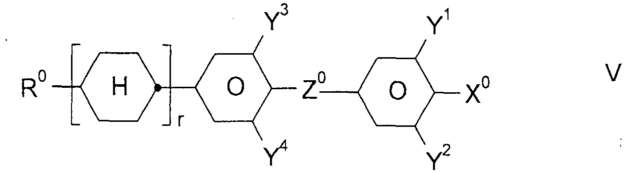 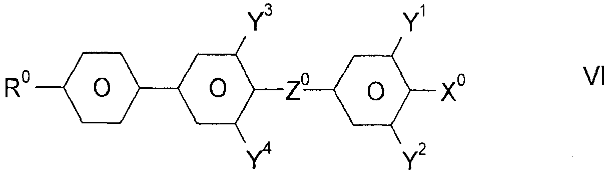 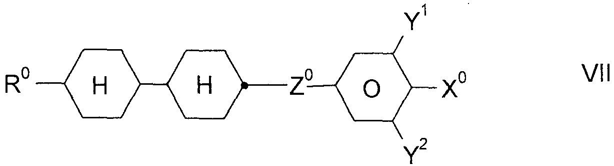 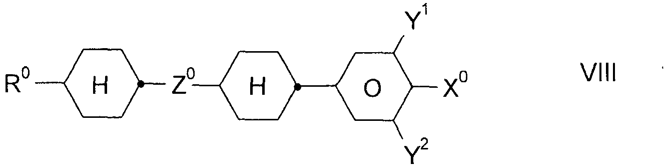 worin R⁰, X⁰ und Y¹⁻⁴ die in Anspruch 3 angegebenen Bedeutungen besitzen,
Z⁰ -C₂H₄-, -(CH₂)₄-, -CH=CH-, -CF=CF-, -C₂F₄-, -CH₂CF₂-, -CF₂CH₂-, -CH₂O-, -OCH₂-, -COO- oder -OCF₂-, in Formel V und VI auch eine Einfachbindung, in Formel V und VIII auch -CF₂O-,
r 0 oder 1, und
s 0 oder 1
bedeuten,
enthält.

5. Flüssigkristallines Medium nach einem oder mehreren der Ansprüche 1 bis 4, **dadurch gekennzeichnet, dass** es zusätzlich eine oder mehrere Verbindungen ausgewählt aus den Formeln IX bis XII, 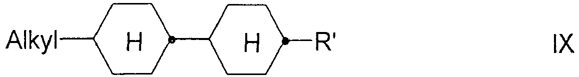 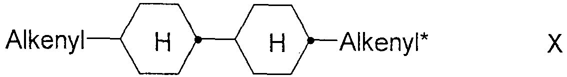 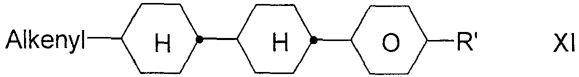 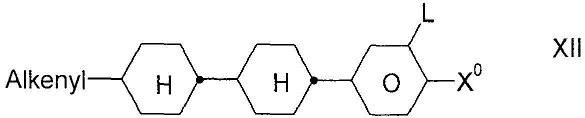 worin X⁰ die in Anspruch 3 angegebenen Bedeutungen besitzt, und
L H oder F,
"Alkyl" C₁₋₆-Alkyl,
R' C₁₋₆-Alkyl, C₁₋₆-Alkoxy oder C₂₋₆-Alkenyl, und
"Alkenyl" und "Alkenyl*"jeweils unabhängig voneinander C₂₋₆-Alkenyl bedeuten,
enthält.

6. Flüssigkristallines Medium nach einem oder mehreren der Ansprüche 1 bis 5, **dadurch gekennzeichnet, dass** es zusätzlich eine oder mehrere Verbindungen der Formel XIII, 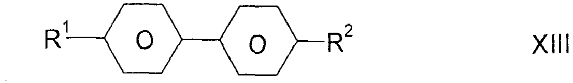 worin R¹ und R² jeweils unabhängig voneinander n-Alkyl, Alkoxy, Oxaalkyl, Fluoralkyl oder Alkenyl mit jeweils bis zu 6 C-Atomen bedeuten,
enthält.

7. Flüssigkristallines Medium nach einem oder mehreren der Ansprüche 1 bis 6, **dadurch gekennzeichnet, dass** es zusätzlich eine oder mehrere Verbindungen der Formel XVII, 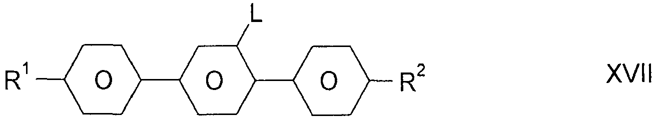 worin R¹ und R² jeweils unabhängig voneinander n-Alkyl, Alkoxy, Oxaalkyl, Fluoralkyl oder Alkenyl mit jeweils bis zu 8 C-Atomen, und L H oder F bedeuten,
enthält.

8. Flüssigkristallines Medium nach einem oder mehreren der Ansprüche 1 bis 7, **dadurch gekennzeichnet, dass** es zusätzlich eine oder mehrere Verbindungen ausgewählt aus der Gruppe der Verbindungen der Formeln XXVII, XXVIII und XXIX, 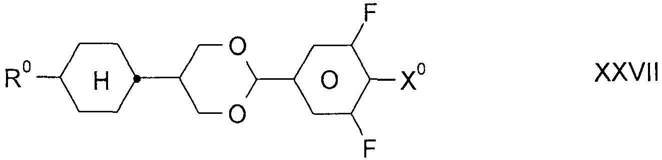 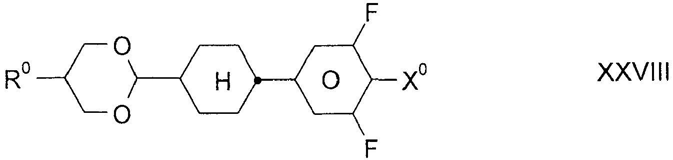 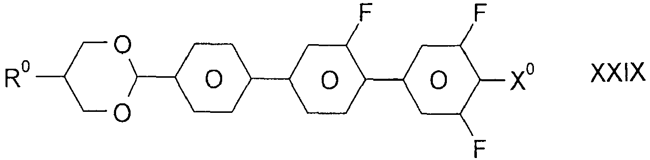 worin R⁰ und X⁰ die in Anspruch 3 angegebenen Bedeutungen haben, enthält.

9. Flüssigkristallines Medium nach einem oder mehreren der Ansprüche 1 bis 8, **dadurch gekennzeichnet, dass** es zusätzlich eine oder mehrere Verbindungen ausgewählt aus der Gruppe der Verbindungen der Formeln XIX, XX, XXI, XXII, XXIII und XXIV, 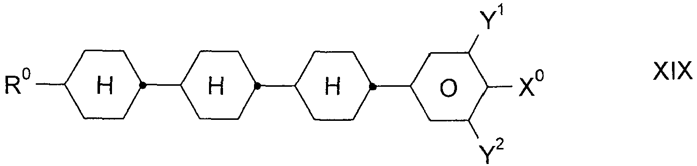 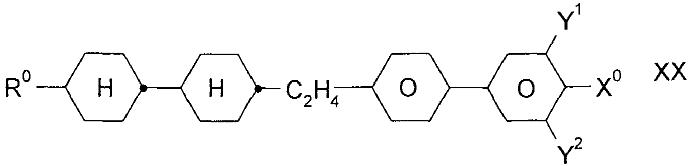 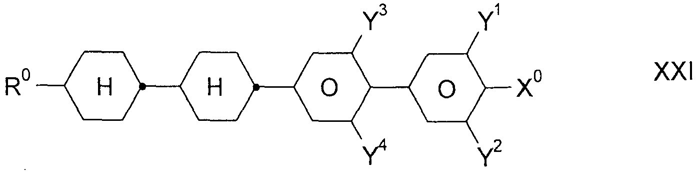 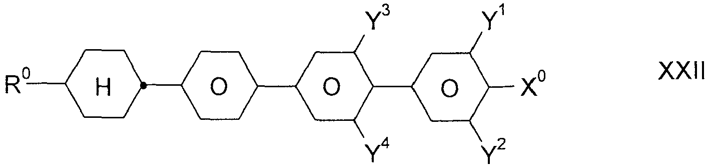 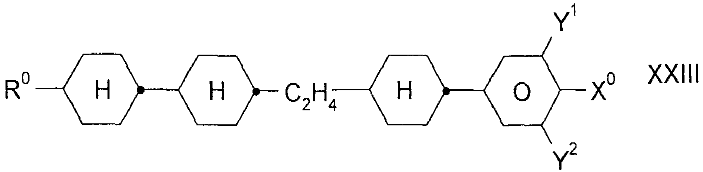 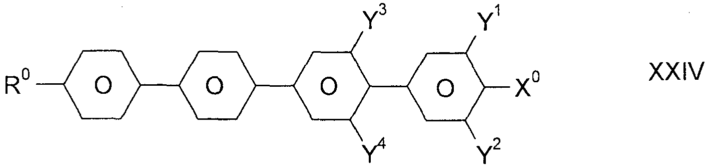 worin R⁰ und X⁰ die in Anspruch 3 angegebenen Bedeutungen haben und Y¹⁻⁴ jeweils unabhängig voneinander H oder F bedeuten,
enthält.

10. Flüssigkristallines Medium nach einem oder mehreren der Ansprüche 1 bis 9, **dadurch gekennzeichnet, dass** es ≥ 20 Gew.% der Verbindung der Formel IXb, 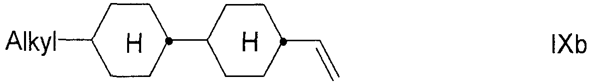 worin Alkyl die in Anspruch 5 angegebene Bedeutung hat,
enthält.

11. Flüssigkristallines Medium nach einem oder mehreren der Ansprüche 1 bis 10, **dadurch gekennzeichnet, dass** es 1-30 Gew.% bezogen auf die Mischung einer oder mehrerer Verbindungen der Formel IA enthält.

12. Flüssigkristallines Medium nach einem oder mehreren der Ansprüche 1 bis 11, **dadurch gekennzeichnet, dass** es zusätzlich ein oder mehrere Additiv(e) ausgewählt aus der Gruppe der UV-Stabilisatoren, Dotierstoffe und Antioxidantien enthält.

13. Flüssigkristallines Medium nach einem oder mehreren der Ansprüche 1 bis 12, **dadurch gekennzeichnet, dass** es zusätzlich eine oder mehrere polymerisierbare Verbindungen enthält.

14. Verfahren zur Herstellung eines flüssigkristallinen Mediums nach einem oder mehreren der Ansprüche 1 bis 13, **dadurch gekennzeichnet, dass** man eine oder mehrere Verbindungen der Formel IA, wie in Anspruch 1 definiert, mit weiteren mesogenen Verbindungen und gegebenenfalls noch mit ein oder mehreren Additiven und/oder mindestens einer polymerisierbaren Verbindung mischt.

15. Verwendung eines flüssigkristallinen Mediums nach einem oder mehreren der Ansprüche 1 bis 13 für elektrooptische Zwecke.

16. Verwendung des flüssigkristallinen Mediums nach Anspruch 15 in Shutter-Brillen, für 3D-Anwendungen, in TN-, PS-TN-, STN-, ECB-, OCB-, IPS-, PS-IPS-, FFS-, PS-FFS- und PS-VA-IPS-Anzeigen.

17. Elektrooptische Flüssigkristallanzeige enthaltend ein flüssigkristallines Medium nach einem oder mehreren der Ansprüche 1 bis 13.

18. Verbindungen der Formel IA, 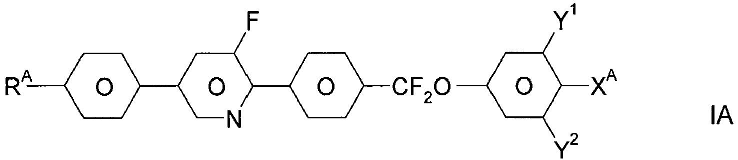 worin
R^{A} einen halogenierten oder unsubstituierten Alkyl- oder Alkoxyrest mit 1 bis 15 C-Atomen, wobei in diesen Resten auch eine oder mehrere CH₂-Gruppen jeweils unabhängig voneinander durch -C≡C-, -CF₂O-, -CH=CH- 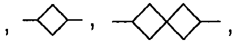 -O-, -CO-O- oder -O-CO- so ersetzt sein können, dass O-Atome nicht direkt miteinander verknüpft sind,
X^{A} F, Cl, CN, SF₅, SCN, NCS, halogenierter Alkylrest, halogenierter Alkenylrest, halogenierter Alkoxyrest oder halogenierter Alkenyloxyrest jeweils mit bis zu 6 C-Atomen, und
Y¹⁻² jeweils unabhängig voneinander H oder F
bedeuten.

19. Verbindungen der Formel IA-g bis IA-i 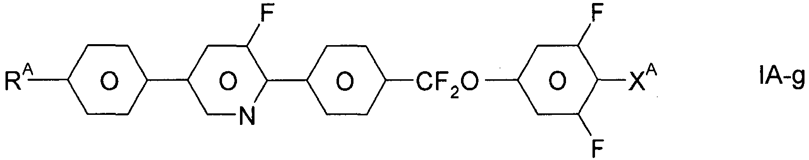 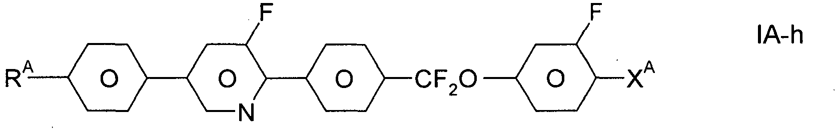 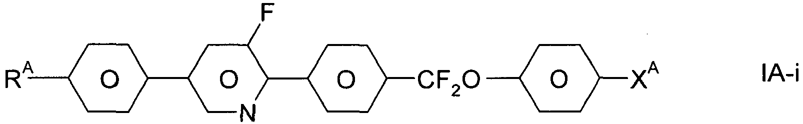 worin
R^{A} und X^{A} die in Anspruch 18 angegebenen Bedeutungen haben.

20. Verbindungen der Formel IA nach Anspruch 18 oder 19, **dadurch gekennzeichnet, dass**
R^{A} ein geradkettiger Alkylrest mit 1 bis 6 C-Atomen oder ein Alkenylrest mit 2 bis 6 C-Atomen, und
X^{A} F oder OCF₃
bedeuten.

## Claims

1. Liquid-crystalline medium, **characterised in that** it comprises one or more compounds of the formula IA, 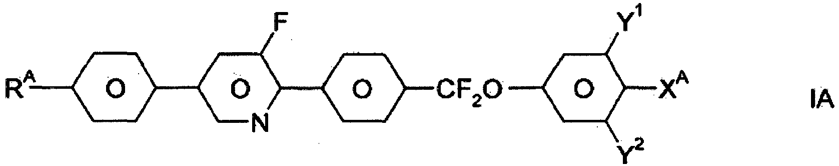 in which
R^{A} denotes a halogenated or unsubstituted alkyl or alkoxy radical having 1 to 15 C atoms, where, in addition, one or more CH₂ groups in these radicals may each be replaced, independently of one another, by -C≡C-, -CF₂O-, -CH=CH-, 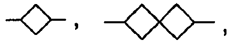 -O-, -CO-O- or -O-CO- in such a way that O atoms are not linked directly to one another,
X^{A} denotes F, Cl, CN, SF₅, SCN, NCS, a halogenated alkyl radical, a halogenated alkenyl radical, a halogenated alkoxy radical or a halogenated alkenyloxy radical, each having up to 6 C atoms, and
Y¹⁻² each, independently of one another, denote H or F.

2. Liquid-crystalline medium according to Claim 1, **characterised in that** it comprises one or more compounds selected from the compounds of the formulae IA-g to IA-i, 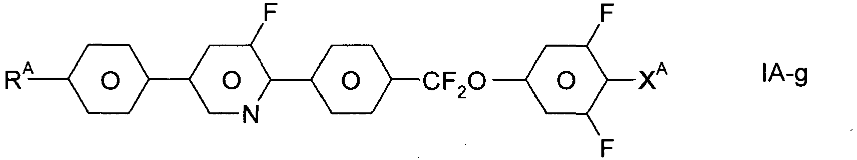 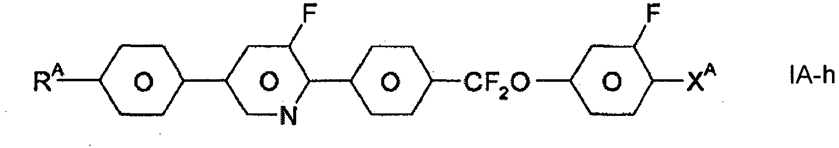 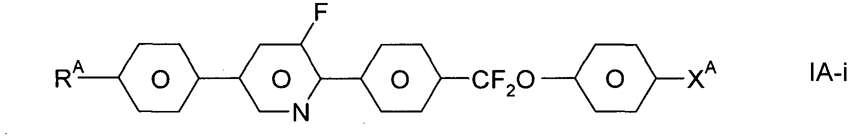 in which R^{A} and X^{A} have the meanings indicated in Claim 1.

3. Liquid-crystalline medium according to Claim 1 or 2, **characterised in that** it additionally comprises one or more compounds selected from the formulae II and/or III, 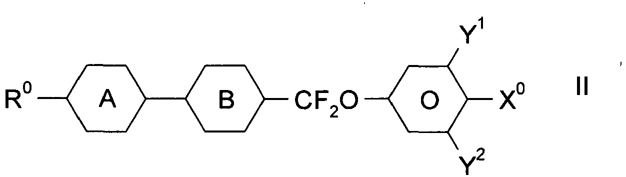 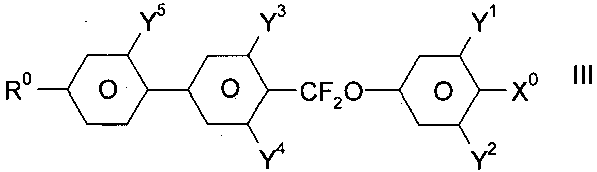 in which
R⁰ denotes a halogenated or unsubstituted alkyl or alkoxy radical, each having 1 to 15 C atoms, where, in addition, one or more CH₂ groups in these radicals may each be replaced, independently of one another, by -C≡C-, -CF₂O-, -CH=CH- 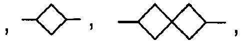 -O-, -CO-O- or -O-CO- in such a way that O atoms are not linked directly to one another,
X⁰ denotes F, Cl, CN, SF₅, SCN, NCS, a halogenated alkyl radical, a halogenated alkenyl radical, a halogenated alkoxy radical or a halogenated alkenyloxy radical, each having up to 6 C atoms, and
Y¹⁻⁵ each, independently of one another, denote H or F, 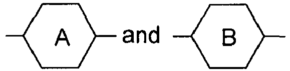 each, independently of one another, denote

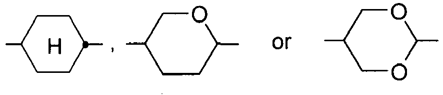

4. Liquid-crystalline medium according to one or more of Claims 1 to 3, **characterised in that** it additionally comprises one or more compounds selected from the formulae IV to VIII, 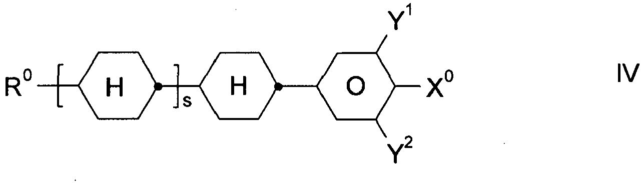 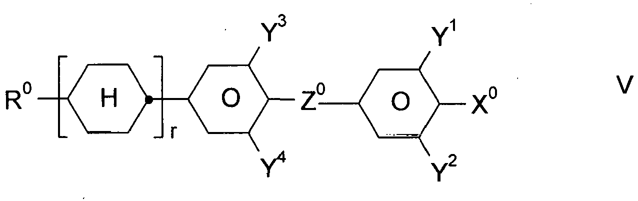 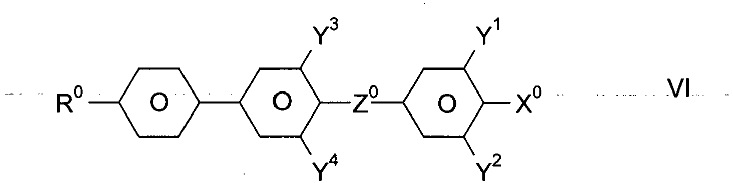 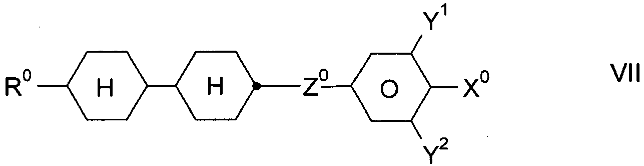 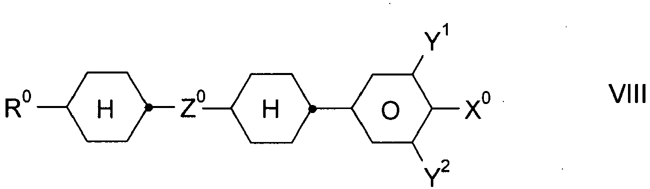 in which R⁰, X⁰ and Y¹⁻⁴ have the meanings indicated in Claim 3,
Z⁰ denotes -C₂H₄-, -(CH₂)₄-, -CH=CH-, -CF=CF-, -C₂F₄-, -CH₂CF₂-, -CF₂CH₂-, -CH₂O-, -OCH₂-, -COO- or -OCF₂-, in formulae V and VI also a single bond, in formulae V and VIII also -CF₂O-,
r denotes 0 or 1, and
s denotes 0 or 1.

5. Liquid-crystalline medium according to one or more of Claims 1 to 4, **characterised in that** it additionally comprises one or more compounds selected from the formulae IX to XII, 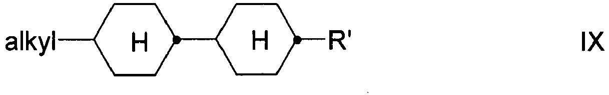 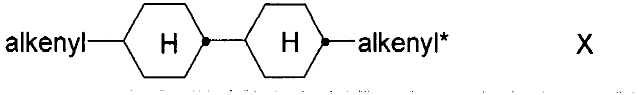 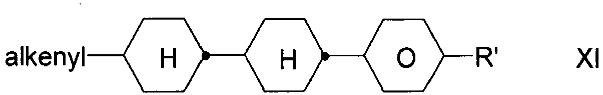 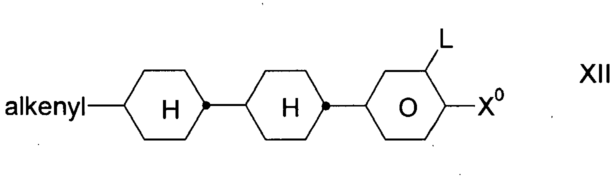 in which X⁰ has the meanings indicated in Claim 3, and
L denotes H or F,
"alkyl" denotes C₁₋₆-alkyl,
R' denotes C₁₋₆-alkyl, C₁₋₆-alkoxy or C₂₋₆-alkenyl, and
"alkenyl" and "alkenyl*" each, independently of one another, denote C₂₋₆-alkenyl.

6. Liquid-crystalline medium according to one or more of Claims 1 to 5, **characterised in that** it additionally comprises one or more compounds of the formula XIII, 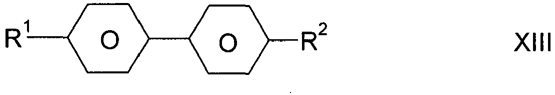 in which R¹ and R² each, independently of one another, denote n-alkyl, alkoxy, oxaalkyl, fluoroalkyl or alkenyl, each having up to 6 C atoms.

7. Liquid-crystalline medium according to one or more of Claims 1 to 6, **characterised in that** it additionally comprises one or more compounds of the formula XVII, 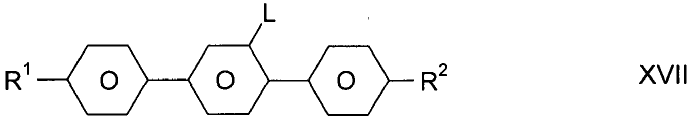 in which R¹ and R² each, independently of one another, denote n-alkyl, alkoxy, oxaalkyl, fluoroalkyl or alkenyl, each having up to 8 C atoms, and L denotes H or F.

8. Liquid-crystalline medium according to one or more of Claims 1 to 7, **characterised in that** it additionally comprises one or more compounds selected from the group of the compounds of the formulae XXVII, XXVIII and XXIX, 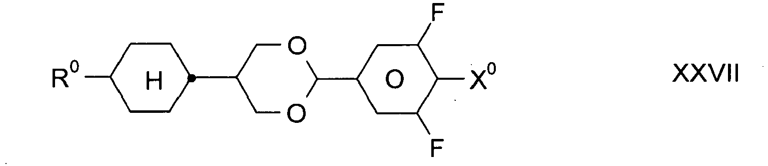 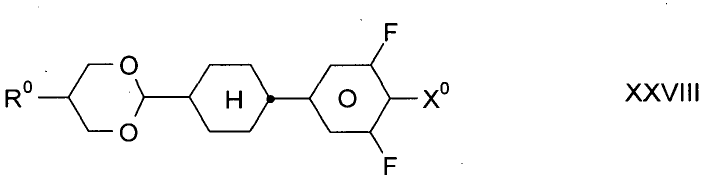 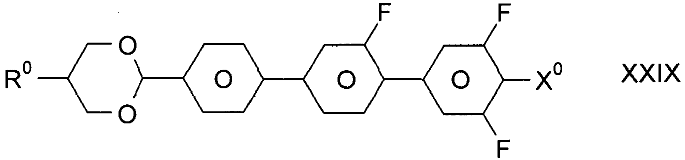 in which R⁰ and X⁰ have the meanings indicated in Claim 3.

9. Liquid-crystalline medium according to one or more of Claims 1 to 8, **characterised in that** it additionally comprises one or more compounds selected from the group of the compounds of the formulae XIX, XX, XXI, XXII, XXIII and XXIV, 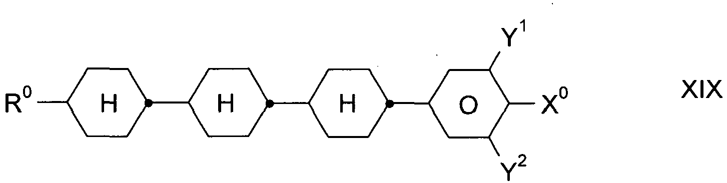 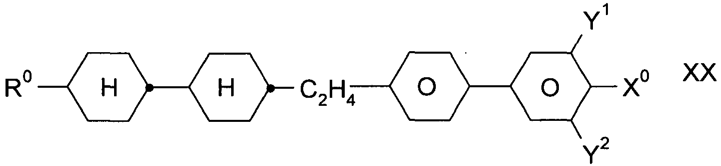 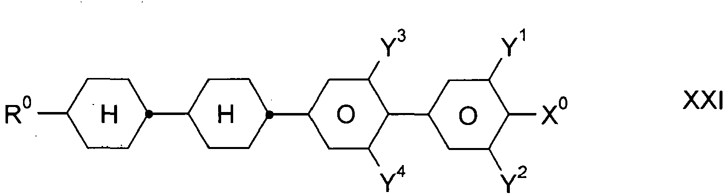 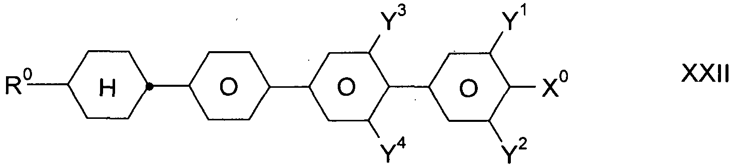 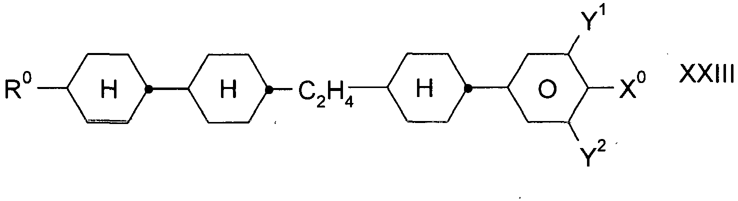 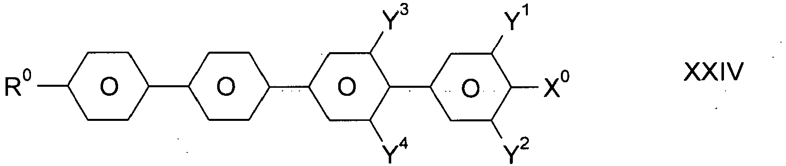 in which R⁰ and X⁰ have the meanings indicated in Claim 3, and Y¹⁻⁴ each, independently of one another, denote H or F.

10. Liquid-crystalline medium according to one or more of Claims 1 to 9, **characterised in that** it comprises ≥ 20% by weight of the compound of the formula IXb, 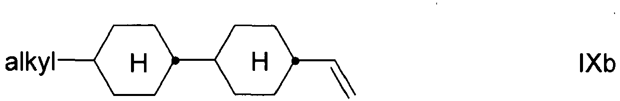 in which alkyl has the meaning indicated in Claim 5.

11. Liquid-crystalline medium according to one or more of Claims 1 to 10, **characterised in that** it comprises 1-30% by weight, based on the mixture, of one or more compounds of the formula IA.

12. Liquid-crystalline medium according to one or more of Claims 1 to 11, **characterised in that** it additionally comprises one or more additive(s) selected from the group of the UV stabilisers, dopants and antioxidants.

13. Liquid-crystalline medium according to one or more of Claims 1 to 12, **characterised in that** it additionally comprises one or more polymerisable compounds.

14. Process for the preparation of a liquid-crystalline medium according to one or more of Claims 1 to 13, **characterised in that** one or more compounds of the formula IA, as defined in Claim 1, are mixed with further mesogenic compounds and optionally also with one or more additives and/or at least one polymerisable compound.

15. Use of a liquid-crystalline medium according to one or more of Claims 1 to 13 for electro-optical purposes.

16. Use of the liquid-crystalline medium according to Claim 15 in shutter spectacles, for 3D applications, in TN, PS-TN, STN, ECB, OCB, IPS, PS-IPS, FFS, PS-FFS and PS-VA-IPS displays.

17. Electro-optical liquid-crystal display containing a liquid-crystalline medium according to one or more of Claims 1 to 13.

18. Compounds of the formula IA, 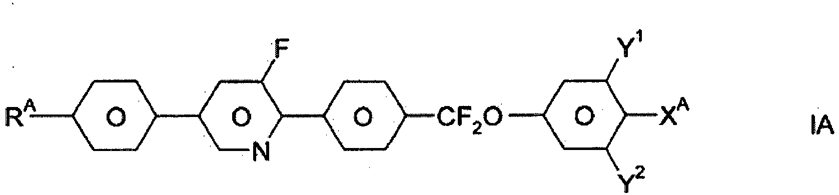 in which
R^{A} denotes a halogenated or unsubstituted alkyl or alkoxy radical having 1 to 15 C atoms, where, in addition, one or more CH₂ groups in these radicals may each be replaced, independently of one another, by -C≡C-, -CF₂O-, -CH=CH-, 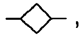 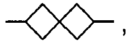 -O-, -CO-O- or -O-CO- in such a way that O atoms are not linked directly to one another,
X^{A} denotes F, Cl, CN, SF₅, SCN, NCS, a halogenated alkyl radical, a halogenated alkenyl radical, a halogenated alkoxy radical or a halogenated alkenyloxy radical, each having up to 6 C atoms, and
Y¹⁻² each, independently of one another, denote H or F.

19. Compounds of the formulae IA-g to IA-i 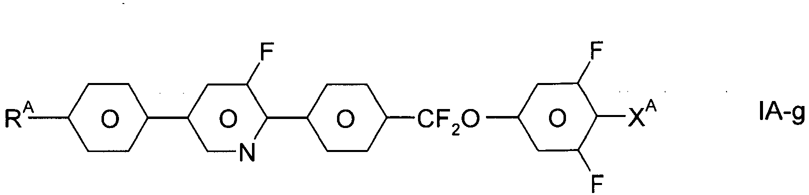 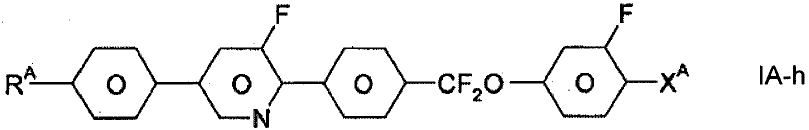 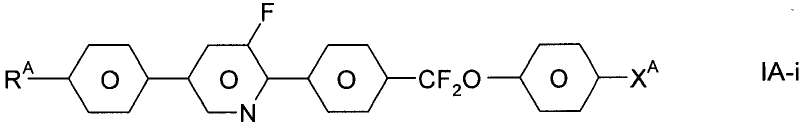 in which
R^{A} and X^{A} have the meanings indicated in Claim 18.

20. Compounds of the formula IA according to Claim 18 or 19, **characterised in that**
R^{A} denotes a straight-chain alkyl radical having 1 to 6 C atoms or an alkenyl radical having 2 to 6 C atoms, and
X^{A} denotes F or OCF₃.

## Revendications

1. Milieu cristallin liquide, **caractérisé en ce qu'**il comprend un ou plusieurs composé(s) de la formule IA : 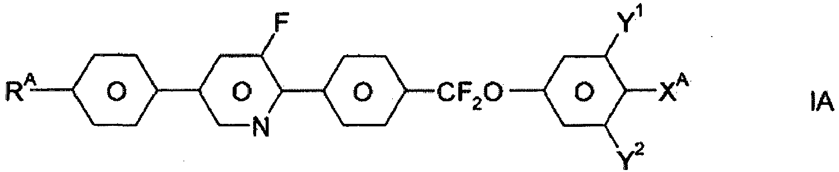 dans laquelle :
R^{A} représente un radical alkyle ou alcoxy halogéné ou non substitué qui comporte 1 à 15 atome(s) de C, où, en outre, un ou plusieurs groupe(s) CH₂ dans ces radicaux peut/peuvent chacun être remplacé(s), indépendamment les uns des autres, par -C≡C-, -CF₂O-, -CH=CH-, 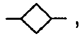 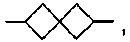 -O-, -CO-O- ou -O-CO- de telle sorte que des atomes de O ne soient pas liés directement les uns aux autres,
X^{A} représente F, CI, CN, SF₅, SCN, NCS, un radical alkyle halogéné, un radical alkényle halogéné, un radical alcoxy halogéné ou un radical alkényloxy halogéné dont chacun comporte jusqu'à 6 atomes de C, et
Y¹⁻² représentent chacun, indépendamment l'un de l'autre, H ou F.

2. Milieu cristallin liquide selon la revendication 1, **caractérisé en ce qu'**il comprend un ou plusieurs composé(s) qui est/sont sélectionnés parmi les composés des formules IA-g à IA-i : 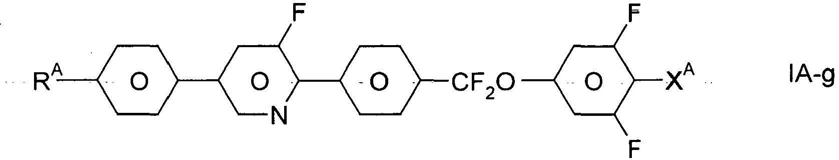 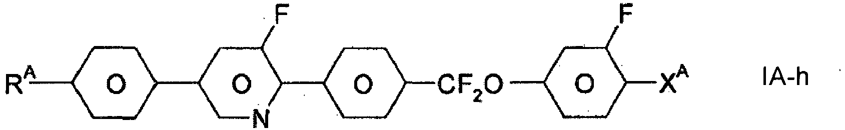 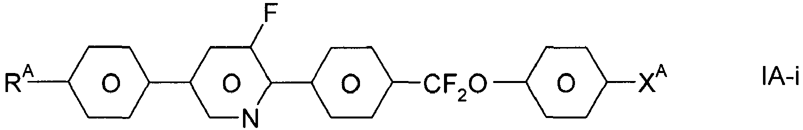 dans lesquelles R^{A} et X^{A} présentent les significations qui sont indiquées selon la revendication 1.

3. Milieu cristallin liquide selon la revendication 1 ou 2, **caractérisé en ce qu'**il comprend, de manière additionnelle, un ou plusieurs composé(s) qui est/sont sélectionné(s) parmi les formules II et/ou III : 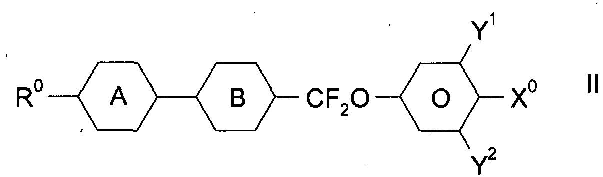 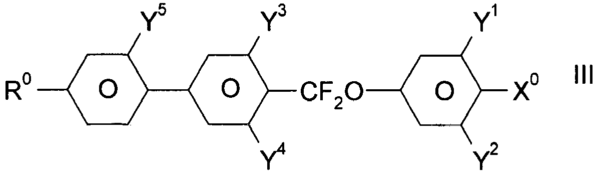 dans lesquelles :
R⁰ représente un radical alkyle ou alcoxy halogéné ou non substitué dont chacun comporte 1 à 15 atome(s) de C, où, en outre, un ou plusieurs groupe(s) CH₂ dans ces radicaux peut/peuvent chacun être remplacé(s), indépendamment les uns des autres, par -C≡C-, -CF₂O-, -CH=CH-, 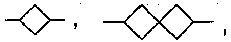 -O-, -CO-O-ou -O-CO- de telle sorte que des atomes de O ne soient pas liés directement les uns aux autres,
X⁰ représente F, CI, CN, SF₅, SCN, NCS, un radical alkyle halogéné, un radical alkényle halogéné, un radical alcoxy halogéné ou un radical alkényloxy halogéné dont chacun comporte jusqu'à 6 atomes de C, et
Y¹⁻⁵ représentent chacun, indépendamment les uns des autres, H ou F, 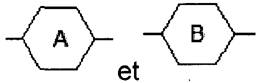 représentent chacun, indépendamment l'un de l'autre,

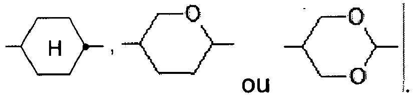

4. Milieu cristallin liquide selon une ou plusieurs des revendications 1 à 3, **caractérisé en ce qu'**il comprend de manière additionnelle un ou plusieurs composé(s) qui est/sont sélectionné(s) parmi les formules IV à VIII : 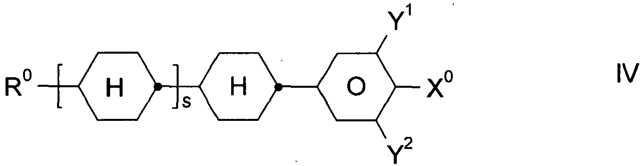 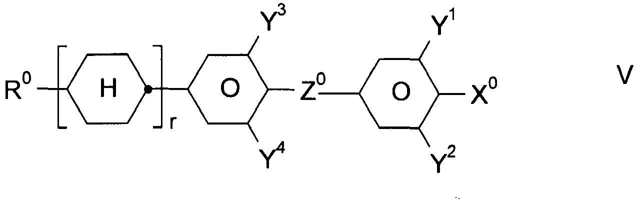 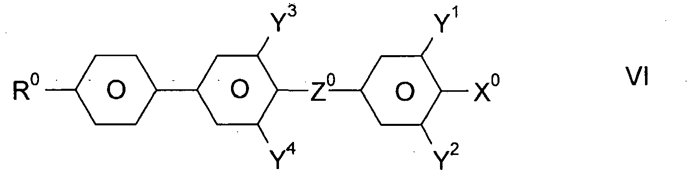 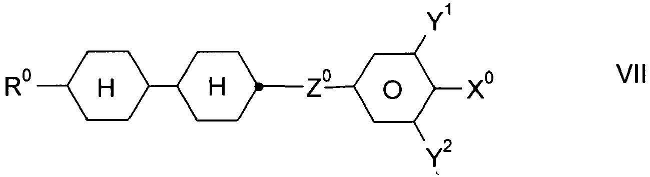 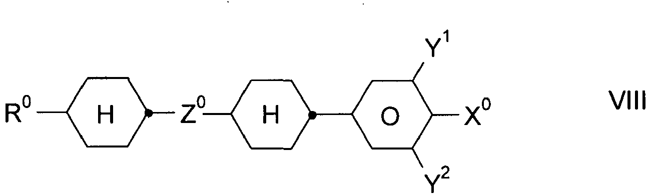 dans lesquelles R⁰, X⁰ et Y¹⁻⁴ présentent les significations qui sont indiquées selon la revendication 3,
Z⁰ représente -C₂H₄-, -(CH₂)₄-, -CH=CH-, -CF=CF-, -C₂F₄-, -CH₂CF₂-, -CF₂CH₂-, -CH₂O-, -OCH₂-, -COO- ou -OCF₂-, dans les formules V et VI également une liaison simple, dans les formules V et VIII également -CF₂O-,
r représente 0 ou 1, et
s représente 0 ou 1.

5. Milieu cristallin liquide selon une ou plusieurs des revendications 1 à 4, **caractérisé en ce qu'**il comprend de manière additionnelle un ou plusieurs composé(s) qui est/sont sélectionné(s) parmi les formules IX à XII : 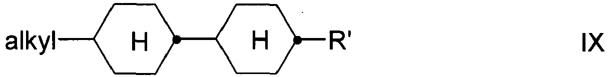 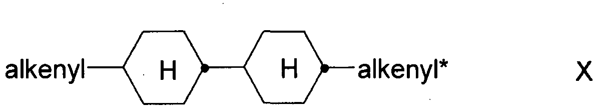 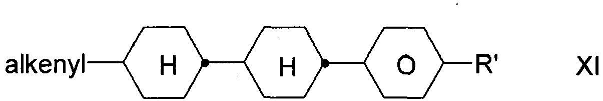 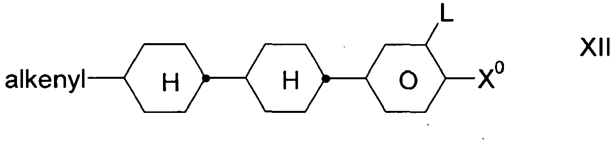 dans lesquelles X⁰ présente les significations qui sont indiquées selon la revendication 3, et
L représente H ou F,
"alkyl" représente C₁₋₆-alkyle,
R' représente C₁₋₆-alkyle, C₁₋₆-alcoxy ou C₂₋₆-alkényle, et
"alkenyl" et "alkenyl*" représentent chacun, indépendamment l'un de l'autre, C₂₋₆-alkényle.

6. Milieu cristallin liquide selon une ou plusieurs des revendications 1 à 5, **caractérisé en ce qu'**il comprend de manière additionnelle un ou plusieurs composé(s) de la formule XIII : 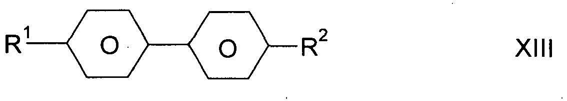 dans laquelle R¹ et R² représentent chacun, indépendamment l'un de l'autre, n-alkyle, alcoxy, oxaalkyle, fluoroalkyle ou alkényle dont chacun comporte jusqu'à 6 atomes de C.

7. Milieu cristallin liquide selon une ou plusieurs des revendications 1 à 6, **caractérisé en ce qu'**il comprend de manière additionnelle un ou plusieurs composé(s) de la formule XVII : 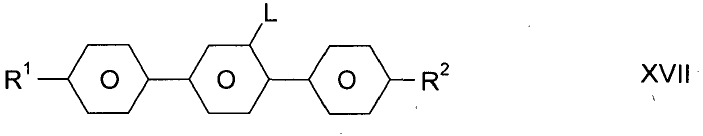 dans laquelle R¹ et R² représentent chacun, indépendamment l'un de l'autre, n-alkyle, alcoxy, oxaalkyle, fluoroalkyle ou alkényle dont chacun comporte jusqu'à 8 atomes de C, et L représente H ou F.

8. Milieu cristallin liquide selon une ou plusieurs des revendications 1 à 7, **caractérisé en ce qu'**il comprend de manière additionnelle un ou plusieurs composé(s) qui est/sont sélectionné(s) parmi le groupe des composés des formules XXVII, XXVIII et XXIX : 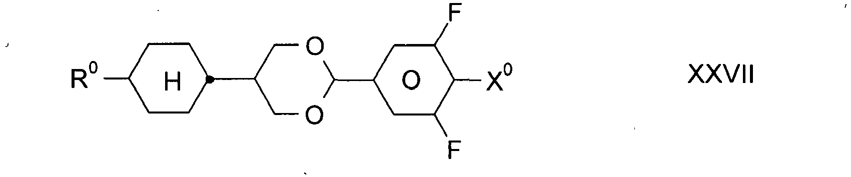 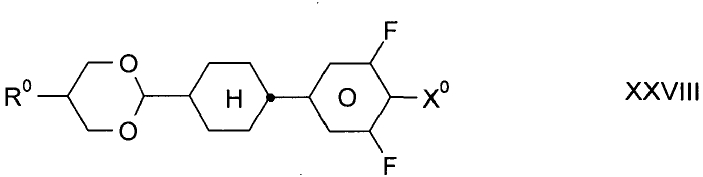 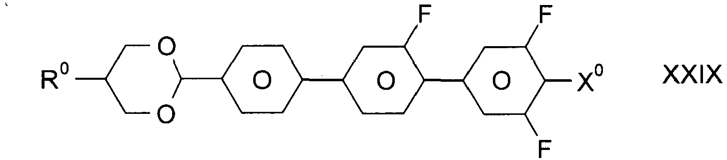 dans lesquelles R⁰ et X⁰ présentent les significations qui sont indiquées selon la revendication 3.

9. Milieu cristallin liquide selon une ou plusieurs des revendications 1 à 8, **caractérisé en ce qu'**il comprend de manière additionnelle un ou plusieurs composé(s) qui est/sont sélectionné(s) parmi le groupe des composés des formules XIX, XX, XXI, XXII, XXIII et XXIV : 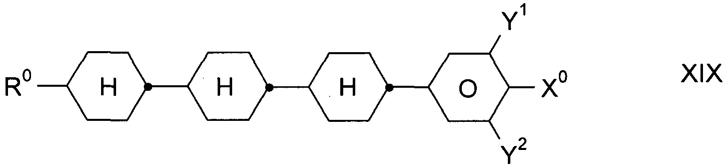 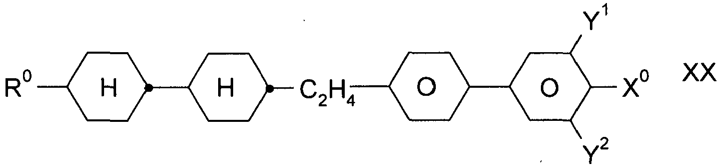 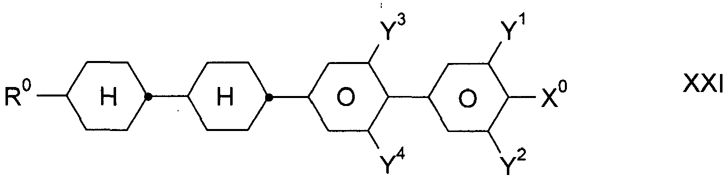 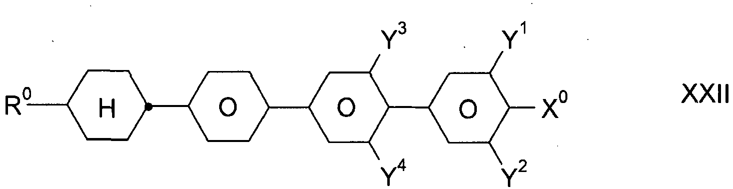 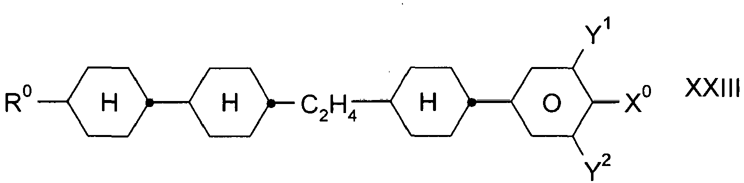 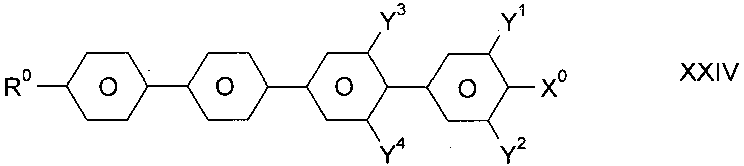 dans lesquelles R⁰ et X⁰ présentent les significations qui sont indiquées selon la revendication 3, et Y¹⁻⁴ représentent chacun, indépendamment les uns des autres, H ou F.

10. Milieu cristallin liquide selon une ou plusieurs des revendications 1 à 9, **caractérisé en ce qu'**il comprend une teneur supérieure ou égale à 20% en poids du composé de la formule IXb : 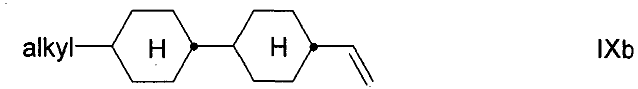 dans laquelle alkyl présente la signification qui est indiquée selon la revendication 5.

11. Milieu cristallin liquide selon une ou plusieurs des revendications 1 à 10, **caractérisé en ce qu'**il comprend 1 à 30% en poids, sur la base du mélange, d'un ou de plusieurs composé(s) de la formule IA.

12. Milieu cristallin liquide selon une ou plusieurs des revendications 1 à 11, **caractérisé en ce qu'**il comprend de manière additionnelle un ou plusieurs additif(s) qui est/sont sélectionné(s) parmi le groupe des sta-biliseurs UV, des dopants et des antioxydants.

13. Milieu cristallin liquide selon une ou plusieurs des revendications 1 à 12, **caractérisé en ce qu'**il comprend de manière additionnelle un ou plusieurs composé(s) polymérisable(s).

14. Procédé pour la préparation d'un milieu cristallin liquide selon une ou plusieurs des revendications 1 à 13, **caractérisé en ce qu'**un ou plusieurs composé(s) de la formule IA, comme définie selon la revendication 1, est/sont mélangé(s) avec d'autres composés mésogènes et en option, également avec un ou plusieurs additif(s) et/ou au moins un composé polymérisable.

15. Utilisation d'un milieu cristallin liquide selon une ou plusieurs des revendications 1 à 13 à des fins électro-optiques.

16. Utilisation du milieu cristallin liquide selon la revendication 15 dans des lunettes à obturateur, pour des applications 3D, dans des affichages TN, PS-TN, STN, ECB, OCB, IPS, PS-IPS, FFS, PS-FFS et PS-VA-IPS.

17. Affichage électro-optique à cristaux liquides contenant un milieu cristallin liquide selon une ou plusieurs des revendications 1 à 13.

18. Composés de la formule IA : 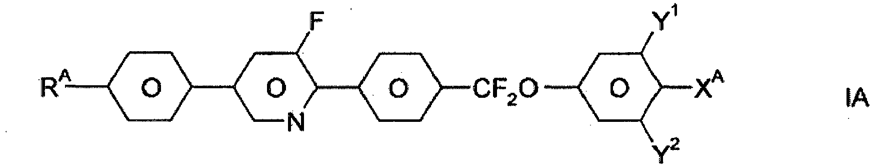 dans laquelle :
R^{A} représente un radical alkyle ou alcoxy halogéné ou non substitué qui comporte 1 à 15 atome(s) de C, où, en outre, un ou plusieurs groupe(s) CH₂ dans ces radicaux peut/peuvent chacun être remplacé(s), indépendamment les uns des autres, par -C≡C-, -CF₂O-, -CH=CH-, 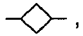 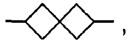 -O-, -CO-O- ou -O-CO- de telle sorte que des atomes de O ne soient pas liés directement les uns aux autres,
X^{A} représente F, CI, CN, SF₅, SCN, NCS, un radical alkyle halogéné, un radical alkényle halogéné, un radical alcoxy halogéné ou un radical alkényloxy halogéné dont chacun comporte jusqu'à 6 atomes de C, et
Y¹⁻² représentent chacun, indépendamment l'un de l'autre, H ou F.

19. Composés des formules IA-g à IA-i : 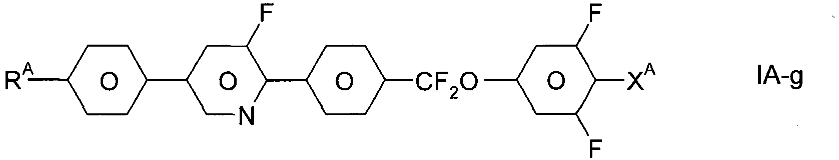 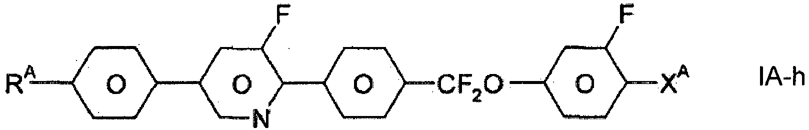 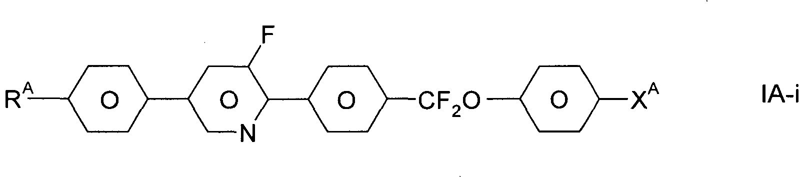 dans lesquelles :
R^{A} et X^{A} présentent les significations qui sont indiquées selon la revendication 18.

20. Composés de la formule IA selon la revendication 18 ou 19, **caractérisé en ce que**:
R^{A} représente un radical alkyle en chaîne droite qui comporte 1 à 6 atome(s) de C ou un radical alkényle qui comporte 2 à 6 atomes de C, et
X^{A} représente F ou OCF₃.
